# EUROPEAN PATENT SPECIFICATION

(11) **EP 3 676 615 B1**
(45) Date of publication and mention of the grant of the patent: **03.08.2022**
(21) Application number: 18765412.4
(22) Date of filing: 31.08.2018
(51) Int. Cl.: G01N 33/573, C07H 21/00, C12Q 1/68

(54) **LATERAL FLOW ASSAY FOR DETECTING THE PRESENCE OF A SPECIFIC MAMMALIAN CELL OR BACTERIA IN A BIOLOGICAL SAMPLE**
LATERALFLUSS-ASSAY ZUR DETEKTION DER ANWESENHEIT EINER SPEZIFISCHEN SÄUGERZELLE ODER -BAKTERIE IN EINER BIOLOGISCHEN PROBE
DOSAGE À ÉCOULEMENT LATÉRAL PERMETTANT DE DÉTECTER LA PRÉSENCE D'UNE CELLULE DE MAMMIFÈRE OU DE BACTÉRIES SPÉCIFIQUES DANS UN ÉCHANTILLON BIOLOGIQUE

(30) Priority: 31.08.2017 EP 17382590
(43) Date of publication of application: 08.07.2020
(60) Divisional application: 21175381.9
(73) Proprietor: SOMAprobes SL, 20019 San Sebastián (ES)
(72) Inventor: HERNÁNDEZ HINCAPIÉ, Frank J., 20009 Donostia-San Sebastián (ES); HERNANDEZ, Luiza Iuliana, 20009 Donostia-San Sebastian (ES); JIMENEZ TRUJILLO, Tania, 20009 - Donostia-San Sebastian (ES)
(74) Representative: Hoffmann Eitle
(86) International application number: PCT/EP2018/073525
(87) International publication number: WO 2019/043187

(56) References cited:
- EP-A1- 2 966 177
- WO-A1-94/29482
- WO-A1-2013/033436
- WO-A1-2013/156794
- WO-A1-2013/156795
- WO-A1-2014/076706
- WO-A1-2015/120406
- WO-A2-2015/059487
- US-A1- 2004 137 479
- US-A1- 2005 227 275
- US-A1- 2011 086 354
- US-B2- 7 998 754
- SHAZIA KHAN ET AL: "Rapid optical determination of ?-lactamase and antibiotic activity", BMC MICROBIOLOGY, BIOMED CENTRAL LTD, GB, vol. 14, no. 1, 4 April 2014 (2014-04-04), page 84, XP021182875, ISSN: 1471-2180, DOI: 10.1186/1471-2180-14-84
- DAVID W. WAREHAM ET AL: "Rapid Detection of Carbapenemases in Enterobacteriaceae: Evaluation of the Resist-3 O.K.N. (OXA-48, KPC, NDM) Lateral Flow Multiplexed Assay", JOURNAL OF CLINICAL MICROBIOLOGY, vol. 55, no. 4, 1 April 2017 (2017-04-01), pages 1223-1225, XP055520026, US ISSN: 0095-1137, DOI: 10.1128/JCM.02471-16
- Thomas Schwartz ET AL: "Detection of antibiotic-resistant bacteria and their resistance genes in wastewater, surface water, and drinking water biofilms", FEMS Microbiology Ecology, 1 January 2003 (2003-01-01), pages 325-335, XP055519891, England DOI: 10.1016/S0168-6496(02)00444-0 Retrieved from the Internet: URL:https://academic.oup.com/femsec/articl e-pdf/43/3/325/18090675/43-3-325.pdf
- ULYASHOVA M M ET AL: "Screening of bacterial genes responsible for resistance to beta-lactam antibiotics using microarrays with enzymatic detection", MOSCOW UNIVERSITY CHEMISTRY BULLETIN, ALLERTON PRESS, HEIDELBERG, vol. 71, no. 4, 27 September 2016 (2016-09-27), pages 236-242, XP036063363, ISSN: 0027-1314, DOI: 10.3103/S002713141604009X [retrieved on 2016-09-27]
- M Abouzied ET AL: "Validation Study of a Receptor-Based Lateral Flow Assay for Detection of Beta- Lactam Antibiotics in Milk Validation Study of a Receptor-Based Lateral Flow Assay for Detection of Beta-Lactam Antibiotics in Milk Performance-Tested Method SM 030802", Journal of AOAC International, 1 May 2009 (2009-05-01), pages 959-974, XP055519959, Retrieved from the Internet: URL:https://www.researchgate.net/profile/M ohamed_Abouzied3/publication/26678121_Vali dation_Study_of_a_Receptor-Based_Lateral_F low_Assay_for_Detection_of_Beta-Lactam_Ant ibiotics_in_Milk/links/53e8fe970cf2dc24b3c 7e7bc/Validation-Study-of-a-Receptor-Based -Lateral-Flow-Assay-for-Detection-of-Beta- Lactam-Ant [retrieved on 2018-10-30]
- DAVID DOUGLAS ET AL: "Validation of a Rapid Lateral Flow Test for the Simultaneous Determination of [beta]-Lactam Drugs and Flunixin in Raw Milk", JOURNAL OF FOOD PROTECTION, vol. 75, no. 7, 1 July 2012 (2012-07-01), pages 1270-1277, XP055519999, US ISSN: 0362-028X, DOI: 10.4315/0362-028X.JFP-11-570

## Description

### Technical field of the invention

The present invention pertains to the medical field, in particular to the use of lateral flow assays for detecting the presence of a specific mammalian cell or bacteria in a biological sample.

### Background of the invention

Emerging pathogens and diseases related to aging of the global population, such as cancer, represent an urgent need for the development of novel methods and devices that reduce detection times, and capable of being highly sensitive and specific to the threatening human condition. Desirable features for the development of diagnostic devices should include: simplicity of design, cost-effectiveness, portability and the ease-of-use, without the need for complex laboratory instrumentation.

Nucleic acids have been proven to be useful recognition molecules for the development of several diagnostic strategies, taking advantage of their flexibility to be adapted to various transduction mechanims, such as fluorescence, electrochemical, pizoelectric, and colorimetric. Although molecular techniques based on the amplification of nucleic acids (e.g. PCR) have been reported as a possible alternative to overcome the present challenges in the diagnostic field, the complexity of use and high cost have limited the wide use of such techniques.

In contrast to nucleic acid-based assays, immunoassays have found wider acceptance due to their affordability, user friendly and versatility of detection formats, making them an excellent choice for point-of-care applications. Among all the systems described using antibodies as recognition molecules, it is notable the high impact of the lateral flow immuno-chromatography (LFIC), which is actually used on those applications where a fast and accurate detection is required.

Recently, interesting approaches based on nucleic acid substrates have been described for targeting specific nucleases that could be used as a blue print of bacterial infections or specific types of cancer. Herein, we describe for the first time, the utility of specific nuclease substrates (nucleic acid probes) that were incorporated in a lateral flow assay, for the rapid, sensitive and specific detection of nuclease activity derived from bacteria or tumour/cancer cells, with a great potential to be used in the clinical, environmental and food safety fields. WO 2015/120406 A1 discloses the similar detectable constructs, as present disclosure, which could be used in the method of both identifying bacteria and determining their antibiotic resistance. WO 2013/033436 A1 discloses in vitro use of a a lateral flow device comprising a polynucleotide sequence susceptible of being cleaved by a specific nuclease derived from a bacteria or a mammalian cell, wherein the polynucleotide sequence is characterized by comprising a capture tag and a reporter molecule at each end of the sequence, for detecting the presence of a specific mammalian cell or bacteria in a biological sample.

WO 2014/076706 A1 describes, methods and compositions directed to improvement of existing PCR methods, for example regarding their ability to confirm a suspected case of sepsis and identify a variety of common pathogen and antibiotic-resistant genes present in the blood, even when present in very low copy number.

### Summary of the invention

The present invention provides for a rapid and sensitive molecular approach for the diagnosis of bacterial infections and tumours/cancer.

For that purpose, an initial aspect of the present invention provides for a method for detecting bacteria and its resistance to beta-lactam antibiotics treatment in a test sample, which comprises: 1) incubating in the test sample, i) a synthetic Substrate or mixture of Substrates for detecting the presence of a bacteria, and 2) simultaneously or subsequently establishing the resistance of the bacteria to beta-lactam antibiotics treatment by incubating in the test sample a β-iactamase responsive-molecule, for a time sufficient for cleavage of the β-iactamase responsive-molecule by a β-lactamase; wherein
a. the Substrate(s) comprises a single-stranded nucleic acid molecule containing at least one ribonucleotide or deoxyribonucleotide or chemically modified nucleotide residue at an internal position that functions as a nuclease cleavage site, a capture tag on one end and i) a reporter molecule or ii) a further capture tag capable of binding the reporter molecule located in the conjugate pad of a support of a lateral flow device, on the other end; and wherein
b. the β-iactamase responsive-molecule comprises a compound comprising a β-lactam ring, preferably a β-lactam antibiotic selected from the list consisting of penicillin derivatives (penams), cephalosporins (cephems), monobactams, and carbapenems, more preferably methicillin, a capture tag on one end and i) a reporter molecule or ii) a further capture tag capable of binding the reporter molecule located in the conjugate pad of a support of a lateral flow device, on the other end.

In a preferred embodiment, the result of the above method is, preferably visually, detected, in particular whether the substrate/s and the β-iactamase responsive-molecule has been cleaved or not by a test sample, by using a lateral flow device or by binding the complexes formed by the substrate/s and the β-iactamase responsive-molecule after the incubation to a solid support that facilitates immunoassay techniques such as enzyme linked immuno-sorbent assay (ELISA), a radioimmunoassay (RIA), an immuno radiometric assay (IRMA), a fluorescent immunoassay (FIA), a chemiluminescent immunoassay (CLIA), magnetic beads-based immunoassay (MBI), or an electro-chemiluminescent immunoassay (ECL).

In a preferred embodiment, the result is, preferably visually, detected, in particular whether the substrate/s and the β-iactamase responsive-molecule has been cleaved or not by a test sample, by using a lateral flow device.

In a preferred embodiment, said β-iactamase responsive-molecule comprises 4-methoxybenzyl (6R,7R)-3-(((4-benzamidophenyl)thio)methyl)-8-oxo-7-(2 phenylacetamido)-5-thia-1-azabicyclo[4.2.0]oct-2-ene-2-carboxylate which is a truncated β-lactam responsive molecule (CEProbe), or (6*S*,7*S*)-7-(((3',6'-dihydroxy-3-oxo-3*H*-spiro[isobenzofuran-1,9'-xanthen]-6-ylcarbonyl)oxy)amino)-8-oxo-3-(((4-(5-((3a*R*,4*R*,6a*S*)-2-oxohexahydro-1*H*-thieno[3,4-*d*]imidazol-4-yl)pentanamido)phenyl)thio)methyl)-5-thia-1- azabicyclo[4.2.0]oct-2-ene-2-carboxylic acid (FAM-CEProbe-Biotin), or 6*S*,7*S*)-7-(5-(((3*S*,5*R*,8*R*,9*S*,10*S*,12*R*,13*S*,14*S*,17*R*)-12,14-dihydroxy-10,13-dimethyl-17-(5-oxo-2,5-dihydrofuran-3-yl)hexadecahydro-1*H*-cyclopenta[*a*]phenanthren-3-yl)oxy)-5-oxopentanamido)-8-oxo-3-(((4-(5-((3a*R*,4*R*,6a*S*)-2-oxohexahydro-1*H*-thieno[3,4-*d*]imidazol-4-yl)pentanamido)phenyl)thio)methyl)-5-thia-1-azabicyclo[4.2.0]oct-2-ene-2-carboxylic acid (Digoxigenin-CEProbe-Biotin).

In a preferred embodiment, the capture tag of the Substrate/s and/or β-iactamase responsive-molecule is selected from the list consisting of BIOTIN, FITC, FAM, DIGOXIGENIN, DNP (DINITROPHENYL), TEXAS RED, TAMRA or any other small antigen.

In a preferred embodiment, the reporter molecule of the Substrate/s and/or β-lactamase responsive-molecule is selected from the list consisting of latex and gold nanoparticles, carbon particles or any other colorimetric reporter molecule.

A further aspect of the present invention refers to a system that comprises:
a. A synthetic Substrate or mixture of Substrates for detecting the presence of a bacteria and the β-iactamase responsive-molecule for establishing the resistance of the bacteria to beta-lactam antibiotics and β-iactamase responsive-molecules; wherein a1. the Substrate(s) comprises a single-stranded nucleic acid molecule containing at least one ribonucleotide or deoxyribonucleotide or chemically modified nucleotide residue at an internal position that functions as a nuclease cleavage site, a capture tag on one end and i) a reporter molecule or ii) a further capture tag capable of binding the reporter molecule located in the conjugate pad of a support of a lateral flow device, on the other end; and wherein a2. the β-lactamase responsive-molecule comprises a compound comprising a β-lactam ring, preferably a β-lactam antibiotic selected from the list consisting of penicillin derivatives (penams), cephalosporins (cephems), monobactams, and carbapenems, more preferably methicillin, a capture tag on one end and i) a reporter molecule or ii) a further capture tag capable of binding the reporter molecule located in the conjugate pad of a support of a lateral flow device, on the other end;
b. A support suitable for lateral flow that comprises:
   b1. A backing card; and
   b2. A sample pad, a membrane and a wicking pad, all of them on top of the backing pad, wherein the membrane is located after the sample pad so that the sample flows from the sample pad to the membrane, wherein the membrane comprises at least two test lines which in turn respectively comprises molecules immobilized in each test line for capturing the capture tag of the polynucleotide sequence/s and for capturing the capture tag of the β-iactamase responsive-molecules, and wherein the wicking pad is located at the end of the membrane.

In a preferred embodiment, the membrane further comprises a control line.

A further aspect of the present invention refers to a system that comprises:
a. A synthetic Substrate or mixture of Substrates for detecting the presence of a bacteria and the β-iactamase responsive-molecule for establishing the resistance of the bacteria to beta-lactam antibiotics and β-iactamase responsive-molecules, wherein a1. the Substrate(s) comprises a single-stranded nucleic acid molecule containing at least one ribonucleotide or deoxyribonucleotide or chemically modified nucleotide residue at an internal position that functions as a nuclease cleavage site, a capture tag on one end and i) a reporter molecule or ii) a further capture tag capable of binding the reporter molecule located in the conjugate pad of a support of a lateral flow device, on the other end; and wherein a2. the β-lactamase responsive-molecule comprises a compound comprising a β-lactam ring, preferably a β-lactam antibiotic selected from the list consisting of penicillin derivatives (penams), cephalosporins (cephems), monobactams, and carbapenems, more preferably methicillin, a capture tag on one end and i) a reporter molecule or ii) a further capture tag capable of binding the reporter molecule located in the conjugate pad of a support of a lateral flow device, on the other end, wherein the
   substrate's and β-iactamase responsive-molecules comprise two capture molecules at each end of the molecules, and wherein one of the capture molecules is capable of binding the reporter molecule located in the conjugate pad of the support; and
b. A support suitable for lateral flow that comprises:
   b1. A backing card; and
   b2. A sample pad, a membrane and a wicking pad, all of them on top of the backing pad, wherein the membrane is located after the sample pad so that the sample flows from the sample pad to the membrane, wherein the membrane comprises at least two test lines which in turn respectively comprises molecules immobilized in each test line for capturing the capture tag of the polynucleotide sequence/s and for capturing the capture tag of the β-iactamase responsive-molecules, wherein the wicking pad is located at the end of the membrane,
      and wherein the system further comprises a conjugate pad between the sample pad and the membrane, which in turn comprises a reporter molecule.

In a preferred embodiment, the membrane further comprises a control line.

In a preferred embodiment, any of the above systems comprises a support to incubate a biological sample and the synthetic Substrate or mixture of Substrates and the β-iactamase responsive-molecules.

In a preferred embodiment, in any of the above systems the β-iactamase responsive-molecule is as defined in the initial aspect of the invention.

A further aspect of the invention refers to the use of any of the above systems for simultaneously detecting bacteria and its resistance to beta-lactam antibiotics such as methicillin in a test sample.

In a preferred embodiment, in the method of the initial aspect, the method further comprises the use of a synthetic Substrate or mixture of Substrates for detecting the presence of mammalian cancer cells in the test sample and/or the resistance to cancer treatment.

### Brief description of the figures

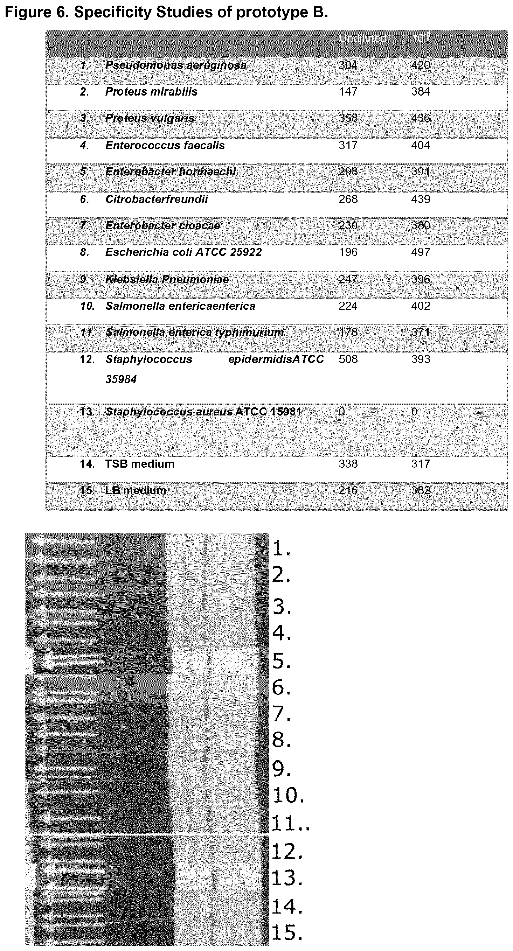

**Figure 1****.** Scheme 1 of the Lateral Flow Probe Chromatography (LFPC), where the polynucleotide probe is functionalized on the surface of the reporter molecule by chemical coupling.
**Figure 2****.** Scheme 2 of the Lateral Flow Probe Chromatography (LFPC), where the polynucleotide probe is modified at both ends with capture tags. In this system the reporter is functionalized with a recognition molecule that binds the polynucleotide.
**Figure 3****.** Proof of concept experiment that demonstrates the feasibility of the LFPC system described in Scheme 1.
   1. *S. aureus* supernatant + TT probe #1
   2. *S. aureus* supernatant + TT probe #2
   3. *S. epidermidis* + TT probe #1
   4. *S. epidermidis* + TT probe #2
**Figure 4****.** Proof of concept experiment that demonstrates the feasibility of the LFPC system described in Scheme 2.
   1. *S. aureus* supernatant + TT probe
   2. *S. epidermidis* + TT probe
   3. TSB (media) + TT probe
   4. Water + TT probe
   5. *S. aureus* supernatant + Control probe (undegradable)
   6. S. *epidermidis* + Control probe (undegradable)
**Figure 5****.** Sensitivity studies of the LFPC were carried out by serial dilutions of 10X of 24h S. *aureus* cultures. The signal reported for each strip was quantified by a band reader and a picture was taken. The data was acquired in duplicates.
   1. Undiluted (Und) S. aureus supernatants + TTProbe #1
   2. Undiluted (Und) S. aureus supernatants + TTProbe #2
   3. Dilution 10⁻¹(-1) S. aureus supernatants + TTProbe #1
   4. Dilution 10⁻¹(-1) S. aureus supernatants + TTProbe #2
   5. Dilution 10⁻² (-2) S. aureus supernatants + TTProbe #1
   6. Dilution 10⁻² (-2) S. aureus supernatants + TTProbe #2
   7. Dilution 10⁻³ (-3) S. aureus supernatants + TTProbe #1
   8. Dilution 10⁻³ (-3) S. aureus supernatants + TTProbe #2
   9. Dilution 10⁻⁴ (-4) S. aureus supernatants + TTProbe #1
   10. Dilution 10⁻⁴ (-4) S. aureus supernatants + TTProbe #2
   11. Dilution 10⁻⁵ (-5) S. aureus supernatants + TTProbe #1
   12. Dilution 10⁻⁵ (-5) S. aureus supernatants + TTProbe #2
   13. Dilution 10⁻⁶ (-6) S. aureus supernatants + TTProbe #1
   14. Dilution 10⁻⁶ (-6) S. aureus supernatants + TTProbe #2
**Figure 6****.** Specificity studies of the LFPC were carried out by testing 12 non-specific bacteria supernatants, along with 2 culture media as controls. The signal reported for each strip was quantified by a band reader and a picture was taken. The bacteria cultures evaluated were enumerated and reported in the table with the value obtained for each strip. Band reader measurements and pictures were acquired.

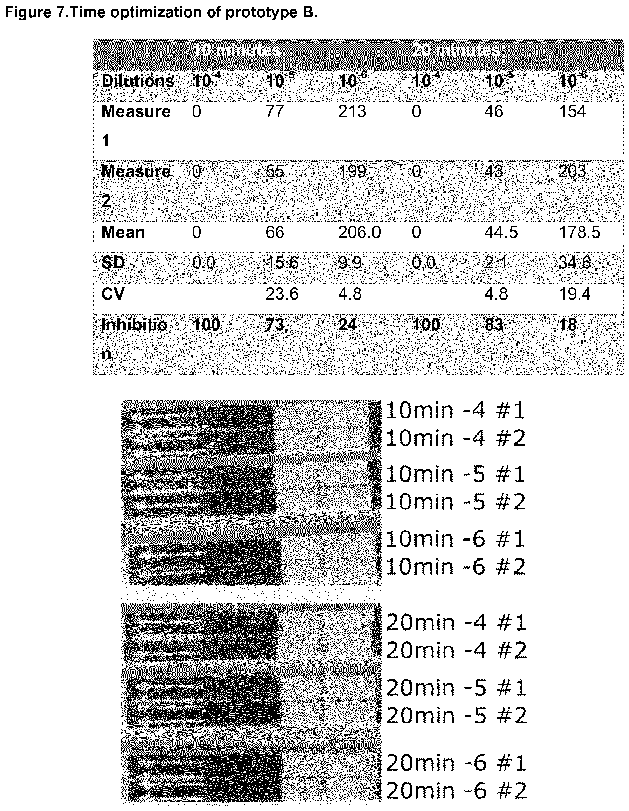

**Figure 7****.** Time experiments were carried out under optimal conditions for the LFPC system described in this invention. Time points of 10 and 20 minutes were selected. For each time point several dilutions of S. *aureus* supernatants were evaluated, as indicated in the table and pictures.

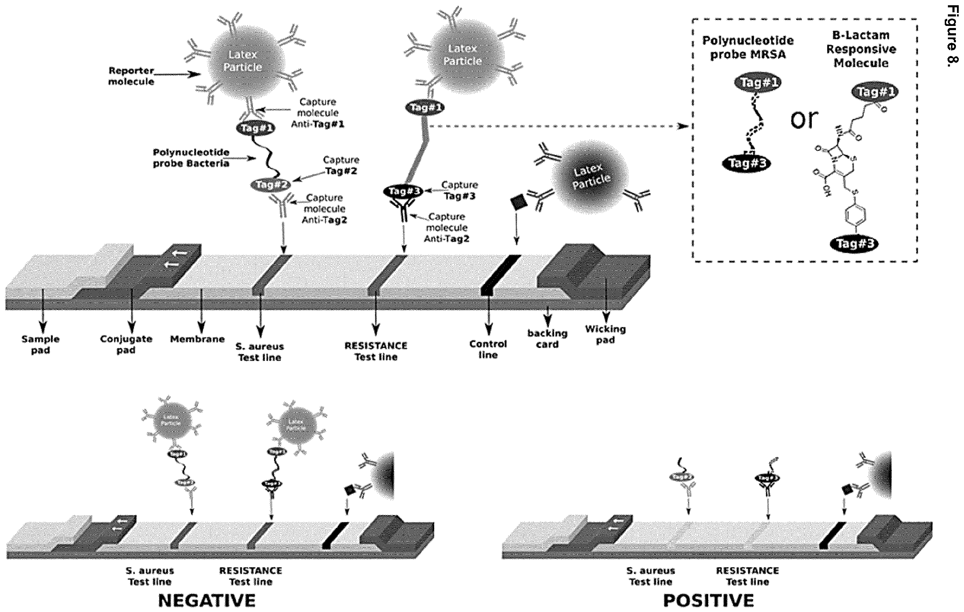

**Figure 8****.** Scheme of double detection system in lateral flow. The first option shows the detection based on two polynucleotides: i) A polynucleotide probe for detecting bacteria and a ii) polynucleotide probe for detecting MRSA. The second option uses iii) a polynucleotide for detecting bacteria and iv) a β-lactam responsive molecule for detecting the presence of β-lactamases.

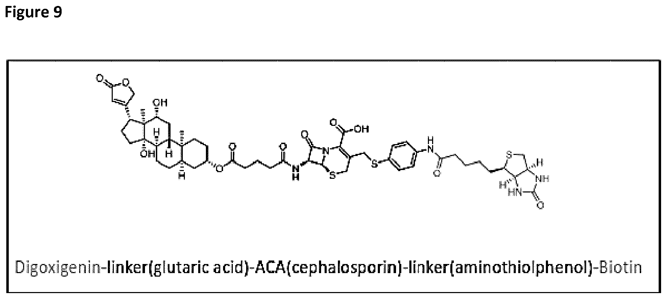

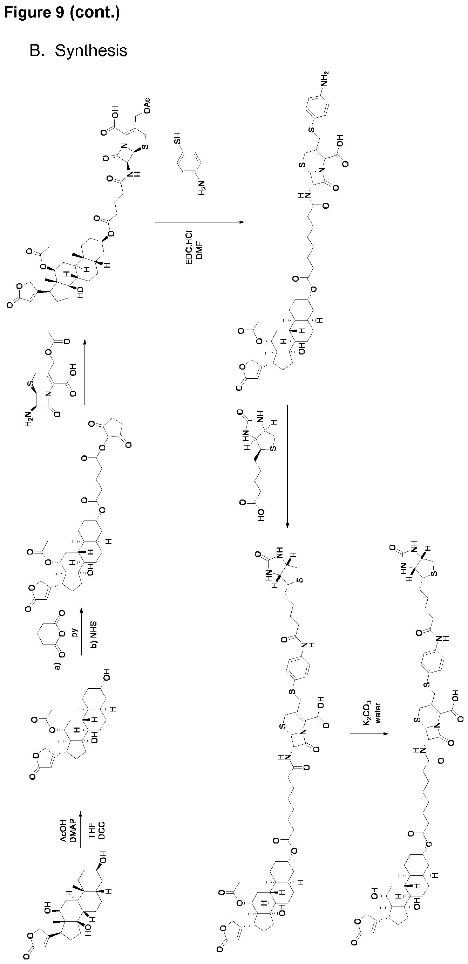

**Figure 9**. A) Novel responsive molecule for detecting antibiotic resistance and B) route of synthesis.

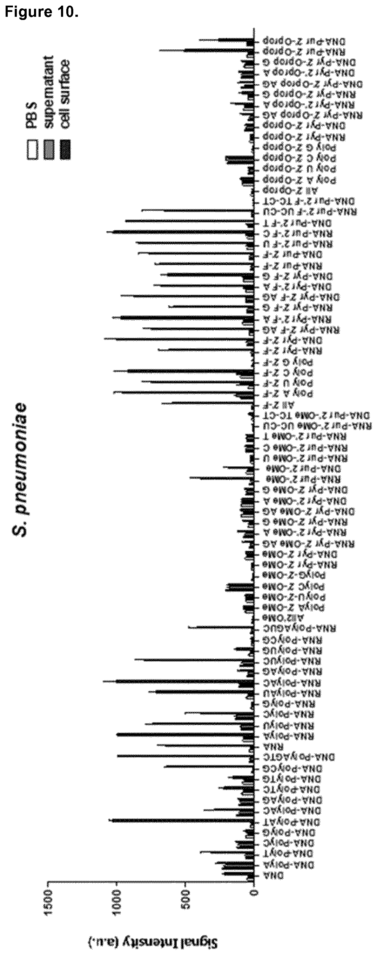

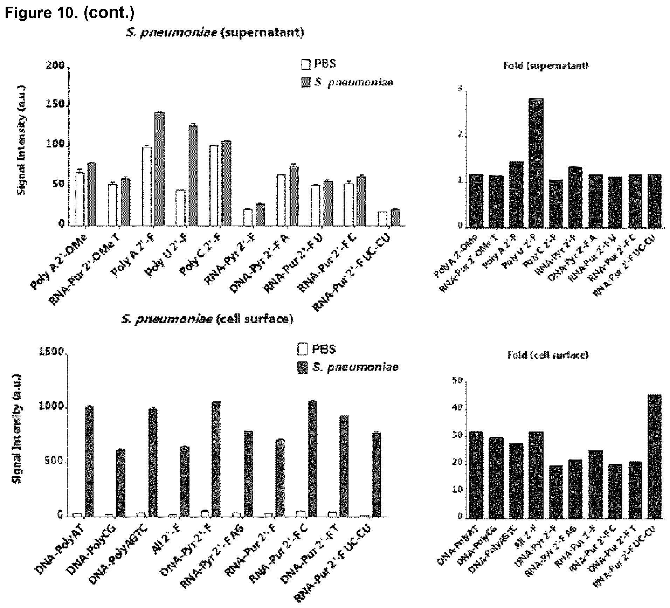

**Figure 10****.** Best performing probes for ***Streptococcus pneumoniae.*** The bars *(upper panel)* indicate the nuclease activity reported by each probe for supernatants and cell surface. The error bars are the standard deviation of three independent measurements. The top ten probes were selected for each case, supernatant and cell surface *(left panels)* and the fold difference (Sample / PBS controls) *(right panels)* is also shown

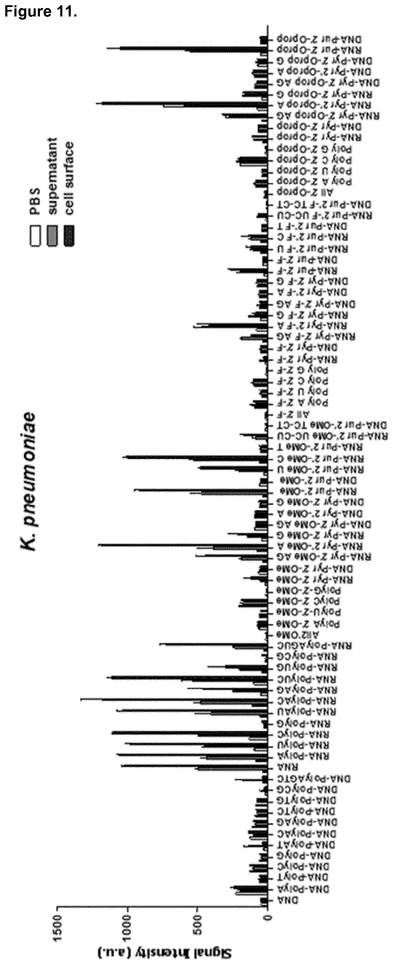

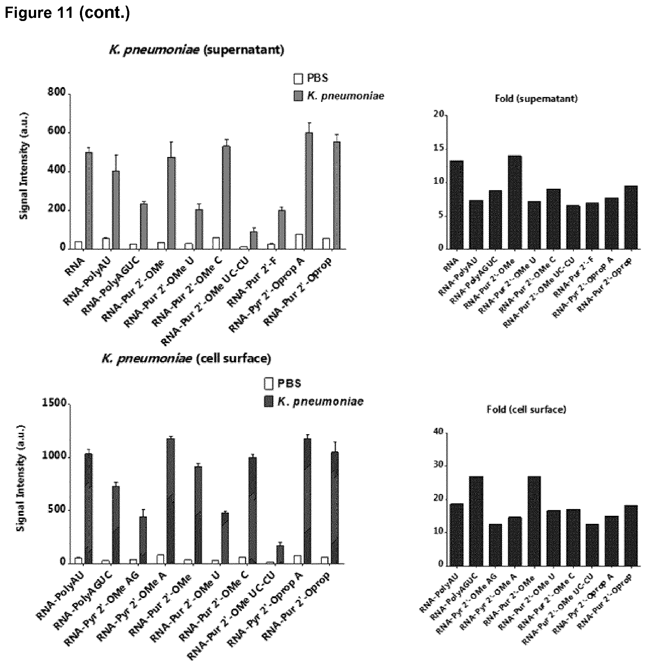

**Figure 11****.** Best performing probes for ***Klebsiella pneumoniae.*** The bars *(upper panel)* indicate the nuclease activity reported by each probe for supernatants and cell surface. The error bars are the standard deviation of three independent measurements. The top ten probes were selected for each case, supernatant and cell surface *(left panels)* and the fold difference (Sample / PBS controls) *(right panels)* is also shown.

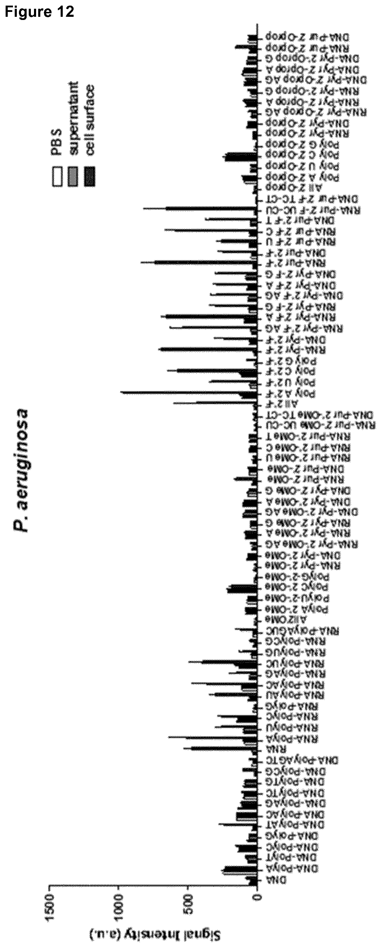

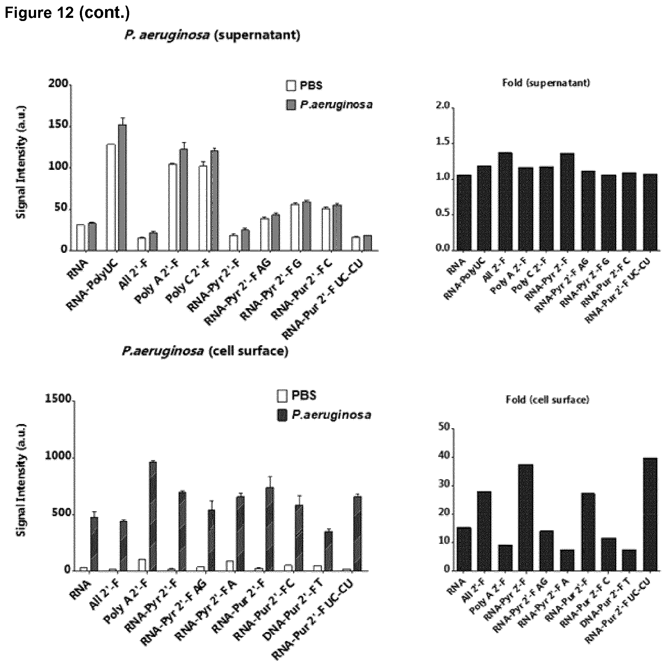

**Figure 12****.** Best performing probes for ***Pseudomona aeruginosa.*** The bars *(upper panel)* indicate the nuclease activity reported by each probe for supernatants and cell surface. The error bars are the standard deviation of three independent measurements. The top ten probes were selected for each case, supernatant and cell surface *(left panels)* and the fold difference (Sample / PBS controls) *(right panels)* is also shown.

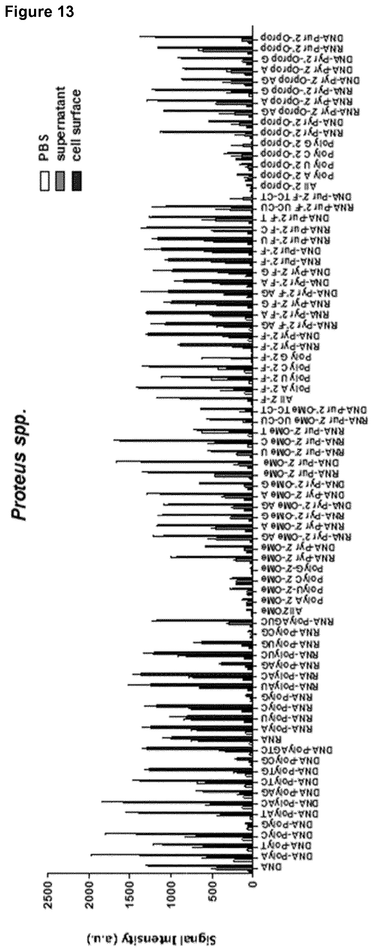

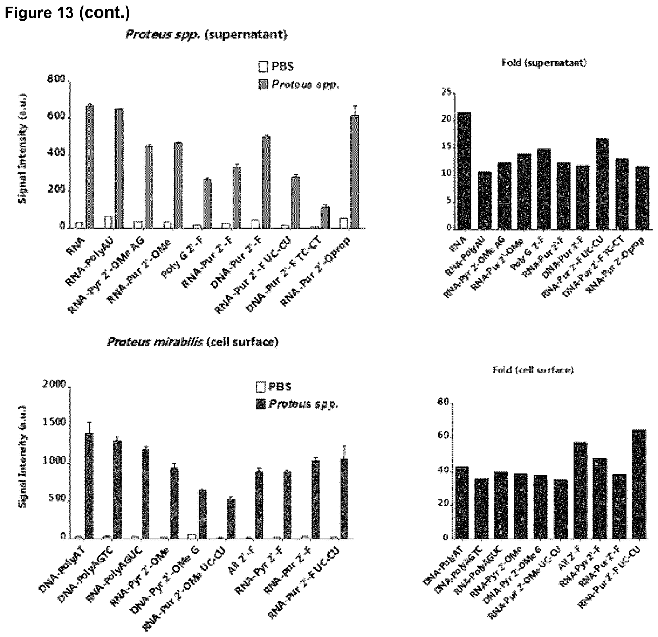

**Figure 13****.** Best performing probes for ***Proteus spp.*** The bars *(upper panel)* indicate the nuclease activity reported by each probe for supernatants and cell surface. The error bars are the standard deviation of three independent measurements. The top ten probes were selected for each case, supernatant and cell surface *(left panels)* and the fold difference (Sample / PBS controls) *(right panels)* is also shown.

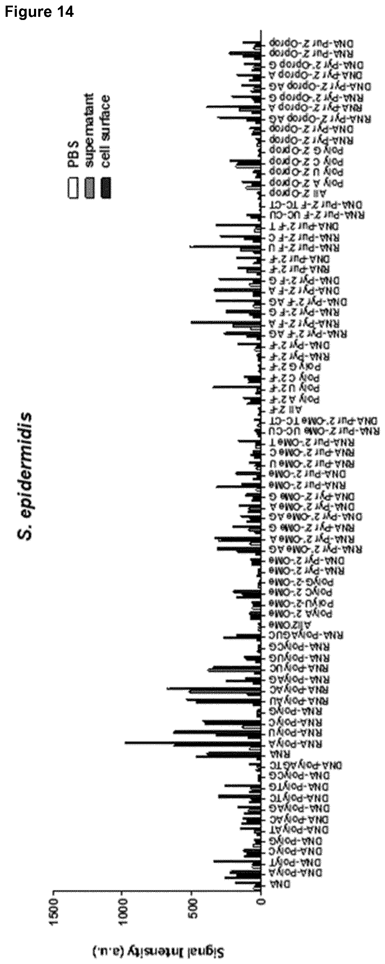

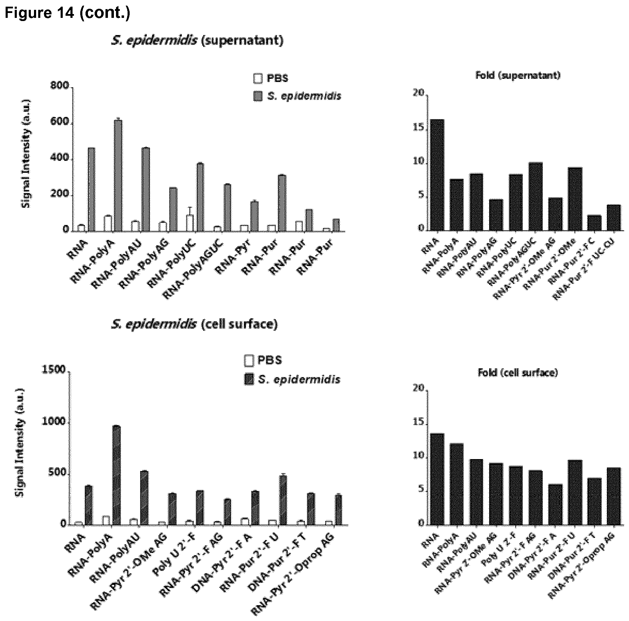

**Figure 14****.** Best performing probes for ***Sthapylococcus epidermidis.*** The bars *(upper panel)* indicate the nuclease activity reported by each probe for supernatants and cell surface. The error bars are the standard deviation of three independent measurements. The top ten probes were selected for each case, supernatant and cell surface *(left panels)* and the fold difference (Sample / PBS controls) *(right panels)* is also shown.

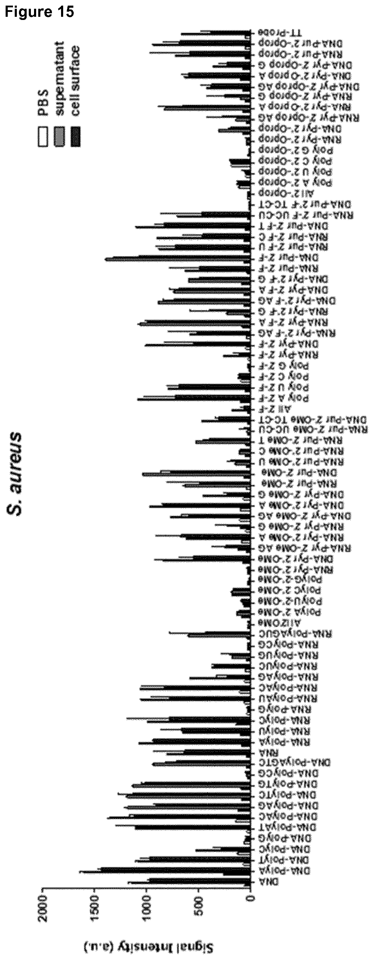

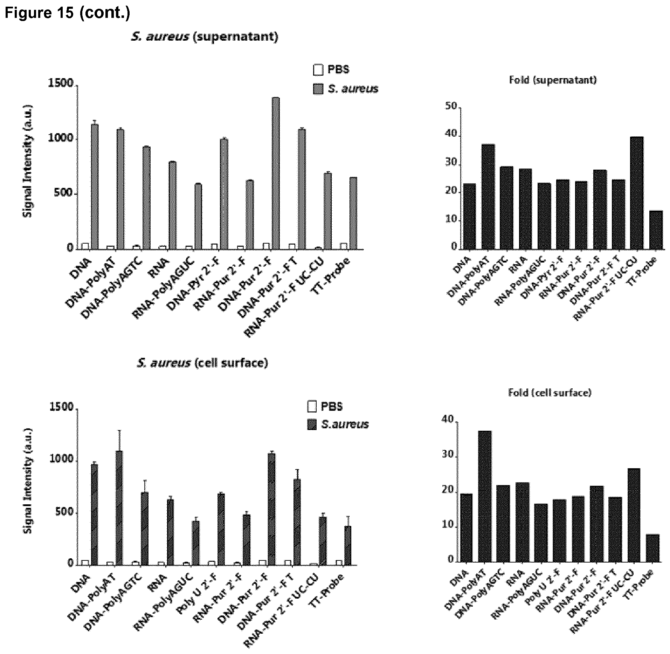

**Figure 15****.** Best performing probes for ***Staphylococcus aureus.*** The bars *(upper panel)* indicate the nuclease activity reported by each probe for supernatants and cell surface. The error bars are the standard deviation of three independent measurements. The top ten probes were selected for each case, supernatant and cell surface *(left panels)* and the fold difference (Sample / PBS controls) *(right panels)* is also shown.

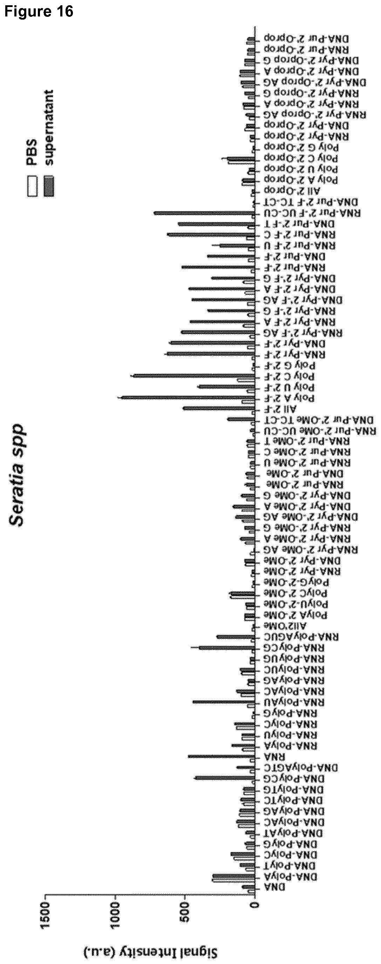

**Figure 16****.** Best performing probes for ***Serratia spp.*** The bars indicate the nuclease activity reported by each probe for supernatants. The error bars are the standard deviation of three independent measurements.

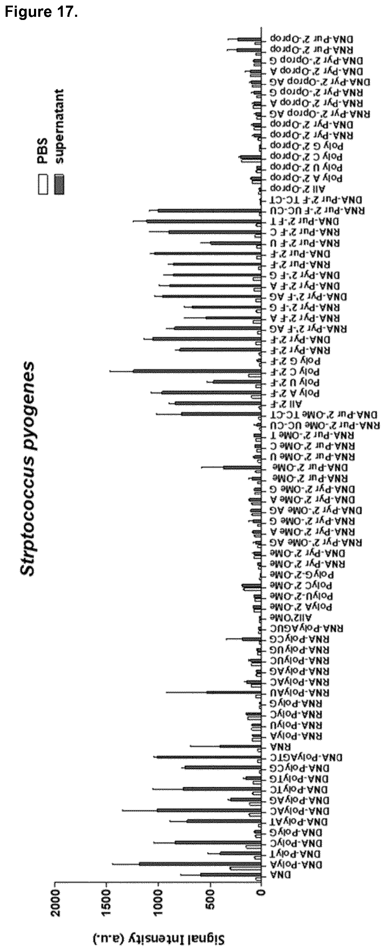

**Figure 17****.** Best performing probes for ***Streptococcus pyogenes.*** The bars indicate the nuclease activity reported by each probe for supernatants. The error bars are the standard deviation of three independent measurements.

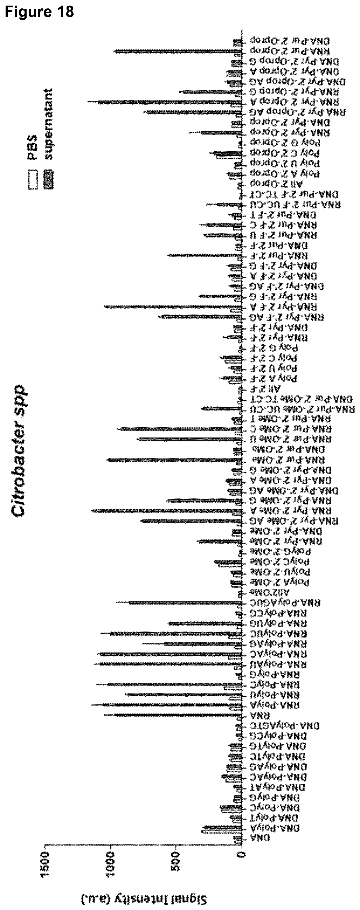

**Figure 18****.** Best performing probes for ***Citrobacter spp.*** The bars indicate the nuclease activity reported by each probe for supernatants. The error bars are the standard deviation of three independent measurements.

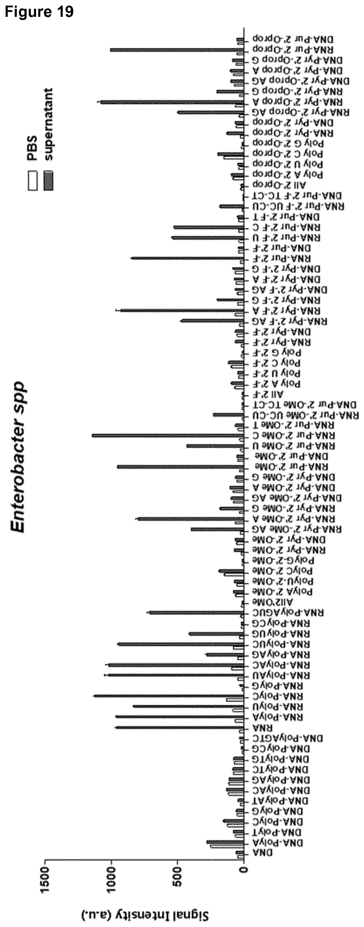

**Figure 19****.** Best performing probes for ***Enterobacter spp.*** The bars indicate the nuclease activity reported by each probe for supernatants. The error bars are the standard deviation of three independent measurements.

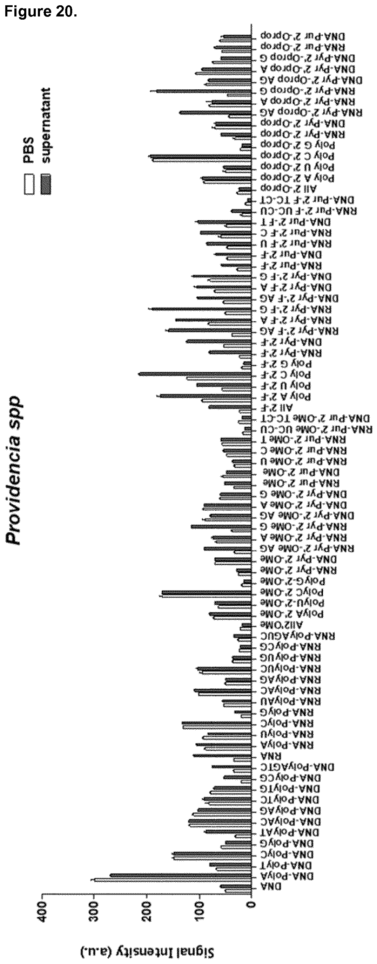

**Figure 20****.** Best performing probes for ***Providencia spp.*** The bars indicate the nuclease activity reported by each probe for supernatants. The error bars are the standard deviation of three independent measurements.

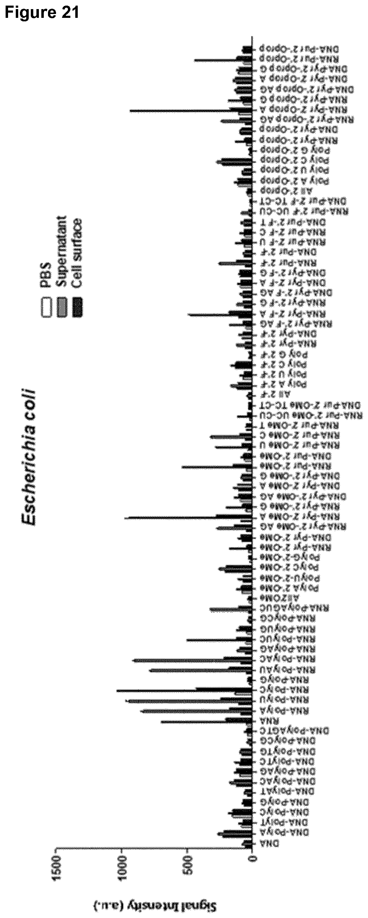

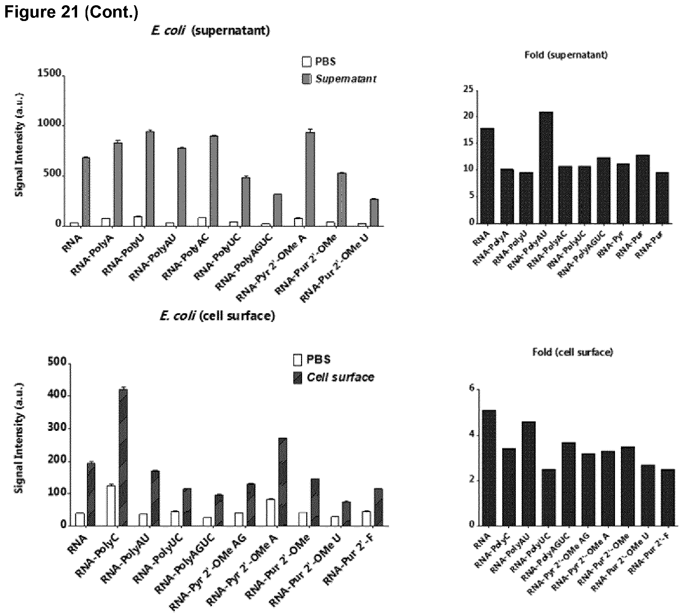

**Figure 21****.** Best performing probes for ***Escherichia coli.*** The bars *(upper panel)* indicate the nuclease activity reported by each probe for supernatants and cell surface. The error bars are the standard deviation of three independent measurements. The top ten probes were selected for each case, supernatant and cell surface *(left panels)* and the fold difference (Sample / PBS controls) *(right panels)* is also shown

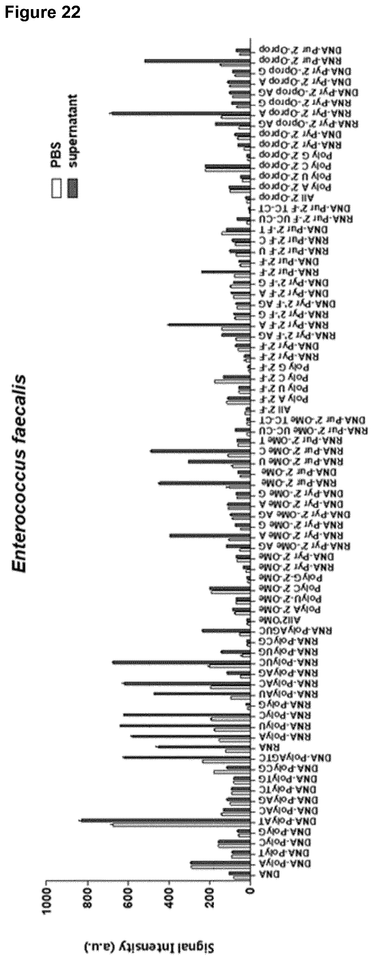

**Figure 22****.** Best performing probes for ***Enterococcus faecalis.*** The bars indicate the nuclease activity reported by each probe for supernatants. The error bars are the standard deviation of three independent measurements.

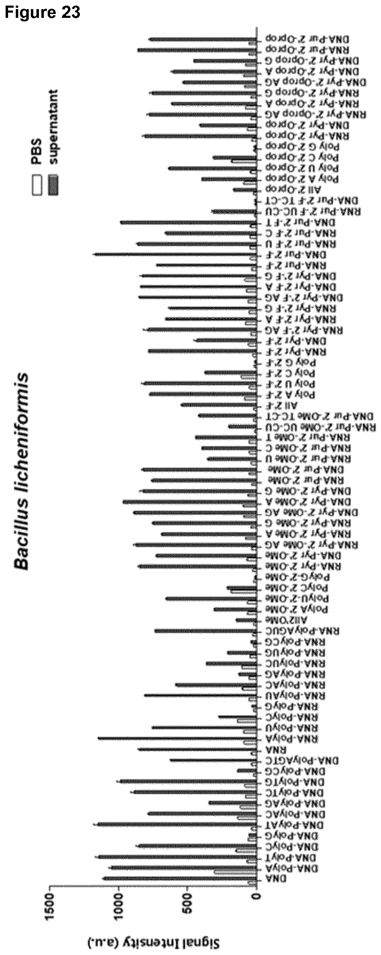

**Figure 23****.** Best performing probes for ***Bacillus licheniformis.*** The bars indicate the nuclease activity reported by each probe for supernatants. The error bars are the standard deviation of three independent measurements.

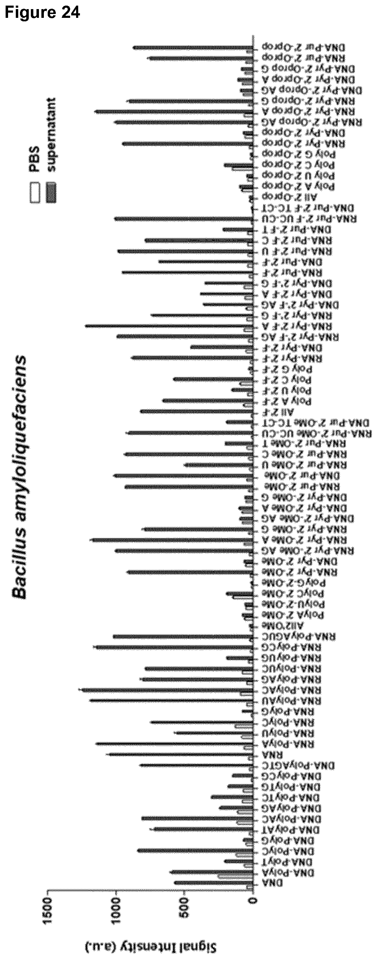

**Figure 24****.** Best performing probes for ***Bacillus amyloliquefaciens.*** The bars indicate the nuclease activity reported by each probe for supernatants. The error bars are the standard deviation of three independent measurements.

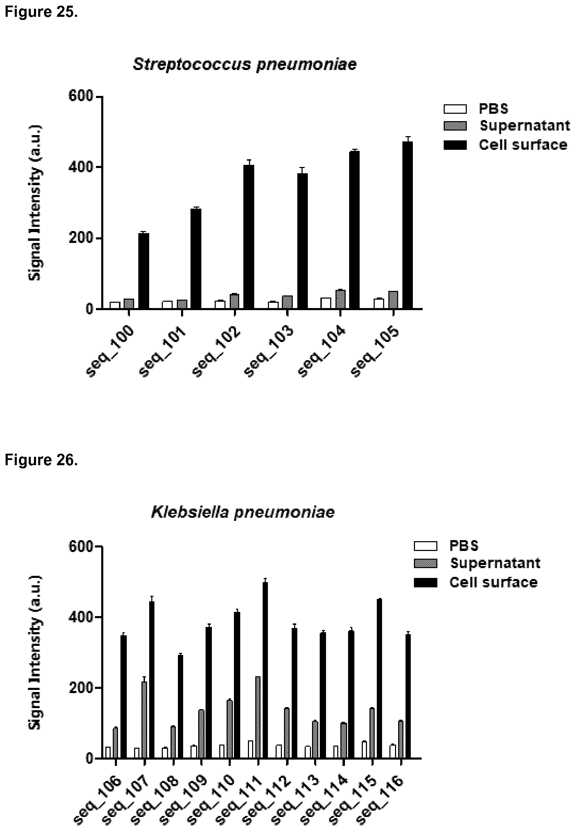

**Figure 25****.** Second generation probes for ***Streptococcus pneumoniae.*** The bars indicate the nuclease activity reported by each probe for the PBS, supernatant and cell surface. The error bars are the standard deviation of three independent measurements.
**Figure 26****.** Second generation probes for ***Klebsiella pneumoniae.*** The bars indicate the nuclease activity reported by each probe for the PBS, supernatant and cell surface. The error bars are the standard deviation of three independent measurements.

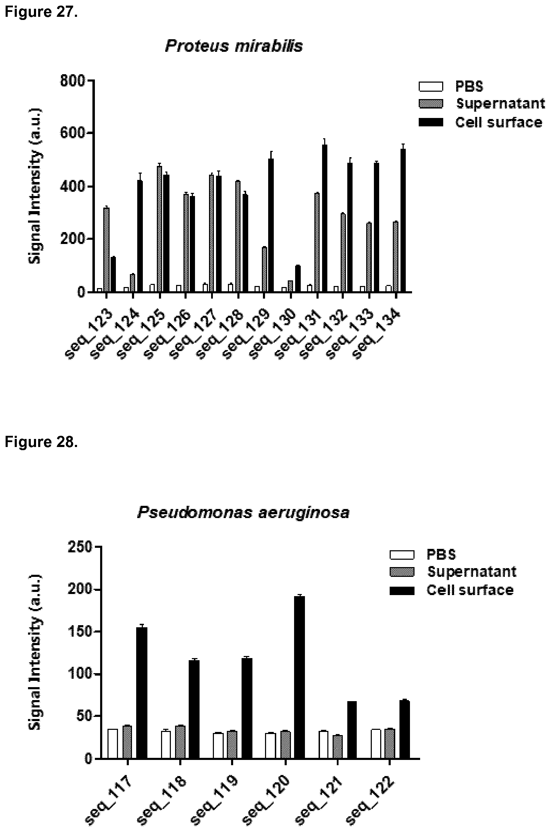

**Figure 27****.** Second generation probes for ***Proteus mirabilis.*** The bars indicate the nuclease activity reported by each probe for the PBS, supernatant and cell surface. The error bars are the standard deviation of three independent measurements.
**Figure 28****.** Second generation probes for ***Pseudomonas aeruginosa.*** The bars indicate the nuclease activity reported by each probe for the PBS, supernatants and cell surface. The error bars are the standard deviation of three independent measurements.

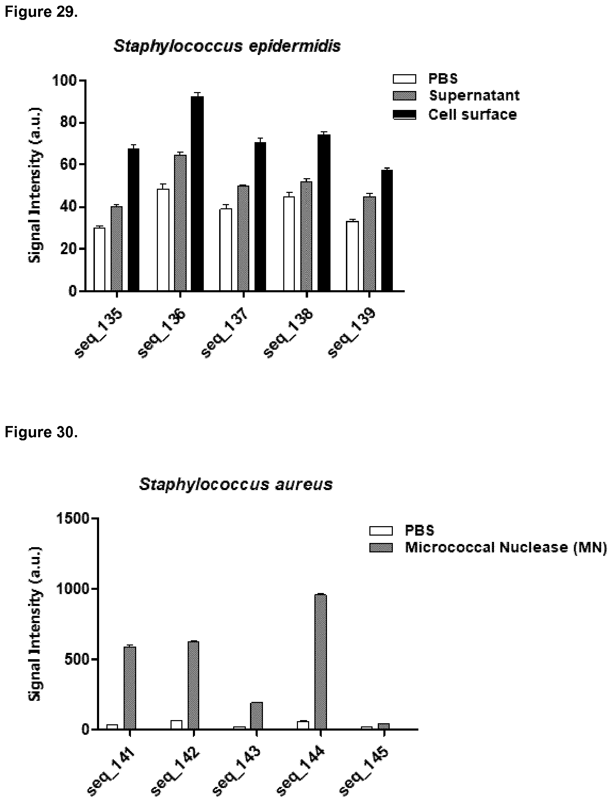

**Figure 29****.** Second generation probes for ***Staphylococcus epidermidis.*** The bars indicate the nuclease activity reported by each probe for the PBS, supernatants and cell surface. The error bars are the standard deviation of three independent measurements.
**Figure 30****.** Second generation probes for ***Staphylococcus aureus.*** The bars indicate the Micrococcal Nuclease activity reported by each probe for the PBS and supernatants. The error bars are the standard deviation of three independent measurements.

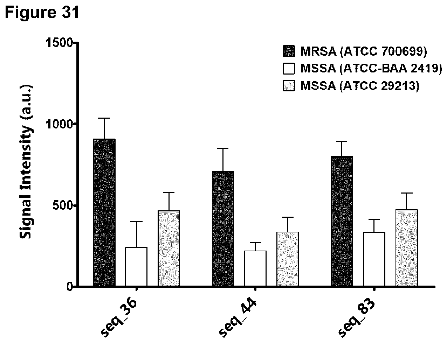

**Figure 31****.** Best performing probes for detecting MRSA strains. The bars indicate the nuclease activity reported by each probe for supernatants. The error bars are the standard deviation of three independent measurements.

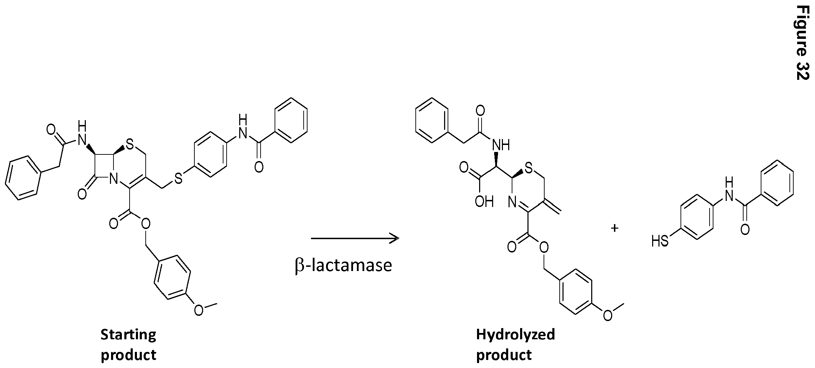

**Figure 32****.** Schematic representation of the reaction products of the β-lactam responsive molecule and β-iactamase activity.

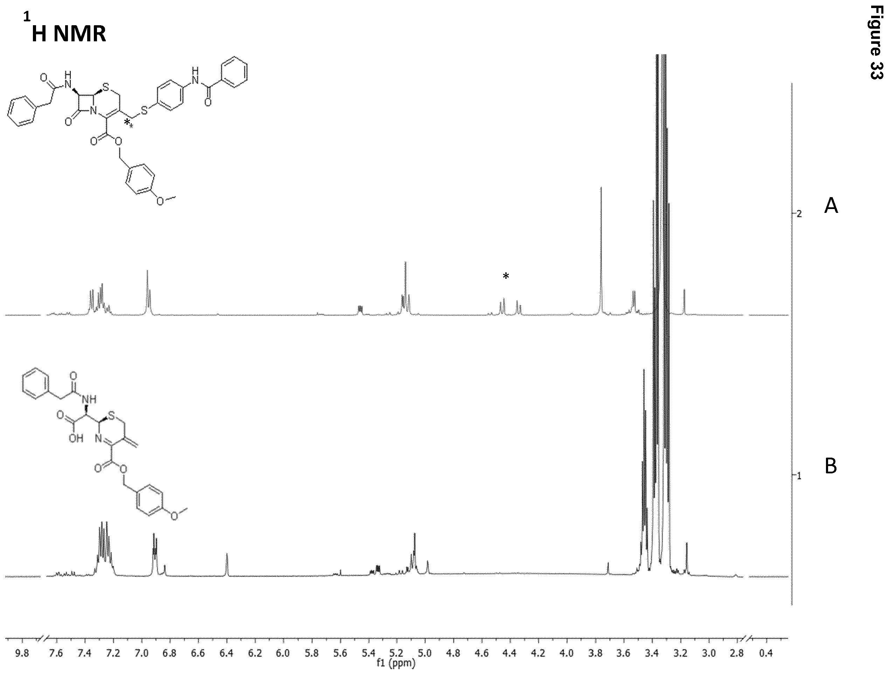

**Figure 33****.** 1H NMR analysis. NMR spectra.

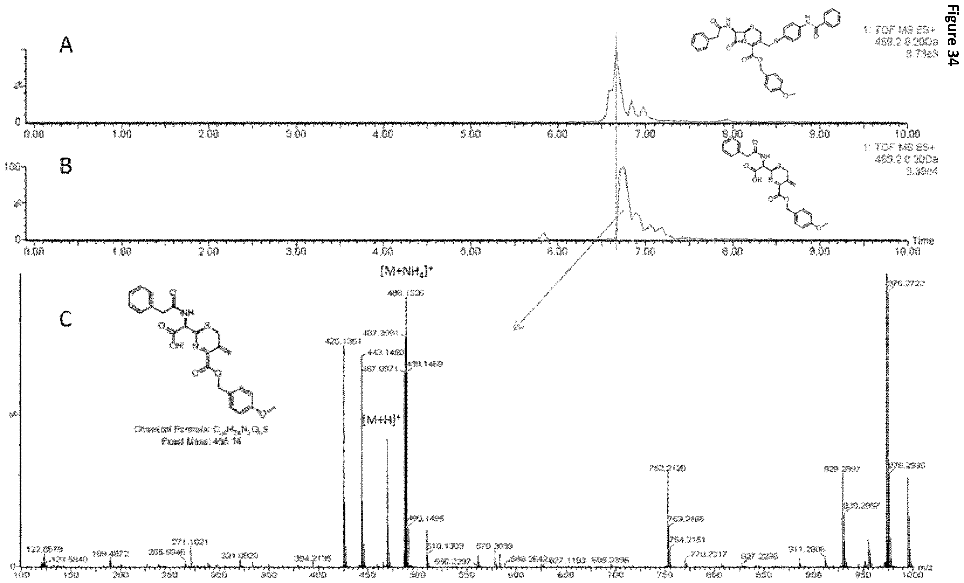

**Figure 34****.** LC-MS analysis of the β-iactamase hydrolysis reaction, showing the starting material and the hydrolyzed product.

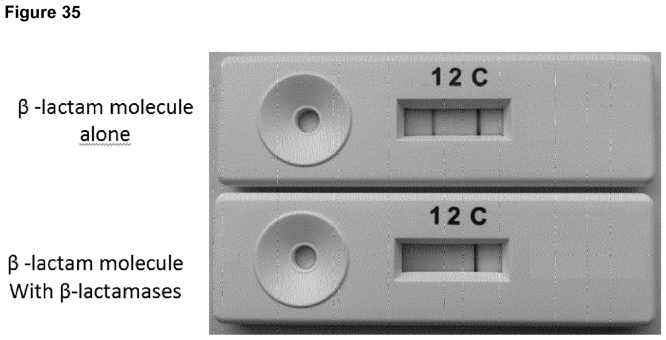

**Figure 35****.** Incorporation of the β-lactam responsive molecule into a lateral flow assay.

### Detailed description of the invention

### Definitions

In the context of the present invention "polynucleotide" is understood as oligonucleotide substrate (single or double stranded) with the capability to recognize a specific nuclease activity derived from bacteria or mammalian cells. The polynucleotide composition consists of natural nucleic acids (DNA and/or RNA), chemically modified nucleic acids or a combination of both.

In the context of the present invention "capture tag" is understood as the modification at the end of the "polynucleotide" that allows binding with the capture molecule and/or the reporter molecule. This binding is carried out by interaction of antigen-antibody or a similar biorecognition process.

In the context of the present invention "capture molecule" is understood as recognition molecule (e.g. biotin antibody) immobilized in the detection line of the lateral flow assay, with the capability to bind one of the ends of the polynucleotide probe that was previously modified with a capture tag (e.g. biotin).

In the context of the present invention "reporter molecule" is understood as a) the molecule that provides the readout of the system and consists of a latex/gold nanoparticle, carbon particle or a similar colorimetric molecule, functionalized with a capture molecule (e.g. antibody) that recognizes the polynucleotide probe via a capture tag. b) The reporter molecule consists of a latex/gold nanoparticle that is functionalized with the polynucleotide probe, at one of its ends by coupling chemistry (e.g. amine), while the other end is modified with a capture tag (e.g. biotin).

In the context of the present invention "lateral flow assay" is understood as a simple methodology to detect, in a specific manner, the presence or absence of a target analyte in a given matrix sample. This type of assay eliminates the need for specialized and costly equipment used in conventional laboratories. Lateral flow technology is used as point of care tool (fast and in situ), in a large range of applications, from the widely used home pregnancy test to more specialized tests for clinical, food safety, environmental and industrial applications.

In the context of the present invention "a backing card" is understood as the material where all the membranes (sample, conjugate, membrane and wicking pads) are mounted and connected to each other. At the same time, the backing card provides better stability and handling to the lateral flow device.

In the context of the present invention "a sample pad" is understood as the element of the device where the sample is introduced or deposited and acts as a sponge that holds the sample fluid. Subsequently, the sample fluid is transported to the membrane by capillary action.

In the context of the present invention "the conjugate pad" is understood as the part of the device where the reporter molecules are dispensed. The reporter molecules are immediately released from the conjugate pad upon contact with the sample fluid.

In the context of the present invention "a membrane" is understood as the material (e.g. nitrocellulose) that transports the sample and provides support to the capture molecules (antibodies, aptamers etc.) and allows or enhances their binding capabilities.

In the context of the present invention "a wicking pad" is understood as element in the system that retains all the assay material acting as a waste container.

In the context of the present invention "biological sample" is understood, as used in this invention, to be any sample of, relating to, caused by, or affecting life or living organisms, biological processes, such as growth and digestion. Examples of a biological sample may include, but are not limited to cell culture, cell culture supernatant, saliva, tears, urine, blood, serum, plasma, sweat, vaginal fluids, semen, feces, mucous, breast milk, ascites, lymph, pleural effusion, synovial fluid, bone marrow, cerebro-spinal fluid, and washings from bodily cavities (e.g., bronchial lavage), hair, tissue, bones, or teeth. Preferably, the biological sample is a liquid sample. Liquid means a state of matter with definite volume but no definite shape at 25° C., like water.

In the context of the present invention "test sample" refers to any material being assayed for nuclease activity or enzymatic activity and in certain embodiments, will be a liquid.

### Description

Identification of a given microorganism (e.g. *Staphylococcus aureus, Salmonella)* or cell type (e.g. breast cancer cell) is now possible taking advantage of the specific nuclease activity exerted by some microorganisms/cells. The identification and/or design of specific nucleic acid probes that are degraded by the target cell or microorganism but not by the rest of the microorganisms/cell types is critical.

In this sense, we have developed specific LFPC strips that can detect non-degraded polynucleotide probes in a given sample. Accordingly, the presence of a polynucleotide probe will give rise to a colored test line. On the other hand, if a specific nuclease is present in the sample it will eventually produce a complete degradation of the polynucleotide probe, and consequently, no test line will be seen.

Such system provides for a rapid and sensitive molecular approach for bacterial infections as well as for tumors/cancers, in particular for diagnosis, environmental and food safety analyses. For that purpose, the present invention provides for an *in vitro* use of a polynucleotide sequence susceptible of being cleaved by a specific nuclease derived from a bacteria or a mammalian cell, in a lateral flow assay.

In certain embodiments, the said polynucleotide sequence susceptible of being cleaved by a specific nuclease derived from a bacteria or a mammalian cell is a short oligonucleotide probe (substrate) composed of nucleic acids and flanked with a capture tag on one end and i) a reporter molecule or ii) a further capture tag capable of binding the reporter molecule located in the conjugate pad of a support of a lateral flow device, on the other end. Upon cleavage of the probes by nucleases (e.g., ribonuclease) in a sample, in particular in a biological sample, the capture tag is released away from the reporter molecule indicating the presence of said nucleases in a biological sample in a lateral flow device (see examples of the present invention). Depending on the type of probes, these can be cleaved by certain mammalian nucleases (in particular from tumor/cancer cells or tumoral tissues), and may also be cleaved by nucleases produced by various bacteria, including pathogenic bacteria such as *Staphylococcus aureus, Streptococcus pneumoniae* or *Salmonella.* The probes can thus be used to detect the presence of bacteria or tumoral tissue in biological samples such as body fluids (blood serum, urine, CSF, semen, sputum, saliva, etc.) and solid tissue homogenized, cell cultures, food, environmental and industrial samples and also *in vivo.*

The present invention thus relates to a method (first method of the invention) for detecting nuclease (e.g., RNase, DNase) activity in a test sample (biological sample), comprising: 1) incubating a synthetic Substrate or mixture of Substrates (from hereinafter "Substrate" or "substrate reagent") in the sample, for a time sufficient for cleavage of the Substrate(s) by a nuclease enzyme, wherein the Substrate(s) comprises a single-stranded (or double-stranded) nucleic acid molecule containing at least one ribonucleotide or deoxyribonucleotide or chemically modified nucleotide residue at an internal position that functions as a nuclease (e.g., ribonuclease) cleavage site (and in certain embodiments a 2'-fluoro modified purine or pyrimidine or 2'-O-methyl modified purine or pyrimidine that renders the oligonucleotide resistant to degradation by non-targeted nuclease activity), a capture tag on one end and i) a reporter molecule or ii) a further capture tag capable of binding the reporter molecule located in the conjugate pad of a support of a lateral flow device, on the other end, and 2) detecting the result, preferably visually, in particular whether the substrate has been cleaved or not by a test sample, by using a lateral flow device. Specific lateral flow devices useful to practice the present invention are illustrated through-out the description as well as in the examples and drawings.

The Substrate oligonucleotide used to practice the present invention thus comprises a capture tag at one end and i) a reporter molecule or ii) a further capture tag capable of binding the reporter molecule located in the conjugate pad of the support of the lateral flow device, at the other end. Cleavage of the Substrate with a nuclease (e.g., ribonuclease) enzyme leads to strand cleavage and physical separation of the capture group from the reporter group. Separation of reporter and capture eliminates the possibility of detecting a signal in the test line of the lateral flow device thus resulting in the detection of nuclease activity in the sample. The absence of the resulting signal can be detected by direct visual inspection of the lateral flow device.

Thus, the present invention is directed to the *in vitro* use of a polynucleotide sequence, or Substrate, susceptible of being cleaved by a specific nuclease derived from a bacteria or a mammalian cell, wherein the polynucleotide sequence is characterized by comprising a capture tag and a reporter molecule at each end of the sequence, for detecting the presence of a specific mammalian cell or bacteria in a biological sample by using a lateral flow device.

Interestingly, in one embodiment of the invention, we have found a way in which the above mentioned *in vitro* use of a lateral flow device can be used not only for detecting the presence of a specific bacteria in a biological sample but also for simultaneously determining the antibiotic susceptibility of said bacteria. In this sense, we have developed two approaches, i) Firstly, an innovative strip-based test able to detect bacteria such as *S*. *aureus* and its resistance to antibiotics such as methicillin, the most common causative bacteria of nosocomial respiratory infections, in less than an hour with the same specificity and sensitivity parameters as the bacteria quantitative culture, by using a synthetic Substrate or mixture of Substrates capable of detecting the presence of bacteria such as S. *aureus* in a biological sample, and establishing its resistance to antibiotics such as methicillin . The added diagnostic value of such test strip is represented by the innovative capability of simultaneously detecting bacteria and its resistance to methicillin (the most frequently used antibiotic). Due to its simple-but-effective strip format, the test can be performed easily *in situ* without the need for specialized lab equipment nor highly qualified personnel. The test can be easily performed by the healthcare giver or nurse at the hospital, by taking a biological sample such as an aspirate sample and mixing it with the reaction buffers and a nucleic acid probe, provided with the kit. Next, the reaction mixture is incubated for 30-45 min at room temperature. Subsequently, the strip is immersed into the reaction mixture, where the detection by lateral flow is carried out within 10 min. Finally, a positive (no line) or negative (line) reading can be visualized by naked eye, based on the presence or absence of target bacteria, along with its susceptibility to antibiotics. The second approach for detecting the presence of a specific bacterium in a biological sample and simultaneously determining the antibiotic susceptibility of said bacteria, is by using a further innovative strip-based test able to detect bacteria, in less than an hour with the same specificity and sensitivity parameters as the bacteria quantitative culture, in a method in which a synthetic Substrate or mixture of Substrates capable of detecting the presence of bacteria in a biological sample is used, and establishing its resistance to antibiotics, by using a β-iactamase responsive-molecule which comprises a compound comprising a β-lactam ring, preferably a β-lactam antibiotic selected from the list consisting of penicillin derivatives (penams), cephalosporins (cephems), monobactams, and carbapenems, more preferably methicillin, a capture tag on one end and i) a reporter molecule or ii) a further capture tag capable of binding the reporter molecule located in the conjugate pad of a support of a lateral flow device, on the other end (see figure 8)

In this sense, the present invention is further directed to a method (second method of the invention) for, preferably simultaneously, detecting bacteria and its resistance to beta-lactam antibiotics such as methicillin in a test sample, which comprises: 1) incubating in the test sample, i) a synthetic Substrate or mixture of Substrates for detecting the presence of a bacteria, and 2) simultaneously or subsequently establishing the resistance of the bacteria to beta-lactam antibiotics by incubating in the test sample i) a synthetic Substrate or mixture of Substrates capable of differentiating between antibiotic-resistant and antibiotic-non-resistant strains of the bacteria and/or ii) by incubating in the test sample a β-iactamase responsive-molecule, for a time sufficient for cleavage of the Substrate(s) by a nuclease enzyme and cleavage of the β-iactamase responsive-molecule by a β-lactamase; wherein
a. the Substrate(s) comprises a single-stranded nucleic acid molecule containing at least one ribonucleotide or deoxyribonucleotide or chemically modified nucleotide residue at an internal position that functions as a nuclease (e.g., ribonuclease) cleavage site (and in certain embodiments a 2'-fluoro modified purine or pyrimidine or 2'-O-methyl modified purine or pyrimidine that renders the oligonucleotide resistant to degradation by non-targeted nuclease activity), a capture tag on one end and i) a reporter molecule or ii) a further capture tag capable of binding the reporter molecule located in the conjugate pad of a support of a lateral flow device, on the other end; wherein
b. the β-iactamase responsive-molecule comprises a compound comprising a β-lactam ring, preferably a β-lactam antibiotic selected from the list consisting of penicillin derivatives (penams), cephalosporins (cephems), monobactams, and carbapenems, more preferably methicillin, a capture tag on one end and i) a reporter molecule or ii) a further capture tag capable of binding the reporter molecule located in the conjugate pad of a support of a lateral flow device, on the other end; and 2) visually detecting the result, in particular whether the substrate/s and optionally the β-lactamase responsive-molecule has been cleaved or not by a test sample, by using a lateral flow device; and wherein
the synthetic Substrate or mixture of Substrates for detecting the presence of a bacteria can be the same or different from the synthetic Substrate or mixture of Substrates capable of differentiating between antibiotic-resistant and antibiotic-non-resistant strains of the bacteria. Preferably, the result of the second method of the invention is, preferably visually, detected, in particular whether the substrate/s and optionally the β-iactamase responsive-molecule has been cleaved or not by a test sample, by using a lateral flow device as illustrated through-out the present specification or by binding the complexes comprising the substrate/s and optionally the β-iactamase responsive-molecule after the incubation to a solid support that facilitates immunoassay techniques such as enzyme linked immuno-sorbent assay (ELISA), a radioimmunoassay (RIA), an immuno radiometric assay (IRMA), a fluorescent immunoassay (FIA), a chemiluminescent immunoassay (CLIA), magnetic beads-based immunoassay (MBI), or an electro-chemiluminescent immunoassay (ECL).

It is important to note that in order to correctly detect whether the substrate and β-lactamase responsive-molecule have been cleaved or not by a test sample by using a lateral flow device, the capture tag (for binding the substrate to the test line of the LFA) used in the Substrate must be different from the capture tag (for binding the β-iactamase responsive-molecule to the β-iactamase test line of the LFA) used in the tag β-iactamase responsive-molecule. In this way the bacteria test line and the β-iactamase test line (see figure 8) can be clearly differentiated when performing the second method of the invention.

A particular example of carrying-out the second method of the invention by using the specific lateral flow devices illustrated in figure 8 is detailed as follows. In this sense, we use the second method of the invention in the innovative strip of figure 8 to detect S. *aureus* in endotracheal aspirates and sputum samples. For that purpose, we used nucleic acid probes susceptible of being cleaved by the specific nuclease derived from a S. *aureus* bacterial infection. The nucleic acid probe (substrate) was composed of a short polynucleotide sequence and flanked with two capture tags (in this specific case Digoxigenin "Dx or Tag#1" and Biotin "B or Tag#2"). The Digoxigenin tag bound a reporter molecule (Latex particle or carbon particles) previously functionalized with anti-digoxigenin and located in the conjugate pad of the strip (see figure 8). On the other hand, the biotin or Tag#2 captured the nucleic acid probe in the test line of the strip (red line for negative and no line for positive). Upon cleavage of the nucleic acid probe by nucleases derived from a clinical sample, the Digoxigenin tag was released away from the reporter molecule indicating the presence of said nucleases in the biological samples in the lateral flow device. In the absence of the targeted bacteria, the detection complex would have remained uncleaved and would have been visualized as a red line in the strip. Simultaneously to the nucleic acid probes susceptible of being cleaved, we used the novel antibiotic substrate with responsive capabilities that allows the detection of β-lactamases based on specific cleavage, in a similar fashion to the nucleic acid probe.

Examples of antibiotic substrates with responsive capabilities that allows the detection of β-lactamases are identified as follows: 4-methoxybenzyl (6R,7R)-3-(((4-benzamidophenyl)thio)methyl)-8-oxo-7-(2phenylacetamido)-5-thia-1-azabicyclo[4.2.0]oct-2-ene-2-carboxylate is a truncated β-lactam responsive molecule (CEProbe), or (6*S*,7*S*)-7-(((3',6'-dihydroxy-3-oxo-3*H*-spiro[isobenzofuran-1,9'-xanthen]-6-ylcarbonyl)oxy)amino)-8-oxo-3-(((4-(5-((3a*R*,4*R*,6a*S*)-2-oxohexahydro-1*H*-thieno[3,4-*d*]imidazol-4-yl)pentanamido)phenyl)thio)methyl)-5-thia-1- azabicyclo[4.2.0]oct-2-ene-2-carboxylic acid (FAM-CEProbe-Biotin, or 6*S*,7*S*)-7-(5-(((3*S*,5*R*,8*R*,9*S*,10*S*,12*R*,13*S*,14*S*,17*R*)-12,14-dihydroxy-10,13-dimethyl-17-(5-oxo-2,5-dihydrofuran-3-yl)hexadecahydro-1*H*-cyclopenta[*a*]phenanthren-3-yl)oxy)-5-oxopentanamido)-8-oxo-3-(((4-(5-((3a*R*,4*R*,6a*S*)-2-oxohexahydro-1*H*-thieno[3,4-*d*]imidazol-4-yl)pentanamido)phenyl)thio)methyl)-5-thia-1-azabicyclo[4.2.0]oct-2-ene-2-carboxylic acid (Digoxigenin-CEProbe-Biotin), or similar molecules that act as a substrate for by β -lactamases (BL). Digoxigenin-CEProbe-Biotin is described in figure 9 and herein below:

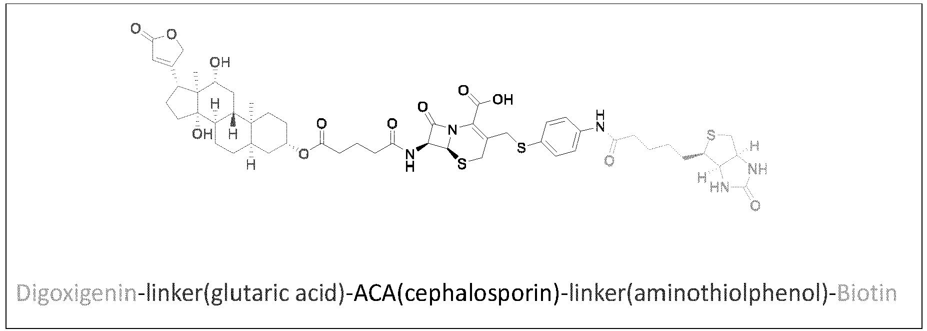

The synthesis of the above molecule is detailed in figure 9 and herein below

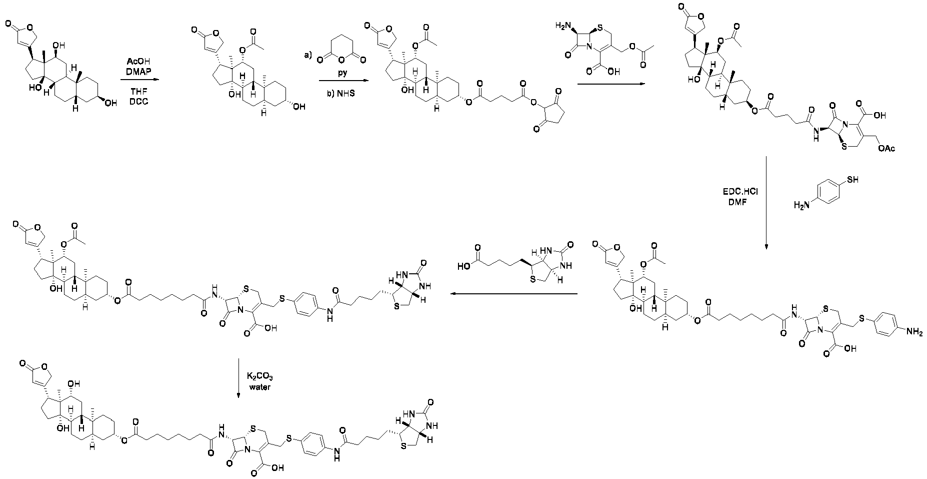

It is important to note that in one aspect of the present invention, both the above molecule and its method of synthesis constitute a part of the present invention. As illustrated above, the molecule is composed of a Tag#1 or Digoxigenin, a linker (glutaric acid), and given antibiotic, a leaving group (aminothiolphenol), and second capture tag (Tag#3), see Figure 9. The Digoxigenin tag bound a reporter molecule (Latex particle) previously functionalized with anti-digoxigenin and located in the conjugate pad of the strip (see figure 8). On the other hand, the Tag#3 captured the β-iactamase responsive-molecule in the β-iactamase test line of the strip (red line for negative and no line for positive). Upon cleavage of the β-iactamase responsive-molecule by β-lactamases derived from a clinical sample, the Digoxigenin tag was released away from the β-iactamase responsive-molecule indicating the presence of said nucleases in the biological samples in the lateral flow device. In the absence of said β-lactamases, the detection complex would have remained uncleaved and would have been visualized as a red line in the strip indicating the sensitivity of the bacteria to β-lactam antibiotics.

It is further noted that, in the second method of the invention, simultaneously to the nucleic acid probes susceptible of being cleaved for determining the presence and the type of bacteria, it is not mandatory to use the antibiotic substrate with responsive capabilities that allows the detection of β-lactamases based on specific cleavage, but such antibiotic resistance could also be determined by using nucleic acid probes susceptible of being cleaved only for antibiotic resistant strains of the bacteria, such as MRSA, and not by antibiotic-sensistive bacteria, such as MSSA (please refer to figure 31).

In one embodiment, the Substrate used to practice the first or second method of the invention to identify the presence and the type of bacteria, is an oligonucleotide comprising ribonucleotide residues. The Substrate can also be a chimeric oligonucleotide comprising RNase-cleavable, e.g., RNA, residues, or modified RNase-resistant RNA residues. Preferably, Substrate composition is such that cleavage is a ribonuclease-specific event and that cleavage by enzymes that are strictly deoxyribonucleases does not occur.

Preferably, the Substrate is a chimeric oligonucleotide comprising ribonucleotide residue(s) and modified ribonucleotide residue(s). In one embodiment, the Substrate is a chimeric oligonucleotide comprising ribonucleotide residues and 2'-O-methyl ribonucleotide residues. In one embodiment, the synthetic Substrate is a chimeric oligonucleotide comprising 2'-O-methyl ribonucleotide residues and one or more of each of the four ribonucleotide residues, adenosine, cytosine, guanosine, and uridine. Inclusion of the four distinct ribonucleotide bases in a single Substrate allows for detection of an increased spectrum of ribonuclease enzyme activities by a single Substrate oligonucleotide.

Preferably, the Substrate is an oligonucleotide comprising deoxyribonucleotide residues. The synthetic Substrate can also be a chimeric oligonucleotide comprising DNase-cleavable, e.g., DNA, residues, or modified DNase-resistant DNA residues. Preferably, Substrate composition is such that cleavage is a deoxyribonuclease-specific event and that cleavage by enzymes that are strictly ribonucleases does not occur.

Preferably, the Substrate is a chimeric oligonucleotide comprising deoxyribonucleotide residue(s) and modified deoxyribonucleotide residue(s). In one embodiment, the synthetic Substrate is a chimeric oligonucleotide comprising deoxyribonucleotide residues and 2'-O-methyl ribonucleotide residues. In one embodiment, the synthetic Substrate is a chimeric oligonucleotide comprising 2'-O-methyl deoxyribonucleotide residues and one or more of each of the four deoxyribonucleotide residues, deoxyadenosine, deoxycytosine, deoxyguanosine, and deoxythymidine. Inclusion of the four distinct deoxyribonucleotide bases in a single Substrate allows for detection of an increased spectrum of deoxyribonuclease enzyme activities by a single Substrate oligonucleotide.

In another embodiment, the oligonucleotide of the Substrate is selected from the list consisting of SEQ ID No 1-2 (S. aureus), SEQ ID No 3 (control probe), SEQ ID No 4-11 (Salmonella).

| **Num.** | **name** | **Sequence** | **Target** |
|---|---|---|---|
| 1 | TT S.aureus | mCmUmCmGTTmCmGmUmUmC | S. aureus |
| 2 | TT -Um S. aurues | mUmUmUmUTTmUmUmUmUmU | S. aureus |
| 3 | Control probe | mUmCmUmCmGmUmAmCmGmUmUmC | No target |
| 4 | Salmo1.1 | mUmUmUAmUmUAmUmUmUAmU | Salmonella |
| 5 | Salmo1.2 | mUmUmUAmUmUmUmUmUmUmUmU | Salmonella |
| 6 | Salmo1.3 | mUmUmUmUmUmUAmUmUmUmUmU | Salmonella |
| 7 | Salmo1.4 | mUmUmUmUmUmUmUmUmUmUAmU | Salmonella |
| 8 | Salmo1.5 | mUmUmUAAmUmUmUmUmUmUmUmU | Salmonella |
| 9 | Salmo1.6 | mUmUmUmUmUmUAAmUmUmUmUmU | Salmonella |
| 10 | Salmo1.7 | mUmUmUmUmUmUmUmUmUmUAAmU | Salmonella |
| 11 | Salmo1.8 | mUAmUAmUAmU | Salmonella |

In another embodiment, the oligonucleotide of the Substrate is selected from the list consisting of SEQ ID No 12 and is useful for the detection of breast normal tissue.

In another embodiment, the oligonucleotide of the Substrate is selected from the list consisting of SEQ ID No 13 and is useful for the detection of breast normal tissue.

In another embodiment, the oligonucleotide of the Substrate is selected from the list consisting of SEQ ID No 14 and is useful for the detection of breast normal tissue.

| **Num.** | **Name** | **Sequece** | **target** |
|---|---|---|---|
| 12 | RNA | ucucguacguuc | breast normal tissue |
| 13 | Pur 2'-F RNA | ufAfAcfGufAcfGfGuc | breast normal tissue |
| 14 | Pur 2'-OMe RNA | umAmAcmGumAcmGmGuc | breast normal tissue |

In another embodiment, the oligonucleotide of the Substrate is selected from the list consisting of SEQ ID No 15 and is useful for the detection of breast cancer tumors.

In another embodiment, the oligonucleotide of the Substrate is selected from the list consisting of SEQ ID No 16 and is useful for the detection of breast cancer tumors.

In another embodiment, the oligonucleotide of the Substrate is selected from the list consisting of SEQ ID No 17 and is useful for the detection of breast cancer tumors.

In another embodiment, the oligonucleotide of the Substrate is selected from the list consisting of SEQ ID No 18 and is useful for the detection of breast cancer tumors.

In another embodiment, the oligonucleotide of the Substrate is selected from the list consisting of SEQ ID No 19 and is useful for the detection of breast cancer tumors.

In another embodiment, the oligonucleotide of the Substrate is selected from the list consisting of SEQ ID No 20 and is useful for the detection of breast cancer tumors.

| **Num.** | **Name** | **sequence** | **target** |
|---|---|---|---|
| 15 | DNA | TCTCGTACGTTC | breast tumoral tissue |
| 16 | Pyr 2'-F DNA | fUfCfUfCGfUAfCGfUfUfC | breast tumoral tissue |
| 17 | Pur 2'-F DNA | TfAfACfGTfACfGfGTC | breast tumoral tissue |
| 18 | Poly A | fAfAfAfAfAfAfAfAfAfAfAfA | breast tumoral tissue |
| 19 | AAA CCC chi | mUmCmUfAfAfAmCmUmCfCfCfCmUmCmU | breast tumoral tissue |
| 20 | AAA UUU chi | mUmCmUfAfAfAmCmUmCfUfUfUmUmCmU | breast tumoral tissue |

In another embodiment the oligonucleotide of the Substrate is selected from any of the sequences of the list consisting of SEQ ID No 21 to 145.

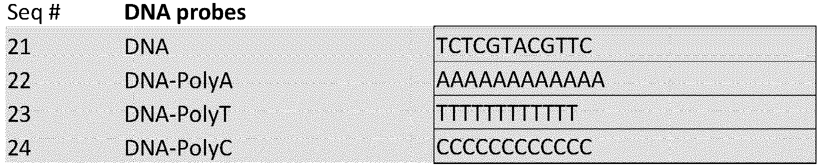

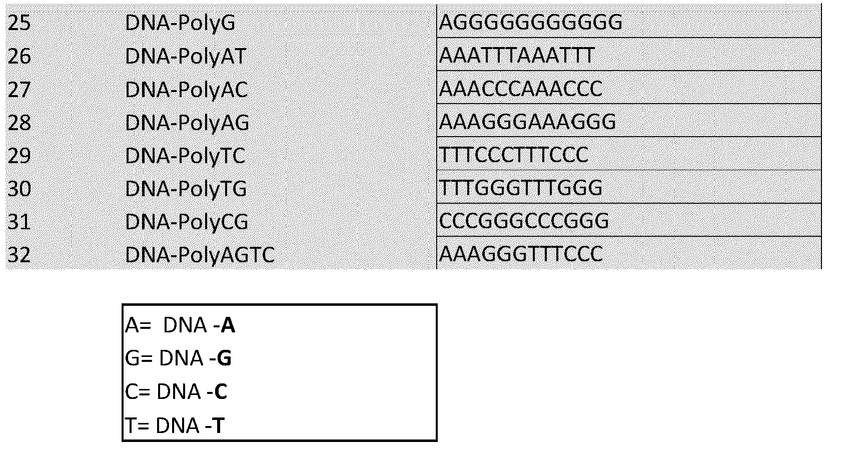

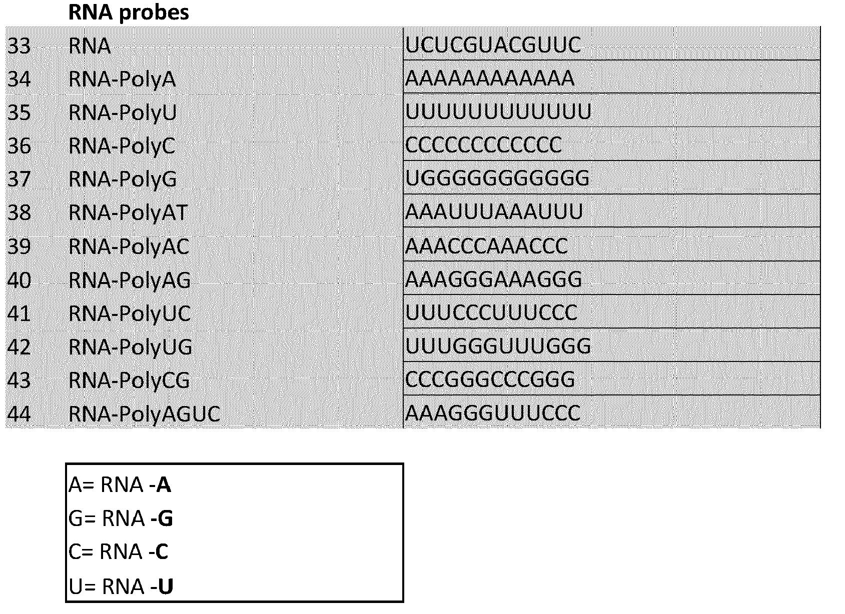

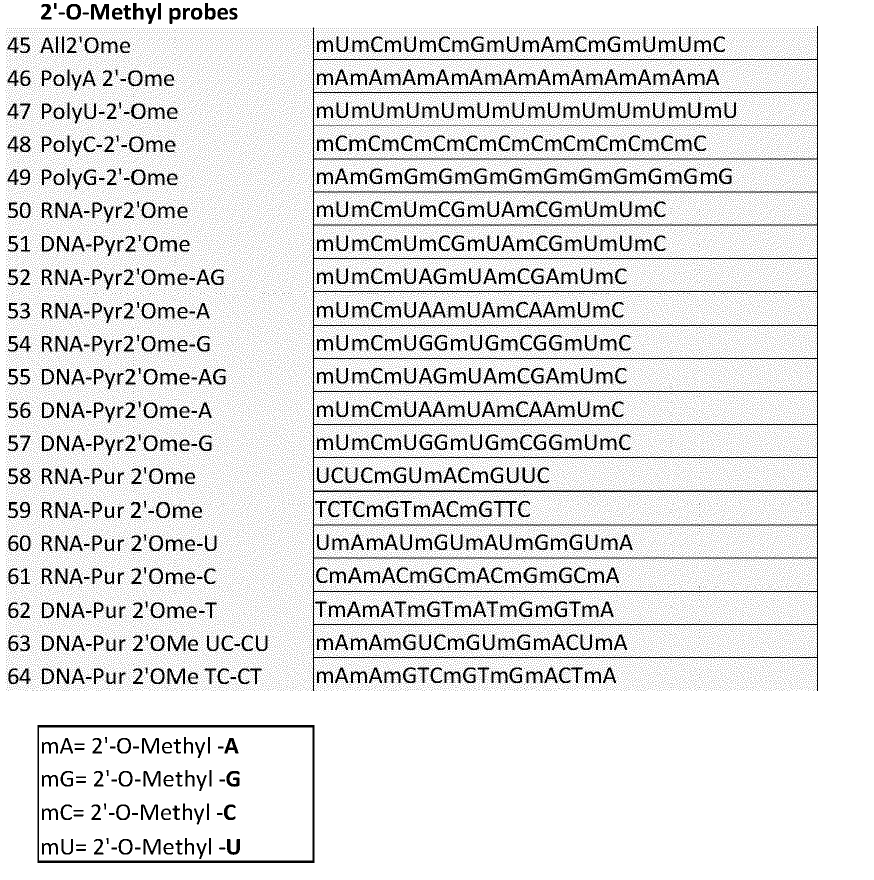

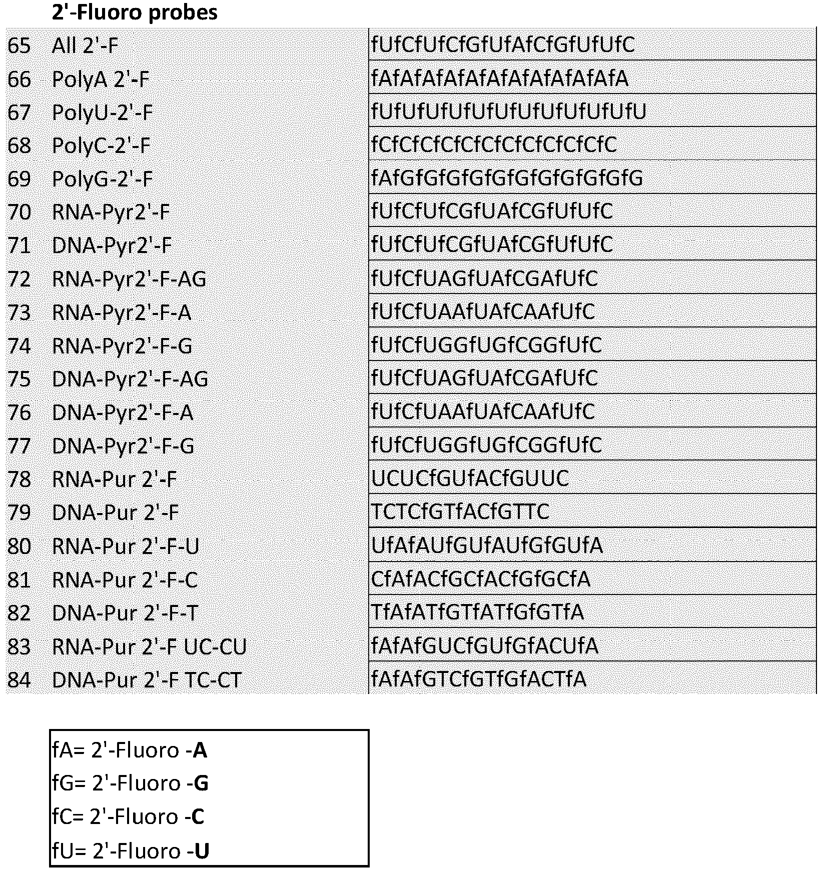

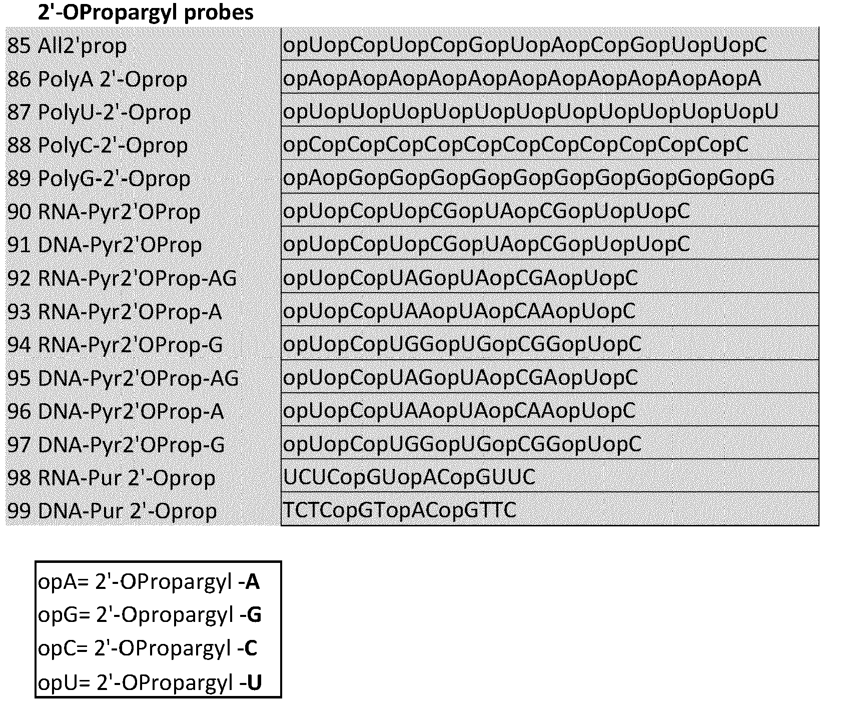

### Second generation of probes

**Streptococcus pneumoniae probes**

| | | |
|---|---|---|
| 100 | 2'F UA - 2'Ome U | mUmUmUmU**fUfA**mUmUmUmU |
| 101 | 2'F AU - 2'Ome U | mUmUmUmU**fAfU**mUmUmUmU |
| 102 | 2'F UAU - 2'Ome U | mUmUmUmU**fUfAfU**mUmUmUmU |
| 103 | 2'F AUA - 2'Ome U | mUmUmUmU**fAfUfA**mUmUmUmU |
| 104 | 2'F UAAU - 2'Ome U | mUmUmUmU**fUfAfAfU**mUmUmUmU |
| 105 | 2'F AUUA - 2'Ome U | mUmUmUmU**fAfUfUfA**mUmUmUmU |
| | | |
| | fA= 2'-Fluoro **-A** | |
| | fU= 2'-Fluoro **-U** | |
| | mU= 2'-O-Methyl **-U** | |

**Klebsiella pneumoniae probes**

| | | |
|---|---|---|
| 106 | RNA UU - 2'Ome U | mUmUmUmU**UU**mUmUmUmU |
| 107 | RNA CC - 2'Ome U | mUmUmUmU**CC**mUmUmUmU |
| 108 | RNA AA - 2'Ome U | mUmUmUmU**AA**mUmUmUmU |
| 109 | RNACUC - 2'Ome U | mUmUmUmU**CUC**mUmUmUmU |
| 110 | RNA UCU - 2'Ome U | mUmUmUmU**UCU**mUmUmUmU |
| 111 | RNA ACA - 2'Ome U | mUmUmUmU**ACA**mUmUmUmU |
| 112 | RNA CAC - 2'Ome U | mUmUmUmU**CAC**mUmUmUmU |
| 113 | RNA AU - 2'Ome U | mUmUmUmU**AU**mUmUmUmU |
| 114 | RNA UA - 2'Ome U | mUmUmUmU**UA**mUmUmUmU |
| 115 | RNA AC - 2'Ome U | mUmUmUmU**AC**mUmUmUmU |
| 116 | RNA CA - 2'Ome U | mUmUmUmU**CA**mUmUmUmU |
| | | |
| | A= RNA -**A** | |
| | C= RNA -**C** | |
| | U= RNA **-U** | |
| | mU= 2'-O-Methyl **-U** | |

**Pseudomona aeruginosa probes**

| | | |
|---|---|---|
| 117 | 2'F CAC - 2'Ome U | mUmUmUmU**fCfAfC**mUmUmUmU |
| 118 | 2'F ACA - 2'Ome U | mUmUmUmU**fAfCfA**mUmUmUmU |
| 119 | 2'F CCC - 2'Ome U | mUmUmUmU**fCfCfC**mUmUmUmU |
| 120 | 2'F AAA - 2'Ome U | mUmUmUmU**fAfAfA**mUmUmUmU |
| 121 | 2'F fC A fC - 2'Ome U | mUmUmUmU**fAAfA**mUmUmUmU |
| 122 | 2'F A fC A - 2'Ome U | mUmUmUmU**AfCA**mUmUmUmU |
| | | |
| | A= RNA -**A** | |
| | fA= 2'-Fluoro **-A** | |
| | fC= 2'-Fluoro **-C** | |
| | mU= 2'-O-Methyl **-U** | |

**Proteus mirabilis probes**

| | | |
|---|---|---|
| 123 | RNA GG - 2'Ome U | mUmUmUmU**GG**mUmUmUmU |
| 124 | RNA GGG - 2'Ome U | mUmUmUmU**GGG**mUmUmUmU |
| 125 | RNA AGA - 2'Ome U | mUmUmUmU**AGA**mUmUmUmU |
| 126 | RNA GAG - 2'Ome U | mUmUmUmU**GAG**mUmUmUmU |
| 127 | RNA AG - 2'Ome U | mUmUmUmU**AG**mUmUmUmU |
| 128 | RNA GA - 2'Ome U | mUmUmUmU**GA**mUmUmUmU |
| 129 | 2'-F GG - 2'Ome U | mUmUmUmU**fGfG**mUmUmUmU |
| 130 | 2'-F GGG - 2'Ome U | mUmUmUmU**fGfGfG**mUmUmUmU |
| 131 | 2'-F AGA - 2'Ome U | mUmUmUmU**fAfGfA**mUmUmUmU |
| 132 | 2'-F GAG - 2'Ome U | mUmUmUmU**fGfAfG**mUmUmUmU |
| 133 | 2'-F AG - 2'Ome U | mUmUmUmU**fAfG**mUmUmUmU |
| 134 | 2'-F GA - 2'Ome U | mUmUmUmU**fGfA**mUmUmUmU |
| | | |
| | A= RNA -**A** | |
| | G= RNA **-G** | |
| | fA= 2'-Fluoro **-A** | |
| | fG= 2'-Fluoro **-G** | |
| | mU= 2'-O-Methyl **-U** | |

**S. epidermidis probes**

| | | |
|---|---|---|
| 135 | RNA AUA - 2'Ome U | mUmUmUmU**AUA**mUmUmUmU |
| 136 | RNA UAU - 2'Ome U | mUmUmUmU**UAU**mUmUmUmU |
| 137 | RNA AAA - 2'Ome U | mUmUmUmU**AAA**mUmUmUmU |
| 138 | RNA UUU - 2'Ome U | mUmUmUmU**UUU**mUmUmUmU |
| 139 | RNA UU - 2'Ome U | mUmUmUm**UUU**mUmUmUmU |
| 140 | RNA AA - 2'Ome U | mUmUmUmU**AA**mUmUmUmU |
| | | |
| | A= RNA -**A** | |
| | U= RNA **-U** | |
| | mU= 2'-O-Methyl **-U** | |

**S. aureus probes**

| 141 | TT DNA probe **(parental)** | mCmUmCmGTTmGmUmUmC |
|---|---|---|
| 142 | TT DNA probe UG 2'Ome | mUmUmUmUmUTTmGmGmGmGmG |
| 143 | TT DNA probe CG 2'Ome | mCmCmCmCmCTTmGmGmGmGmG |
| 144 | TT DNA probe C 2'Ome | mCmCmCmCmCTTmCmCmCmCmC |
| 145 | TT DNA probe UG 2'Ome | mUmGmGmGmGTTmGmGmGmGmG |
| | | |
| | T= DNA -**T** | |
| | mC= 2'-O-Methyl **-C** | |
| | mG= 2'-O-Methyl **-G** | |
| | mU= 2'-O-Methyl **-U** | |

In yet another embodiment the oligonucleotide of the Substrate is selected from the list consisting of ay of the probes identified below (the particular nucleotide sequence for each of these probes is identified in the tables describing sequences 1 to 145).

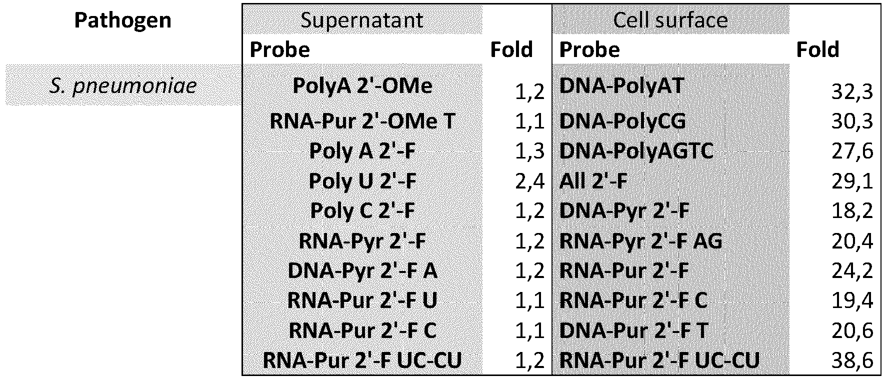

The probes identified in the table above, are particularly useful for detecting S. *pneumoniae* in a test sample according to the first or second method of the invention (see figure 10). Consequently, the present invention is further directed to the first or second method for detecting bacteria of the invention, wherein the bacteria is S. *pneumoniae* and wherein the method is preferably carry-out by using one or more of the probes capable of detecting the presence of bacteria in suspension in the test sample.

In yet another embodiment the oligonucleotide of the Substrate is selected from the list consisting of ay of the probes identified below (the particular nucleotide sequence for each of these probes is identified in the tables describing sequences 1 to 145).

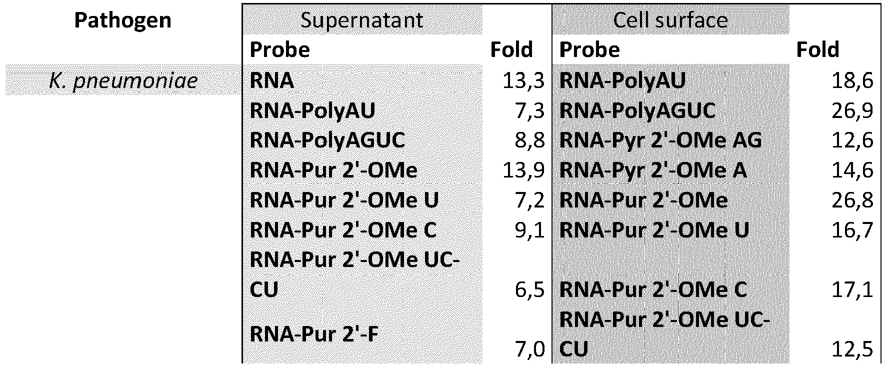

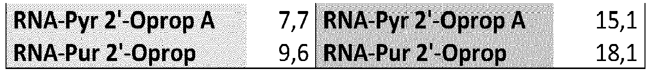

The probes identified in the table above are particularly useful for detecting *K. pneumoniae* in a test sample according to the first or second method of the invention (see figure 11). Consequently, the present invention is further directed to the first or second method for detecting bacteria of the invention, wherein the bacteria is *K. pneumoniae* and wherein the method is preferably carry-out by using one or more of the probes capable of detecting the presence of bacteria in suspension in the test sample and/or wherein the method is preferably carry-out by using one or more of the probes capable of indirectly detecting the presence of the bacteria by detecting the nucleases secreted in the test sample.

In yet another embodiment the oligonucleotide of the Substrate is selected from the list consisting of ay of the probes identified below (the particular nucleotide sequence for each of these probes is identified in the tables describing sequences 1 to 145).

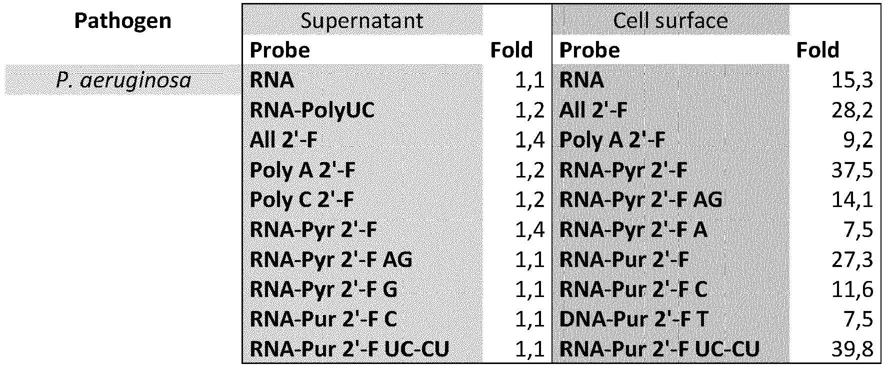

The probes identified in the table above are particularly useful for detecting *P. aeruginosa* in a test sample according to the first or second method of the invention (see figure 12). Consequently, the present invention is further directed to the first or second method for detecting bacteria of the invention, wherein the bacteria is *P. aeruginosa* and wherein the method is preferably carry-out by using one or more of the probes capable of detecting the presence of bacteria in suspension in the test sample.

In yet another embodiment the oligonucleotide of the Substrate is selected from the list consisting of ay of the probes identified below (the particular nucleotide sequence for each of these probes is identified in the tables describing sequences 1 to 145).

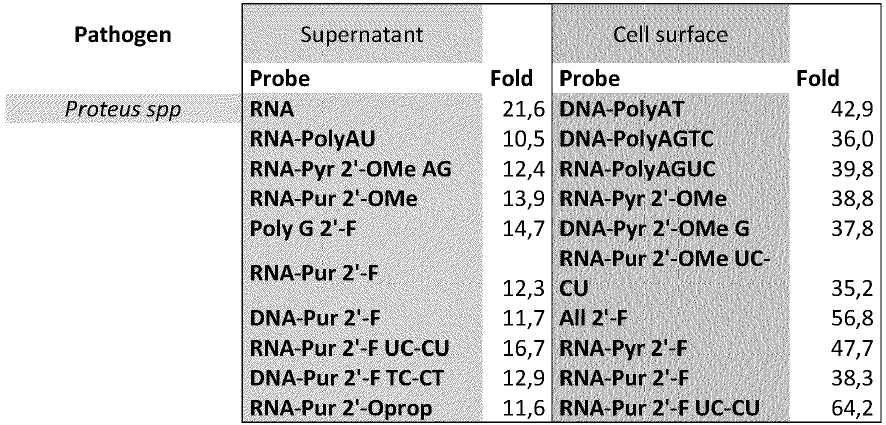

The probes identified in the table above are particularly useful for detecting *Proteus spp* in a test sample according to the first or second method of the invention (see figure 13). Consequently, the present invention is further directed to the first or second method for detecting bacteria of the invention, wherein the bacteria is *Proteus spp* and wherein the method is preferably carry-out by using one or more of the probes capable of detecting the presence of bacteria in suspension in the test sample and/or wherein the method is preferably carry-out by using one or more of the probes capable of indirectly detecting the presence of the bacteria by detecting the nucleases secreted in the test sample.

In yet another embodiment the oligonucleotide of the Substrate is selected from the list consisting of ay of the probes identified below (the particular nucleotide sequence for each of these probes is identified in the tables describing sequences 1 to 145).

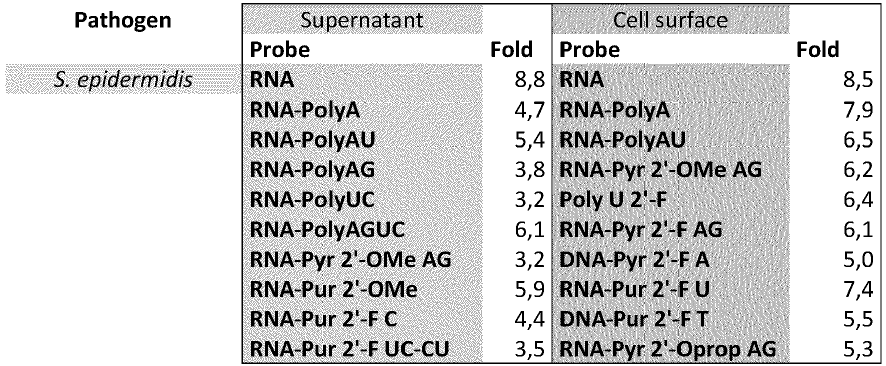

The probes identified in the table above are particularly useful for detecting S. *epidermidis* in a test sample according to the first or second method of the invention (see figure 14). Consequently, the present invention is further directed to the first or second method for detecting bacteria of the invention, wherein the bacteria is *S. epidermidis* and wherein the method is preferably carry-out by using one or more of the probes capable of detecting the presence of bacteria in suspension in the test sample and/or wherein the method is preferably carry-out by using one or more of the probes capable of indirectly detecting the presence of the bacteria by detecting the nucleases secreted in the test sample.

In yet another embodiment the oligonucleotide of the Substrate is selected from the list consisting of ay of the probes identified below (the particular nucleotide sequence for each of these probes is identified in the tables describing sequences 1 to 145).

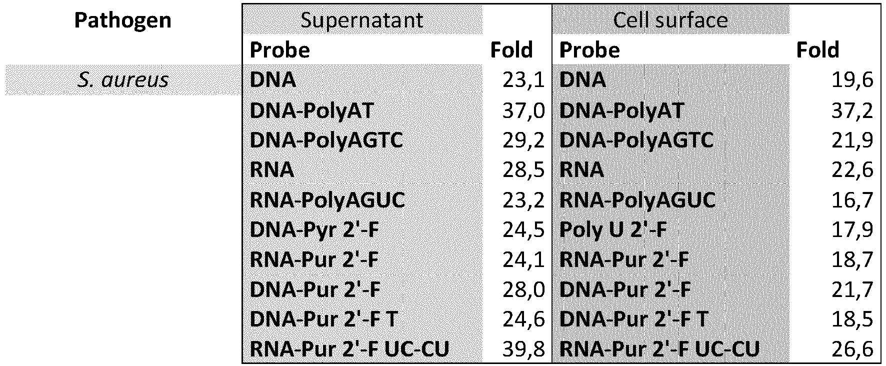

The probes identified in the table above are particularly useful for detecting *S. aureus* in a test sample according to the first or second method of the invention (see figure 15). Consequently, the present invention is further directed to the first or second method for detecting bacteria of the invention, wherein the bacteria is *S. aureus* and wherein the method is preferably carry-out by using one or more of the probes capable of detecting the presence of bacteria in suspension in the test sample and/or wherein the method is preferably carry-out by using one or more of the probes capable of indirectly detecting the presence of the bacteria by detecting the nucleases secreted in the test sample.

In yet another embodiment the oligonucleotide of the Substrate is selected from the list consisting of ay of the probes identified below (the particular nucleotide sequence for each of these probes is identified in the tables describing sequences 1 to 145).

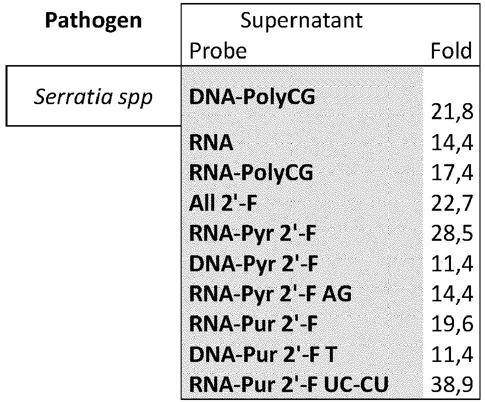

The probes identified in the table above are particularly useful for detecting *Serratia spp* in a test sample according to the first or second method of the invention (see figure 16). Consequently, the present invention is further directed to the first or second method for detecting bacteria of the invention, wherein the bacteria is *Serratia spp* and wherein the method is preferably carry-out by one or more of the above probes capable of indirectly detecting the presence of the bacteria by detecting the nucleases secreted in the test sample.

In yet another embodiment the oligonucleotide of the Substrate is selected from the list consisting of ay of the probes identified below (the particular nucleotide sequence for each of these probes is identified in the tables describing sequences 1 to 145).

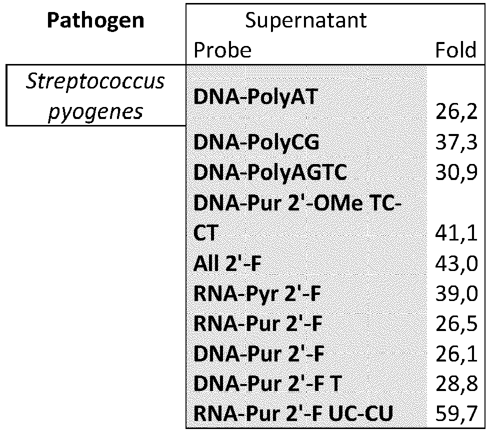

The probes identified in the table above are particularly useful for detecting *Streptococcus pyogenes* in a test sample according to the first or second method of the invention (see figure 17). Consequently, the present invention is further directed to the first or second method for detecting bacteria of the invention, wherein the bacteria is *Streptococcus pyogenes* and wherein the method is preferably carry-out by one or more of the above probes capable of indirectly detecting the presence of the bacteria by detecting the nucleases secreted in the test sample.

In yet another embodiment the oligonucleotide of the Substrate is selected from the list consisting of ay of the probes identified below (the particular nucleotide sequence for each of these probes is identified in the tables describing sequences 1 to 145).

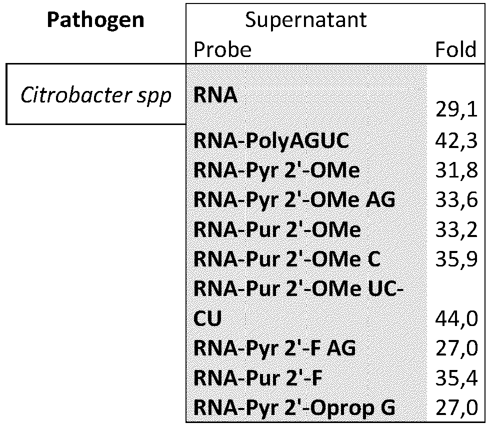

The probes identified in the table above are particularly useful for detecting *Citrobacter spp* in a test sample according to the first or second method of the invention (see figure 18). Consequently, the present invention is further directed to the first or second method for detecting bacteria of the invention, wherein the bacteria is *Citrobacter spp* and wherein the method is preferably carry-out by one or more of the above probes capable of indirectly detecting the presence of the bacteria by detecting the nucleases secreted in the test sample.

In yet another embodiment the oligonucleotide of the Substrate is selected from the list consisting of ay of the probes identified below (the particular nucleotide sequence for each of these probes is identified in the tables describing sequences 1 to 145).

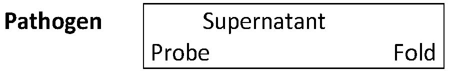

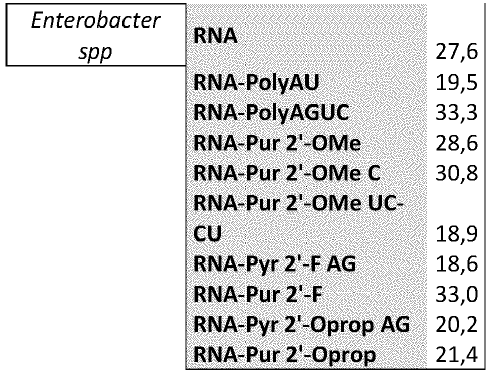

The probes identified in the table above are particularly useful for detecting *Enterobacter spp* in a test sample according to the first or second method of the invention (see figure 19). Consequently, the present invention is further directed to the first or second method for detecting bacteria of the invention, wherein the bacteria is *Enterobacter spp* and wherein the method is preferably carry-out by one or more of the above probes capable of indirectly detecting the presence of the bacteria by detecting the nucleases secreted in the test sample.

In yet another embodiment the oligonucleotide of the Substrate is selected from the list consisting of ay of the probes identified below (the particular nucleotide sequence for each of these probes is identified in the tables describing sequences 1 to 145).

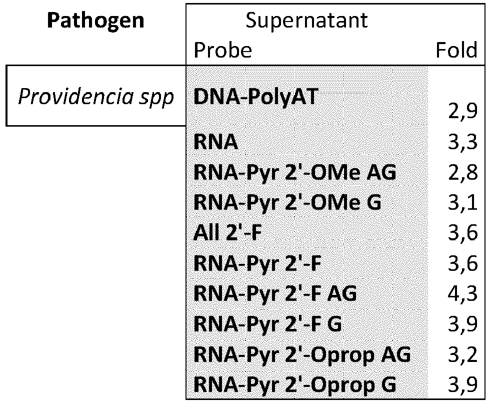

The probes identified in the table above are particularly useful for detecting *Providencia spp* in a test sample according to the first or second method of the invention (see figure 20). Consequently, the present invention is further directed to the first or second method for detecting bacteria of the invention, wherein the bacteria is *Providencia spp* and wherein the method is preferably carry-out by one or more of the above probes capable of indirectly detecting the presence of the bacteria by detecting the nucleases secreted in the test sample.

In yet another embodiment the oligonucleotide of the Substrate is selected from the list consisting of any of the probes identified below (the particular nucleotide sequence for each of these probes is identified in the tables describing sequences 1 to 145).

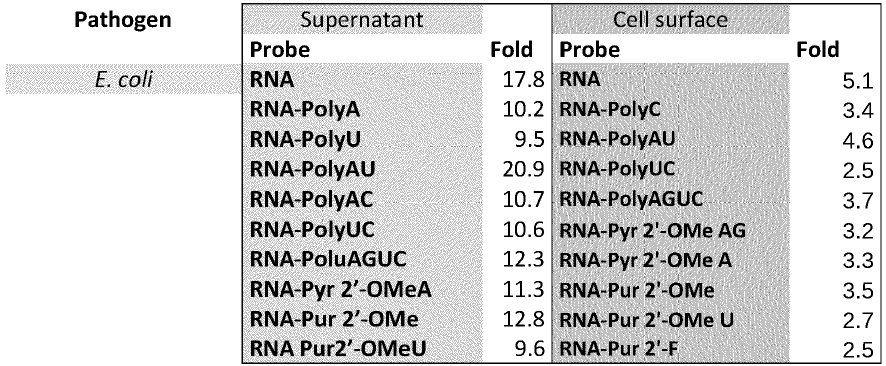

The probes identified in the table above are particularly useful for detecting *E. coli* in a test sample according to the first or second method of the invention (see figure 21). Consequently, the present invention is further directed to the first or second method for detecting bacteria of the invention, wherein the bacteria is *E. coli* and wherein the method is preferably carry-out by using one or more of the probes capable of detecting the presence of bacteria in suspension in the test sample and/or wherein the method is preferably carry-out by using one or more of the probes capable of indirectly detecting the presence of the bacteria by detecting the nucleases secreted in the test sample.

In yet another embodiment the oligonucleotide of the Substrate is selected from the list consisting of ay of the probes identified below (the particular nucleotide sequence for each of these probes is identified in the tables describing sequences 1 to 145).

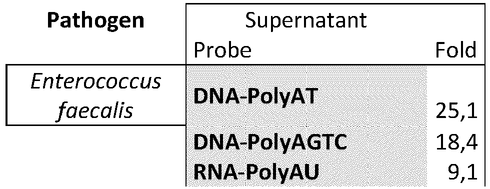

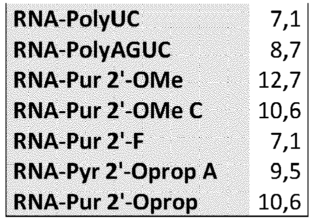

The probes identified in the table above are particularly useful for detecting *Enterococcus faecalis* in a test sample according to the first or second method of the invention (see figure 22). Consequently, the present invention is further directed to the first or second method for detecting bacteria of the invention, wherein the bacteria is *Enterococcus faecalis* and wherein the method is preferably carry-out by one or more of the above probes capable of indirectly detecting the presence of the bacteria by detecting the nucleases secreted in the test sample.

In yet another embodiment the oligonucleotide of the Substrate is selected from the list consisting of ay of the probes identified below (the particular nucleotide sequence for each of these probes is identified in the tables describing sequences 1 to 145).

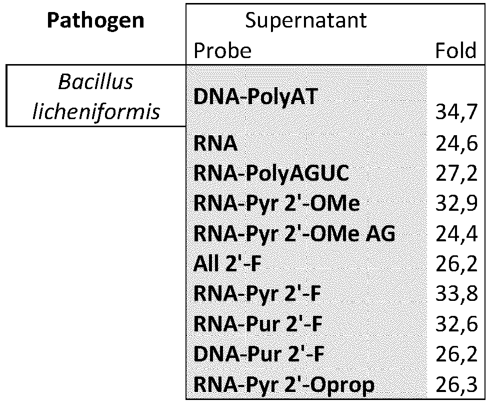

The probes identified in the table above are particularly useful for detecting *Bacillus licheniformis* in a test sample according to the first or second method of the invention (see figure 23). Consequently, the present invention is further directed to the first or second method for detecting bacteria of the invention, wherein the bacteria is *Bacillus licheniformis* and wherein the method is preferably carry-out by one or more of the above probes capable of indirectly detecting the presence of the bacteria by detecting the nucleases secreted in the test sample.

In yet another embodiment the oligonucleotide of the Substrate is selected from the list consisting of ay of the probes identified below (the particular nucleotide sequence for each of these probes is identified in the tables describing sequences 1 to 145).

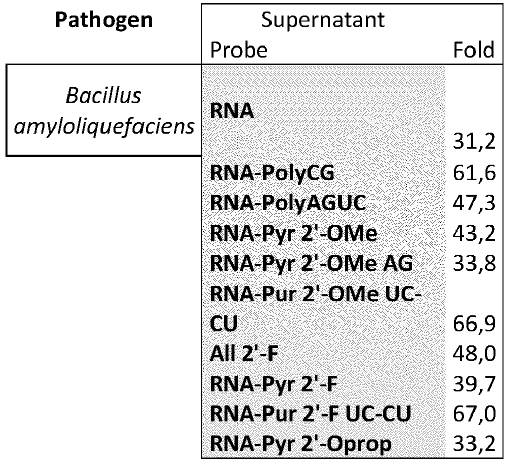

The probes identified in the table above are particularly useful for detecting *Bacillus amyloliquefaciens* in a test sample according to the first or second method of the invention (see figure 24). Consequently, the present invention is further directed to the first or second method for detecting bacteria of the invention, wherein the bacteria is *Bacillus amyloliquefaciens* and wherein the method is preferably carry-out by one or more of the above probes capable of indirectly detecting the presence of the bacteria by detecting the nucleases secreted in the test sample.

It is noted that the first and second methods of the invention proceed in two steps. First, the test sample is mixed with the Substrate reagent and, in the case of the second method, also with the β-lactamase responsive-molecule and/or the nucleic acid probes susceptible of being cleaved only for antibiotic resistant strains of the bacteria, such as MRSA, and not by antibiotic-sensistive bacteria, such as MSSA (please refer to figure 31), and incubated. Both the Substrate/s and optionally the β-iactamase responsive-molecule can be mixed alone with the test sample or will be mixed with an appropriate buffer. Second, there is a, preferably visual, detection of the result by using a lateral flow device. As illustrated in the examples and figures of the present invention, the presence of a control line and the lack of presence of a test line in the lateral flow device, indicates whether that nuclease and/or B-lactamase activity is present in the test sample. The methods provide that this step can be done with unassisted visual inspection.

While the methods of the invention, when practiced by using a lateral flow device, can be practiced without the use of a control line in the lateral flow device, in certain embodiments of the invention it is desirable to include said control lines, such as where the control line is used as a negative control, the control sample, in some embodiments, contains no detectable nuclease or B-lactamase activity. Thus, the present invention further provides a method, in accordance with the first method of the invention, for detecting nuclease activity in a test sample, comprising: (a) contacting the test sample with a substrate, thereby creating a test reaction mixture, wherein the substrate comprises a nucleic acid molecule comprising (i) a cleavage domain comprising a single-stranded region; (ii) a capture tag at one side of the nucleic acid molecule; and (iii) a i) a reporter molecule or ii) a further capture molecule capable of binding the reporter molecule located in the conjugate pad of the support of the lateral flow device, at the other side of the nucleic acid molecule; (b) incubating the test reaction mixture for a time sufficient for cleavage of the substrate by a nuclease in the sample; and (c) determining whether a detectable signal is present in the test line of the lateral flow device, wherein presence of the said signal and the presence of a signal in the control line indicates that the sample fails to contain nuclease activity and absence of said signal and presence of a signal in the control line indicates that the sample contains nuclease activity.

In addition, the present invention further provides a method, in accordance with the second method of the invention, for detecting the presence of a bacteria and its antibiotic resistance in a test sample, comprising: (a) contacting the test sample with a substrate and a β-iactamase responsive-molecule and/or the nucleic acid probes susceptible of being cleaved only for antibiotic resistant strains of the bacteria, such as MRSA, and not by antibiotic-sensistive bacteria, such as MSSA (please refer to figure 31), thereby creating a test reaction mixture, wherein the substrate comprises a nucleic acid molecule comprising (i) a cleavage domain comprising a single-stranded region; (ii) a capture tag at one side of the nucleic acid molecule; and (iii) a i) a reporter molecule or ii) a further capture molecule capable of binding the reporter molecule located in the conjugate pad of the support of the lateral flow device, at the other side of the nucleic acid molecule; and wherein the β-lactamase responsive-molecule comprises a compound comprising a β-lactam ring, preferably a β-lactam antibiotic selected from the list consisting of penicillin derivatives (penams), cephalosporins (cephems), monobactams, and carbapenems, more preferably methicillin, a capture tag on one end and i) a reporter molecule or ii) a further capture tag capable of binding the reporter molecule located in the conjugate pad of a support of a lateral flow device, on the other end; and (b) incubating the test reaction mixture for a time sufficient for cleavage of the substrate and β-lactamase responsive-molecule by a nuclease and β -lactamase in the sample; and (c) determining whether a detectable signal is present in the nuclease test line and the β-iactamase of the lateral flow device, wherein presence of the said signal in the nuclease test line and the presence of a signal in the control line indicates that the sample fails to contain nuclease activity, wherein absence of said signal in the nuclease test line and presence of a signal in the control line indicates that the sample contains nuclease activity, wherein absence of the signal and the presence of a signal in the control line indicates that the sample fails to contain nuclease activity, wherein absence of said signal in the nuclease test line, presence of a signal in the control line and absence of said signal in the β -lactamase test line indicates that the sample contains nuclease activity and β -lactamase activity and wherein absence of said signal in the nuclease test line, presence of a signal in the control line and presence of said signal in the β-iactamase test line indicates that the sample contains nuclease activity but no β -lactamase activity.

The methods of the invention can further entail contacting the test sample with a buffer before or during step (a).

The present invention further discloses compositions and kits for practicing the present methods. Thus, in certain embodiments, the present invention discloses a kit comprising: (a) in one container, a substrate, the substrate comprising a nucleic acid molecule comprising a single stranded region, the single-stranded region comprising: (i) a cleavage domain; (ii) a capture tag on one side of the nucleic acid molecule; (iii) a i) a reporter molecule or ii) a further capture tag capable of binding the reporter molecule located in the conjugate pad of the support of the lateral flow device, on the other side of the nucleic acid molecule; and optionally a β-lactamase responsive-molecule which in turn comprises a compound comprising a β-lactam ring, preferably a β-lactam antibiotic selected from the list consisting of penicillin derivatives (penams), cephalosporins (cephems), monobactams, and carbapenems, more preferably methicillin, a capture tag on one end and i) a reporter molecule or ii) a further capture tag capable of binding the reporter molecule located in the conjugate pad of a support of a lateral flow device, on the other end; and (b) preferably a lateral flow device. These kits are further described at the end of the description in the present specification, in the section entitled "Lateral flow devices and tests".

With respect to the capture tags, any compound that can be captured in a lateral flow assay and that can be bound to the single stranded region can be used in the methods and compositions of the invention. Numerous compounds are capable of that, such as BIOTIN, FITC, FAM, DIGOXIGENIN, DNP (DINITROPHENYL), TEXAS RED, TAMRA or any other small antigen.

In certain embodiments, the capture molecule is an antibody or other recognition molecules such as fab-fragments, nanobodies, aptamers, etc.

With respect to the reporter group, numerous compounds are capable of that, such as latex or gold nanoparticles, carbon particles, or other colorimetric reporter molecules.

The nucleic acids of the invention, including those for use as substrates in the methods of the invention, in certain embodiments are single-stranded or double stranded molecules. In other embodiments, the nucleic acids of the invention are chimeric oligonucleotides comprising a nuclease resistant modified ribonucleotide residue. Exemplary RNase resistant modified ribonucleotide residues include 2'-fluoro ribonucleotides, 2'-O-methyl ribonucleotides, 2'-methoxyethoxy ribonucleotides, 2'-O-allyl ribonucleotides, 2'-O-pentyl ribonucleotides, LNA, UNA and 2'-O-butyl ribonucleotides. In one mode of the embodiment, the modified ribonucleotide residue is at the 5'-terminus or the 3'-terminus of the cleavage domain. In yet other embodiments, the nucleic acids of the invention are chimeric oligonucleotides comprising a deoxyribonuclease resistant modified deoxyribonucleotide residue. In specific modes of the embodiments, the deoxyribonuclease resistant modified deoxyribonucleotide residue is a phosphotriester deoxyribonucleotide, a methylphosphonate deoxyribonucleotide, a phosphoramidate deoxyribonucleotide, a phosphorothioate deoxyribonucleotide, a phosphorodithioate deoxyribonucleotide, or a boranophosphate deoxyribonucleotide. In yet other embodiments of the invention, the nucleic acids of the invention comprise a ribonuclease-cleavable modified ribonucleotide residue.

The nucleic acids of the invention, including those for use as substrates in the methods of the invention, are at least 3 nucleotides in length, such as 5-30 nucleotides in length. In certain specific embodiments, the nucleic acids of the invention are 5-20, 5-15, 5-10, 7-20, 7-15, 7-10, 5-50 or 10-50 nucleotides in length.

In certain embodiments, the capture tag of the nucleic acids of the invention is 5' to the cleavage domain and the reporter molecule is 3' to the cleavage domain. In a specific embodiment, the capture tag is at the 5' terminus of the substrate. In another specific embodiment, the reporter molecule is at the 3' terminus of the substrate.

In certain embodiments, the reporter molecule of the nucleic acids of the invention is 5' to the cleavage domain and the capture molecule is 3' to the cleavage domain. In a specific embodiment, the reporter molecule is at the 5' terminus of the substrate. In another specific embodiment, the capture tag is at the 3' terminus of the substrate.

In one embodiment of the invention, a nucleic acid of the invention comprising the formula: 5'-N1-n-N2 -3', wherein: (a) "N1" represents zero to five 2'-modified ribonucleotide residues; (b) "N2" represents one to five 2'-modified ribonucleotide residues; and (c) "n" represents one to ten, such as four to ten unmodified ribonucleotide residues. In a certain specific embodiment, "N1" represents one to five 2'-modified ribonucleotide residues. In certain modes of the embodiment, the capture molecule or the reporter molecule is attached to the 5'-terminal 2'-modified ribonucleotide residue of N1.

In the nucleic acids of the invention, including nucleic acids with the formula: 5'-N1-n-N2-3', the capture tag can be 5' to the cleavage domain and the reporter molecule is 3' to the cleavage domain; alternatively, the reporter molecule is 5' to the cleavage domain and the capture molecule is 3' to the cleavage domain.

The β-iactamase responsive-molecules suitable for use in the methods of the invention are already indicated through-out the specification as any molecule comprising a compound comprising a β-lactam ring, preferably a β-lactam antibiotic selected from the list consisting of penicillin derivatives (penams), cephalosporins (cephems), monobactams, and carbapenems, more preferably methicillin, a capture tag on one end and i) a reporter molecule or ii) a further capture tag capable of binding the reporter molecule located in the conjugate pad of a support of a lateral flow device, on the other end. Preferably such molecule is the one shown in figure 9.

With respect to the kits of the invention, in addition to comprising a nucleic acid of the invention, the kits can further comprise one or more of the following: a nuclease; ribonuclease-free water, a buffer, and nuclease-free laboratory plastic ware.

### Substrate Oligonucleotides

As already stated the oligonucleotide Substrates used to practice the present invention, no matter if these are used for detecting the presence of a bacterium or those susceptible of being cleaved only for antibiotic resistant strains of the bacteria, such as MRSA, and not by antibiotic-sensistive bacteria, such as MSSA (please refer to figure 31) or viceversa, are substrates for nuclease (e.g., ribonuclease) enzymes. Such substrate oligonucleotides comprise: 1) one or more nuclease-cleavable bases, e.g., RNA bases, some or all of which function as scissile linkages, 2) a capture tag and a i) a reporter molecule or ii) a further capture tag capable of binding the reporter molecule located in the conjugate pad of the support of the lateral flow device, and 3) may optionally contain RNase-resistant modified RNA bases, nuclease-resistant DNA bases, or unmodified DNA bases. Synthetic oligonucleotide RNA-DNA chimeras wherein the internal RNA bonds function as a scissile linkage are described in U.S. Pat. Nos. 6,773,885 and 7,803,536.

In certain embodiments, the substrate oligonucleotide probes are single-stranded or double-stranded oligoribonucleotides. In certain embodiments, the oligonucleotide probes are composed of modified oligoribonucleotides. The term "modified" encompasses nucleotides with a covalently modified base and/or sugar. For example, modified nucleotides include nucleotides having sugars, which are covalently attached to low molecular weight organic groups other than a hydroxyl group at the 3' position and other than a phosphate group at the 5' position. Thus modified nucleotides may also include 2' substituted sugars such as 2'-O-methyl-; 2-O-alkyl; 2'-O-allyl; 2'-S-alkyl; 2'-S-allyl; 2'-fluoro-; 2'-halo or 2'-azido-ribose, carbocyclic sugar analogues α-anomeric sugars; epimeric sugars such as arabinose, xyloses or lyxoses, pyranose sugars, furanose sugars, and sedoheptulose. In certain embodiments, the Substrate includes, but is not limited to, 2'-O-methyl RNA, 2'-methoxyethoxy RNA, 2'-O-allyl RNA, 2'-O-pentyl RNA, and 2'-O-butyl RNA.

Modified nucleotides are known in the art and include, by example and not by way of limitation, alkylated purines and/or pyrimidines; acylated purines and/or pyrimidines; or other heterocycles. These classes of pyrimidines and purines are known in the art and include, pseudoisocytosine; N4, N4-ethanocytosine; 8-hydroxy-N6-methyladenine; 4-acetylcytosine, 5-(carboxyhydroxylmethyl) uracil; 5-fluorouracil; 5-bromouracil; 5-carboxymethylaminomethyl-2-thiouracil; 5-carboxymethylaminomethyl uracil; dihydrouracil; inosine; N6-isopentyl-adenine; 1-methyladenine; 1-methylpseudouracil; 1-methylguanine; 2,2-dimethylguanine; 2-methyladenine; 2-methylguanine; 3-methylcytosine; 5-methylcytosine; N6-methyladenine; 7-methylguanine; 5-methylaminomethyl uracil; 5-methoxy amino methyl-2-thiouracil; β-D-mannosylqueosine; 5-methoxycarbonylmethyluracil; 5-methoxyuracil; 2-methylthio-N6-isopentenyladenine; uracil-5-oxyacetic acid methyl ester; psueouracil; 2-thiocytosine; 5-methyl-2 thiouracil, 2-thiouracil; 4-thiouracil; 5-methyluracil; N-uracil-5-oxyacetic acid methylester; uracil 5-oxyacetic acid; queosine; 2-thiocytosine; 5-propyluracil; 5-propylcytosine; 5-ethyluracil; 5-ethylcytosine; 5-butyluracil; 5-pentyluracil; 5-pentylcytosine; and 2,6,-diaminopurine; methylpsuedouracil; 1-methylguanine; 1-methylcytosine.

The oligonucleotides used to practice the present invention may be synthesized using conventional phosphodiester linked nucleotides and synthesized using standard solid or solution phase synthesis techniques, which are known in the art. Linkages between nucleotides may use alternative linking molecules. For example, linking groups of the formula P(O)S, (thioate); P(S)S, (dithioate); P(O)NR'2; P(O)R'; P(O)OR6; CO; or CONR'2 wherein R is H (or a salt) or alkyl (1-12C) and R6 is alkyl (1-9C) is joined to adjacent nucleotides through -O- or -S-.

In certain embodiments of the present invention, the oligonucleotides have additional modifications, such as 2'-O-methyl modification of the pyrimidines. In other embodiments, all of the nucleotides in the oligonucleotides are 2'-O-methyl modified. Alternatively, the pyrimidines, or all the nucleotides, may be modified with 2' fluoros (both pyrimidines and purines).

The oligonucleotides are short, such as between 2-30 nucleotides in length (or any value in between). In certain embodiments, that oligonucleotide is between 10-15 nucleotides in length. In certain embodiments, that oligonucleotide is between 11-13 nucleotides in length. In general, shorter sequences will give better signal to noise ratios than longer probes and will therefore be more sensitive. However, in certain embodiments, shorter probes might not be the best substrate for the nuclease, so some degree of empiric optimization for length is needed. In certain embodiments, the oligonucleotide comprises 0-50% purines (or any value in between). In certain embodiments the oligonucleotide comprises 100% pyrimidines.

It should be noted that the specific sequence of the oligonucleotide is not critical. Certain combinations of purines and pyrimidines are susceptible to bacterial endonucleases, while resisting mammalian nucleases. Endonucleases are enzymes that cleave the phosphodiester bond within a polynucleotide chain, in contrast to exonucleases, which cleave phosphodiester bonds at the end of a polynucleotide chain. These bacterial nucleases are not sequence-specific like restriction enzymes, which typically require a recognition site and a cleavage pattern. Some endonucleases cleave single-stranded nucleic acid molecules, while others cleave double-stranded nucleic acid molecules.

### Linkers or bounders

In certain embodiments, the oligonucleotide and/or β-lactamase responsive-molecules is linked to the capture tag and/or reporter molecule by means of a linker or bounder. The capture molecule could be an antibody or other biorecognition molecules such as fab-fragments, nanobodies, aptamers, etc.

In certain embodiments, an aliphatic or ethylene glycol linker (as are well known to those with skill in the art) is used. In certain embodiments, the linker is a phosphodiester linkage. In certain embodiments, the linker is a phosphorothioate linkage.

### Substrate Synthesis

Synthesis of the nucleic acid Substrates used to practice the present invention can be performed, but without being limited to, using solid-phase phosphoramidite chemistry (U.S. Pat. No. 6,773,885) with automated synthesizers, although other methods of nucleic acid synthesis (e.g., the H-phosphonate method) may be used. Chemical synthesis of nucleic acids allows for the production of various forms of the nucleic acids with modified linkages, chimeric compositions, and nonstandard bases or modifying groups attached in chosen places throughout the nucleic acid's entire length.

### Detection Methods

The present invention provides a first method for detecting bacteria or tumoral cells in a sample and a second method for simultaneously detecting bacteria and its resistance to antibiotics such as methicillin in a test sample *in vitro* by using a lateral flow device. The methods of the invention proceed in the following steps: combine "test or biological sample" with Substrate(s) and optionally in the context of the second method of the invention β-iactamase responsive-molecules and/or the nucleic acid probes susceptible of being cleaved only for antibiotic resistant strains of the bacteria, such as MRSA, and not by antibiotic-sensistive bacteria, such as MSSA (please refer to figure 31) or viceversa, to produce a mixture, the mixture being the Assay Mix, incubate, and detect whether a signal is produced in a test line of a lateral flow device, to practice the second method of the invention the lateral flow device shall comprise at least a test line of nuclease activity and a further test line of B-lactamase activity. "Test sample" refers to any material being assayed for nuclease and optionally B-lactamase activity or antibiotic resistance and in certain embodiments, will be a liquid. Solids can be indirectly tested for the presence of nuclease contamination by washing or immersion in solvent, e.g., water, followed by assay of the solvent.

For example, one can contact a sample with an oligonucleotide probe and optionally β-iactamase responsive-molecules as described herein, and detect the presence of bacterial endonucleases and optionally B-lactamase activity using a lateral flow device.

In certain embodiments, the probes and molecules of the present invention are also useful to detect bacterial contamination in settings such as research laboratories.

Each of the steps of the method will be further illustrated, but without being limited to, as follows below:
**1. Assay Mix.** The Substrate/s and optionally β-lactamase responsive-molecules is mixed and incubated with the test sample. This mixture constitutes the Assay Mix. Ideally, the Assay Mix is a small volume, from about 1 µl to about 10 ml, or, from about 10 to 100 µl. The precise volume of the Assay Mix will vary with the nature of the test sample and the detection method. Optionally, a buffer can be added to the Assay Mix. Nucleases, including some ribonucleases, require the presence of divalent cations for maximum activity and providing an optimized buffered solution can increase the reaction rate and thereby increase assay sensitivity. Buffers of different composition can be used, as described in U.S. Pat. No. 6,773,885.
**2. Incubation.** The Assay Mix (e.g., the test sample plus Substrate/s and optionally β-iactamase responsive-molecules) is incubated. Incubation time and conditions can vary from a few minutes to 24 hours or longer depending upon the sensitivity required. Incubation times of one hour or less are desirable. Nucleases are catalytic. Increasing incubation time should therefore increase sensitivity of the Assay, provided that background cleavage of the Substrate (hydrolysis) remains low. As is evident, assay background is stable over time and Assay sensitivity increases with time of incubation. Incubation temperature can generally vary from room temperature to 37 degree C. but may be adjusted to the optimal temperature of a specific nuclease suspected as being present as a contaminant.

### 3. Signal Detection.

For signal detection two different systems may be employed according to the present invention:
A first system comprises:
a. The polynucleotide sequence/s as defined in the first aspect of the invention and optionally β-lactamase responsive-molecules conjugated to a reporter molecule;
b. A support suitable for lateral flow that comprises:
   b1. A backing card; and
   b2. A sample pad, a membrane and a wicking pad, all of them on top of the backing pad, wherein the membrane is located after the sample pad so that the sample flows from the sample pad to the membrane, wherein the membrane comprises a test line which in turn comprises molecules immobilized in the test line for capturing the capture molecule of the polynucleotide sequence, and optionally a second test line which in turn comprises molecules immobilized in said second test line for capturing the capture molecule of the β-iactamase responsive-molecules, and wherein the wicking pad is located at the end of the membrane.

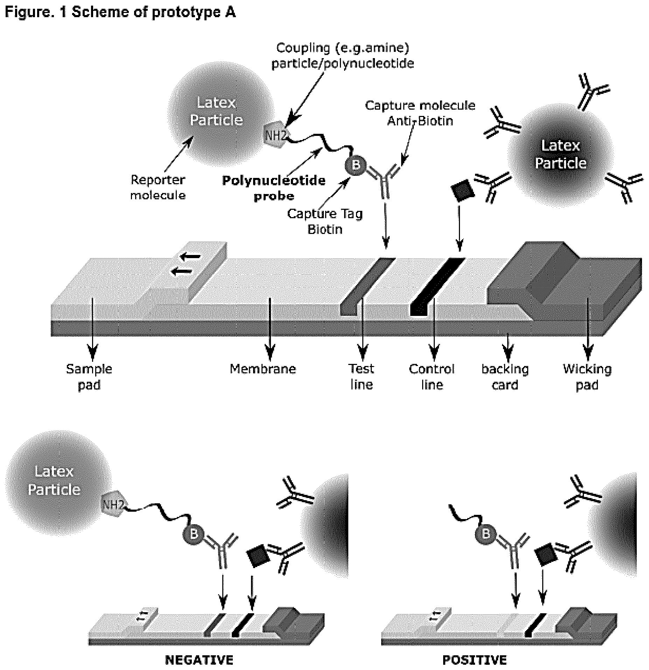

Assay Mix. The sample is incubated with the polynucleotide sequence as defined in the first aspect of the invention and optionally β-iactamase responsive-molecules conjugated to a reporter molecule, in this system latex/gold nanoparticles can be use to functionalized the polynucleotide sequence as defined in the first aspect of the invention and optionally the β-lactamase responsive-molecules using coupling chemistry (e.g. amine coupling) at one end of each of these moieties. The other end of the moieties can be modified with a capture tag, if practicing the second method of the invention a different capture tag should be use for each of the moieties. After incubation at desired temperature and time to allow efficient degradation of the polynucleotide and optionally the β-iactamase responsive-molecule, the assay mix is dispensed on the sample pad of the lateral flow device. Subsequently, the sample fluid is transported to the membrane and the capture tags can be detected by the capture molecules at the test line/s. If the nuclease activity is present, the capture molecule will detect a fragment of the polynucleotide that was functionalized with the capture tag, with no visible signal. If the nuclease activity is absent, the capture molecule will capture the biotinylated polynucleotide along with the reporter molecule, and thus will provide a readout signal that can be evaluated by naked eye. A schematic representation of this system is provided in Figure 1 and figure 8. The determination of the presence or absence of B-lactamase activity is determined within the same lateral flow device similarly to the way in which the nuclease activity is determined as already explained throughout the present specification.

A second system comprises:
a. The polynucleotide sequence/s as defined in the third aspect of the invention and optionally β-lactamase responsive-molecules conjugated in both ends to capture molecules, wherein one of the capture molecules of each moiety (the polynucleotide sequence and optionally the β-lactamase responsive-molecule) is capable of binding the reporter molecule located in the conjugate pad of the support; and
b. A support suitable for lateral flow that comprises:
   b1. A backing card; and
   b2. A sample pad, a conjugate pad, a membrane and a wicking pad, all of them on top of the backing pad, wherein the membrane is located after the sample pad so that the sample flows from the sample pad to the membrane, wherein the membrane comprises a test line which in turn comprises molecules immobilized in the test line for capturing the capture molecule of the polynucleotide sequence, and optionally a second test line, to practice the second method of the invention, which in turn comprises molecules immobilized in said second test line for capturing the capture molecule of the β-lactamase responsive-molecules, wherein the wicking pad is located at the end of the membrane.
and wherein the system further comprises a conjugate pad between the sample pad and the membrane which in turn comprises a reporter molecule.

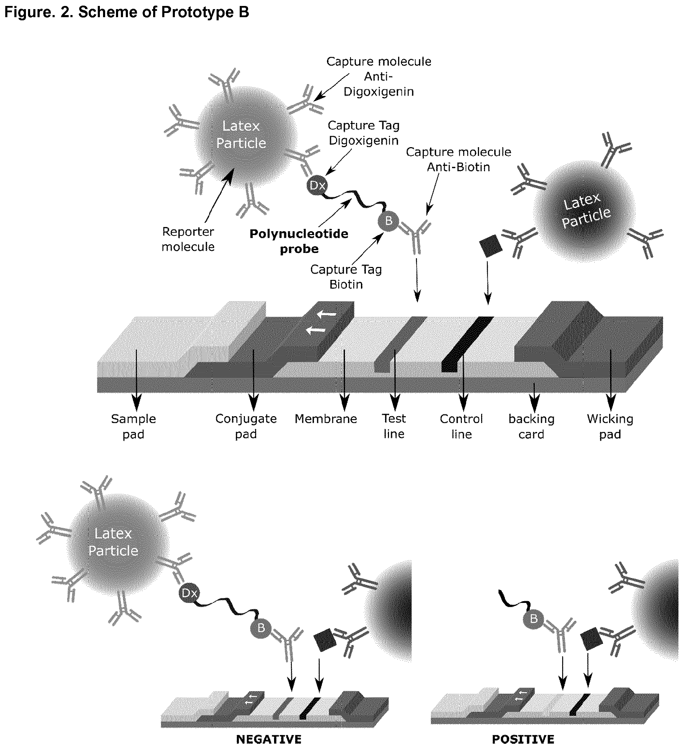

Assay Mix. In this system, the sample is incubated with the polynucleotide probe and optionally the β-iactamase responsive-molecule, wherein both moieties are modified at both ends with two different capture tags. After incubation at desired temperature and time to allow efficient degradation of the polynucleotide and of the β-lactamase responsive-molecule, the assay mix is dispensed on the sample pad of the lateral flow device. Subsequently, the sample fluid is transported to the conjugate pad, where the reporter molecule was previously dispensed. This reporter molecule was modified with a capture molecule (e.g. anti-digoxigenin) that recognizes one of the capture tags of the polynucleotide (e.g. digoxigenin) and optionally the β-iactamase responsive-molecule. Next, the assay mix and the reporter molecule are transported to the membrane where the test line/s is located and the capture molecule for detecting each of the moieties is immobilized. In this regard, one test line (the nuclease test line) shall be used to practice the first method of the invention and at least two test lines (the nuclease test line and the β-lactamase test line) shall be use to practice the second method of the invention. Regarding the former, it is again noted that both capture molecules immobilized in each test line must be different in order to clearly differentiate nuclease activity form β-iactamase activity at the time of practicing the second method of the invention. If the nuclease activity is present in the sample, the capture molecule present in the nuclease test line will detect a fragment of the polynucleotide that was functionalized with the second capture tag (e.g. biotin), with no visible signal. Likewise, if the β-lactamase activity is present in the sample, the capture molecule present in the β-iactamase test line will detect the fragment of the β-lactamase responsive-molecule that was functionalized with the further capture tag different from the second capture molecule, with no visible signal. If the nuclease activity is absent, the second capture molecule will detect the biotinylated polynucleotide along with the reporter molecule, and thus will provide a readout signal that can be evaluated by naked eye. A schematic representation of this system is provided in Figure 2 and figure 8.

### 4. Visual Detection Method. Following incubation, the Assay Mix is used in each of the systems described above as detailed as follows.

Regarding the first system, the assay mix used once incubated is placed in the sample pad of the lateral flow system. Next, the sample fluid is transported to the membrane and the capture molecule immobilized in the test line/s (e.g. anti-biotin) will recognize (each) of the capture tags located at one of the end of the polynucleotide probe and optionally the β-lactamase responsive-molecule. If the nuclease activity is present, the capture molecule will detect a fragment of the polynucleotide that was functionalized with the capture tag, with no visible signal. If the nuclease activity is absent, the capture molecule will capture the biotinylated polynucleotide along with the reporter molecule, and thus will provide a readout signal that can be evaluated by naked eye. The determination of the presence or absence of B-lactamase activity is determined within the same lateral flow device similarly to the way in which the nuclease activity is determined as already explained throughout the present specification.

This system will provide a readout signal that can be evaluated by naked eye.

Regarding the second system, the assay mix used once incubated is placed in the sample pad of the lateral flow system and later mixed with the conjugate pad, where the reporter molecule was previously dispensed. Next, the sample fluid is transported to the membrane and the capture tag immobilized in the test line/s will recognize the capture tags located at one of the end of the polynucleotide probe and optionally the β-lactamase responsive-molecule. In this regard, one test line (the nuclease test line) shall be used to practice the first method of the invention and at least two test lines (the nuclease test line and the B-lactamase test line) shall be used to practice the second method of the invention. Regarding the former, it is again noted that both capture molecules immobilized in each test line must be different in order to clearly differentiate nuclease activity form B-lactamase activity at the time of practicing the second method of the invention. If the nuclease activity is present in the sample, the capture molecule present in the nuclease test line will detect a fragment of the polynucleotide that was functionalized with the second capture tag (e.g. biotin), with no visible signal. Likewise, If the B-lactamase activity is present in the sample, the capture molecule present in the B-lactamase test line will detect the fragment of the β-lactamase responsive-molecule that was functionalized with the further capture tag different from the second capture molecule, with no visible signal. If the nuclease activity is absent, the second capture molecule will detect the biotinylated polynucleotide along with the reporter molecule, and thus will provide a readout signal that can be evaluated by naked eye.

Therefore, an Assay Mix in which the Substrate remains intact will provide for a test signal and will visually appear. An Assay Mix in which the Substrate has been cleaved will not provide for a test signal and will visually appear clear or dark (no color). The visual detection method is primarily intended for use as a qualitative nuclease assay, with results being simply either "positive" or "negative".

The Assay Mix will ideally constitute a relatively small volume, for example 10µl to 1000µL although greater or lesser volumes can be employed. Small volumes allow for maintaining high concentrations of Substrate yet conserves use of Substrate. The visual detection Assay in one embodiment uses 50 pmoles of Substrate at a concentration of 0.5 µM in a 100 µl final volume Assay Mix. Lower concentration of Substrate (e.g., below 0.1 pM) will decrease assay sensitivity. Higher concentrations of Substrate (e.g., above 1 µM) will increase background and will unnecessarily consume Substrate.

Steps (mixing, incubating), can be performed in one tube. In one embodiment, the tube is a small, thin-walled, UV transparent micro tube, although tubes of other configuration may be used.

### Lateral flow devices and tests

The present invention further discloses lateral flow devices and kits containing the same for detecting nuclease activity and optionally B-lactamase activity in a sample, comprising Substrate nucleic acid(s), optionally β-lactamase responsive-molecule and instructions for use. Such kits may optionally contain one or more of: a positive control nuclease, DNase/RNase-free water, a buffer, and other reagents.

Lateral Flow tests, also known as Lateral Flow Immunochromatography (LFIC) tests or just Lateral Flow Immunoassays (LFI), are simple small devices intended to detect a target analyte in a given sample. These tests are simple, fast, and cheap and do not require specialized and costly equipment nor qualified personnel. Even though the LFIC technology was born in the early 80s, it is still going to be a major player in the next decades in the rapid test industry. In the beginning, LFIC tests were exclusively used in medical diagnostics (either for home testing, point of care testing, or laboratory use) but their presence in other areas are now very common. The first application was the well-known home pregnancy test. Although there are several commercially available semiquantitative tests, LFIC tests are mainly qualitative tests and they just give a positive or negative result based on the presence or absence of the target analyte at a specific concentration.

The technology is based on a series of capillary beds with porous materials that has the capacity to transport liquids spontaneously. When the target analyte is big (e.g. a protein) a sandwich format is commonly used. Usually, the first element (the conjugate pad) has stored the so-called colloids, a dried format of bioactive particles (mainly gold nanoparticles or latex microparticles bound to a specific antibody against the target analyte) in a salt-sugar matrix. This conjugate pad usually acts also as the sample pad and it contains everything to facilitate the recognition of the target antigen by the colloid. Once the conjugate pad is soaked by the sample, the fluid dissolves the salt-sugar matrix and the colloids. Therefore, the sample and the colloid mix while they flow through the porous structure. In this way, the analyte binds to the colloid and the complex is transported into the nitrocellulose membrane. This material has one specific area called test line where a third molecule (usually another different antibody against the target analyte) has been immobilized. When the complex (colloid-antigen) reaches the test line it gets bound to this antibody and after a while, when more and more fluid has flown, colloids accumulate and the test line acquires color. Typically, there is also a control line (after the test line) that captures the colloids that have not bound to the test line. In the control line, a fourth molecule (could be an antibody against the Fc fraction of the first antibody) is adsorbed and its color acquisition ensures the test functionality. After passing these reaction zones the fluid enters the final porous material, the wick or sink that simply acts as a waste container.

To practice the present invention, a large variety of different lateral flow system devices could be used, however, two devices to which the present invention is not limited to are herein provided just for illustrative purposes:
A first lateral flow device comprising a support suitable for lateral flow, which in turn comprises:
a1. A backing card; and
a2. A sample pad, a membrane and a wicking pad, all of them on top of the backing pad, wherein the membrane is located after the sample pad so that the sample flows from the sample pad to the membrane, wherein the membrane comprises a test line which in turn comprises molecules immobilized in the test line for capturing the capture molecule of the polynucleotide sequence, and optionally a second test line, to practice the second method of the invention, which in turn comprises molecules immobilized in said second test line for capturing the capture molecule of the β-iactamase responsive-molecules, wherein the molecules immobilized in the test line are different from those immobilized in the second test line since the capture molecules of the β-lactamase responsive-molecules and of the polynucleotide sequence shall differ from one another, and wherein the wicking pad is located at the end of the membrane.

A second lateral flow device comprising a support suitable for lateral flow, which in turn comprises:
a1. A backing card; and
a2. A sample pad, a membrane and a wicking pad, all of them on top of the backing pad, wherein the membrane is located after the sample pad so that the sample flows from the sample pad to the membrane, wherein the membrane comprises a test line which in turn comprises molecules immobilized in the test line for capturing the capture molecule of the polynucleotide sequence, and optionally a second test line, to practice the second method of the invention, which in turn comprises molecules immobilized in said second test line for capturing the capture molecule of the β-iactamase responsive-molecules, wherein the wicking pad is located at the end of the membrane, wherein the molecules immobilized in the test line are different from those immobilized in the second test line since the capture molecules of the β-iactamase responsive-molecules and of the polynucleotide sequence shall differ from one another;
and wherein the support further comprises a conjugate pad between the sample pad and the membrane, which in turn comprises a reporter molecule.

In addition, one kit of the invention includes a universal Substrate. The kit is intended to detect nuclease activity from one or from a variety of sources. The kit further includes a lateral flow device, in particular a lateral flow device as illustrated above. In certain embodiments, the Substrate will be provided in dry form in individual thin-walled, UV transparent microtubes, or in multiwell (e.g., 96 well) formats suitable for high throughput procedures. Lyophilized Substrate has improved long-term stability compared to liquid solution in water or buffer. If provided in liquid solution, stability is improved with storage at least below -20° C., such as at -80° C. Storage in individual aliquots limits potential for contamination with environmental nucleases. Alternatively, the Substrate can be provided in bulk, either lyophilized or in liquid solution. Alternatively, substrate can be provided in bulk and can be dispersed at the discretion of the user.

The following examples merely serve to illustrate the present invention and do not limit the same.

### Examples

### Example 1. STAPHYLOCOCCUS AUREUS DETECTION BY LATERAL FLOW (prototype A)

Identification of a given microorganism (e.g. *Staphylococcus aureus)* or cell type (e.g. breast cancer cell) is now possible taking advantage of the specific nuclease activity exerted by the mentioned microorganisms/cells or tissue. The identification and/or design of specific nucleic acid probes that are degraded by the target cell or microorganism but not by the rest of the microorganism/cell types are critical.

We have developed specific LFPC strips that can detect non-degraded nucleic acid probes in a given sample. Accordingly, the presence of a nucleic acid probe will give rise to a colored test line. On the other hand, if a specific nuclease is present in the sample it will eventually produce a complete degradation of the nucleic acid probe, and consequently, no test line will be seen.

### Material and methods.

The conjugation of polynucleotide probe and latex particles and the strips were prepared as described in scheme 1. The protocol for prototype A was as follows:
The assay mix contains: 100µL sample and 20µL of TT probe* (800 fmol) diluted in PBS +/+ *TT probe (SEQ No 1 or SEQ No 2) was modified with an amine group at the 5'-end and a biotin at the 3'-end. The amine group was used for coupling to the latex nanoparticles and the biotin as the tag detected by the capture molecule (anti-biotin) immobilized in the test line.

The assay mix was then incubated at 37°C for 1 hour in a 1.5 mL tube. Next, a strip was placed inside the 1.5 mL tube. After 10 minutes, the strip was removed from the tube and the detection signal was visualized.

### Results

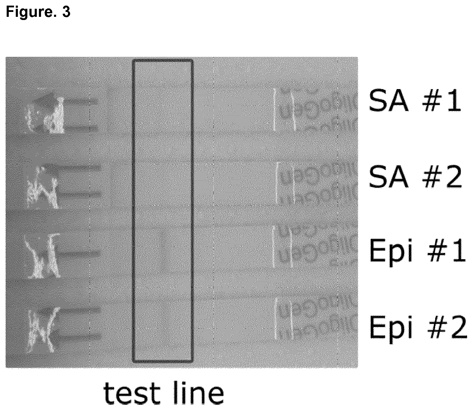

As a proof of concept, we have incubated samples of Staphylococcus aureus (SA) supernatants and Staphylococcus epidermidis (Epi) supernatants with the polynucleotide prototype described in Figure 1. Briefly, the reporter molecule (latex/gold nanoparticle) was functionalized with the polynucleotide probe using one of the ends, via amine coupling. The other end of the polynucleotide probe was modified with biotin. This reagent was incubated with cultures of S. aureus and S. epidermidis as described above in the methods section. Next, the assay mix was dispensed in the sample pad of the lateral flow device, with a subsequent transport of the mix to the membrane. In the test line, the anti-biotin was immobilized to capture the polynucleotide probe. As shown in Figure 3, a visible signal in red was observed for the S. epidermidis samples. These results suggest that the polynucleotide probes were not cleaved and the reporter molecule was retained in the test line, indicating absence of nuclease activity. In contrast, the S. aureus samples have reported non-visible signal at the test line, indicating the presence of nuclease activity as consequence of polynucleotide probe degradation. These results were observed by naked eye by duplicate. Altogether, these results confirm that the nuclease activity derived from bacteria can be evaluated using lateral flow analysis and that the polynucleotide probe was successfully integrated in this detection system providing specific detection of S. aureus.

### Example 2. STAPHYLOCOCCUS AUREUS DETECTION BY PROTOTYPE B:

The previous example demonstrates the successful identification of bacteria using polynucleotide probes and a lateral flow device. To explore other alternatives for detecting bacteria, mammalian cells or tissues, another prototype where the polynucleotide probe is not functionalized on the surface of the reporter molecule has been described. It is expected that in some embodiments this system could be more efficient, since the polynucleotide probe can be more easily accessible for nuclease degradation.

To exemplify the above, we created a system that will detect any nucleic acid probe modified with digoxigenin at one end and biotin at the other end. The key of the system is the production of a specific LFPC strip in which an anti-biotin antibody is adsorbed in the test line and a specific colloid, with anti-digoxigenin antibody conjugated on the surface of latex microparticle, is dried into the conjugate pad. A scheme of the above said LFPC strip is represented together with a nucleic acid probe conjugated to biotin and digoxigenin in figure 2.

The following test aims to detect *Staphylococcus aureus* using a specific and modified nucleic acid probe (the so-called TT probe). However, and as mentioned above, these LFPC strips permit the detection of any specific nucleic acid probe (a DNA probe conjugated to digoxigenin and biotin at its ends). Therefore, detection of any microorganism or cell type is now possible by just modifying the sequence of the mentioned DNA probes (as each DNA probe targets a specific nuclease from a specific microorganism or cell type).

### 2.1 Materials and methods

The strips were prepared as described in scheme 2, and the protocol for prototype B was as follows:
The *Staphylococcus aureus* ATCC 15981 strain was used to validate these experiments, along with buffer and other control bacteria (*Staphylococcus epidermidis*). The assay mix encompasses the following components: 1.6 µL Tris 100 mM pH 8.0, 1.6 µL CaCl₂ 1000 mM in water, 155.8 µL sample and 1 µL 250 fmol/µL TT probe*or control probe**
TT probe* (SEQ No 1, SEQ No 2) or control probe** (SEQ No 3) were modified with two capture tags, a digoxigenin group at the 5'-end and a biotin at the 3'-end. The digoxigenin tag was used for binding the capture probe (anti-digoxigenin) immobilized on the latex nanoparticles, and the biotin tag was used as the tag detected by the capture molecule (anti-biotin) immobilized in the test line.

The assay mixtures were placed into 1.5 mL tubes, which were incubated for 30 minutes (or otherwise indicated) at 37°C in an oven. Then, the incubation solutions were transferred into a microtitter plate and a LFPC strip was placed in each of the wells for 10 minutes. Finally, the strips were removed from the microtitter plate, and the test lines were measured using a Qiagen band reader and pictures were taken.

### 1.1. Results

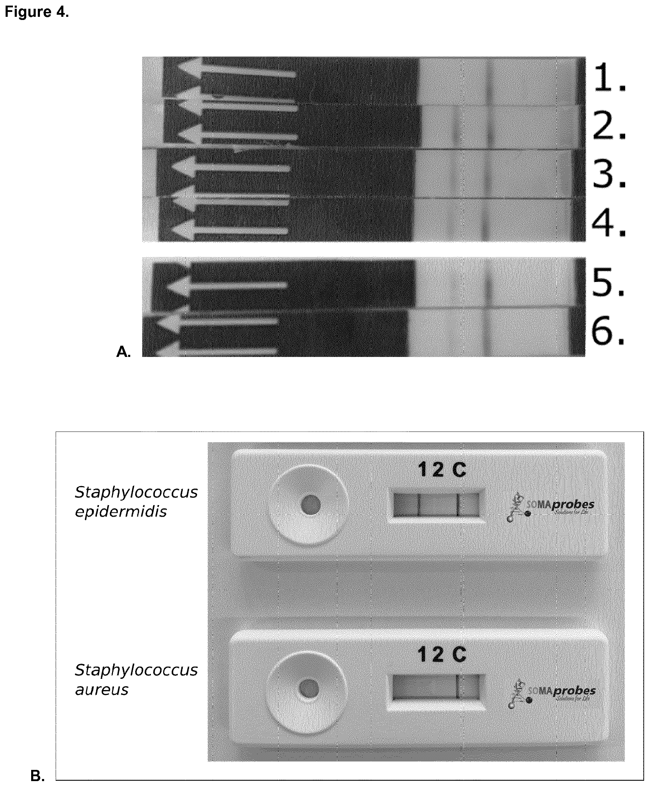

To prove the feasibility of the second prototype the functionality and specificity of the present approach were tested. For that purpose, we used the mentioned TT probe and we tested several samples: S. aureus supernatant and S. epidermidis supernatant as control bacteria, along with TSB culture media and Water. As shown in figure 4, only S. aureus samples were able to degrade the probe and no band (1) was observed by naked eye. In contrast, S. epidermidis (2), TSB (3) and water (4) samples have shown a visible red band indicating absence of nuclease activity. Then, we sought to evaluate the specificity of the polynucleotide probe by testing a nucleic acid probe resistant to nuclease activity (called Control probe, which has all the nucleotides 2'-O-methyl modified). Figure 4 has shown a visible band for both cultures: S. aureus supernatant (5) and S. epidermidis supernatant (6), confirming that only specific polynucleotide probes (e.g. TTprobe) can be digested by S. aureus cultures. These data confirm the utility of specific polynucleotide probes and lateral flow system for targeted detection of bacteria samples.

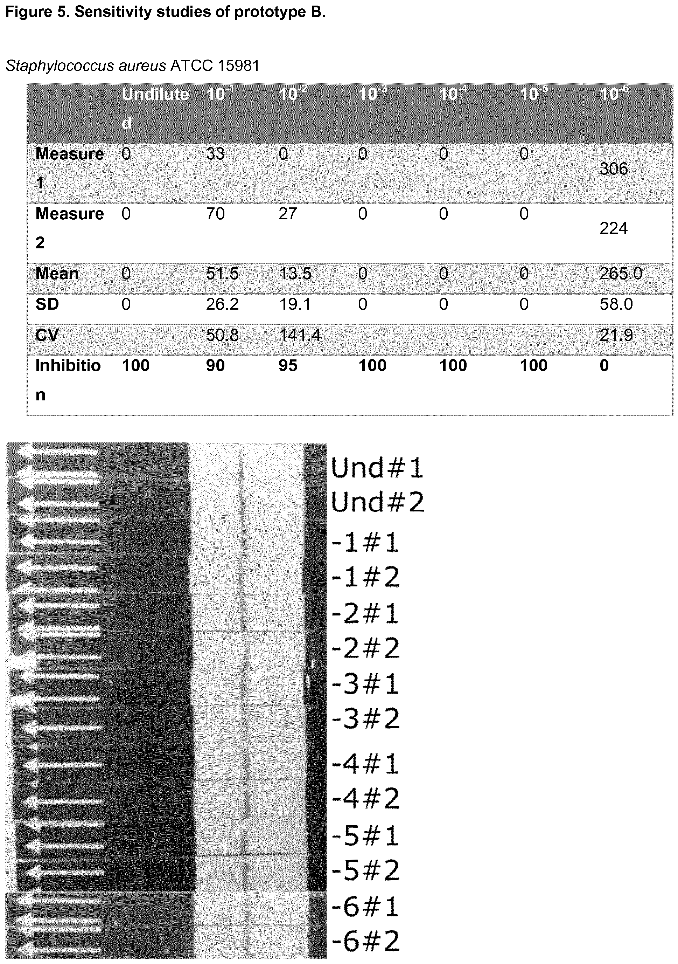

Next, we evaluated the sensitivity of the prototype B. To do so, S. aureus cultures were tested using undiluted (und) samples and 10 fold serial dilutions from 10⁻¹ to 10⁻⁶, as indicated in the table and picture of figure 5. After samples incubation and processing as indicated in the methods section, band reader acquisition for quantitative analysis and pictures were taken. These results have shown that dilutions up to 10⁻⁵ can be detected by this prototype, demonstrating high sensitivity for detecting nuclease activity derived from bacteria in a lateral flow fashion. Then, we completed the specificity studies using 12 different bacteria (Figure 6, 1 to 12), along with the culture media TSB (14) and LB (15). Figure 6 shows the quantification of each culture tested and its respective picture. Only S. aureus cultures have reported a value of 0 using the band reader detection, and no visible band in the picture. In contrast, all the other bacteria culture samples and culture media have reported values ranging from 150 to 400 arbitrary units by the band reader and visible bands in the picture. These results, confirm that the prototype B is highly sensitive and specific, and thus provides a novel method for detecting bacteria using lateral flow analysis.

Finally, we optimized the time needed to obtain reliable results in the lateral flow system. We reduced the detection time to 10 and 20 minutes and several dilutions of S. aureus cultures were evaluated. As indicated in the table and pictures of figure 7, by reducing the time to 10 and 20 min respectively, there is also a reduction of sensitivity. However, detection of dilution 10⁻⁴ was possible for both time points, suggesting that the reaction time can be optimized depending on the final application.

### Example 3. Proof of concept of the β-lactam-responsive molecule.

### Methodology

Proton nuclear magnetic resonance (1H NMR) is the application of nuclear magnetic resonance in NMR spectroscopy with respect to hydrogen-1 nuclei within the molecules of a substance, in order to determine the structure of its molecules. NMR spectra were recorded on Bruker AVD 500 spectrometers at room temperature. In dmso-d6, 1H NMR spectra were referenced to the solvent residual signal. Chemical shifts are quoted in ppm and coupling constants in Hz.

Liquid chromatography-mass spectrometry (LC-MS) is an analytical chemistry technique that combines the physical separation capabilities of liquid chromatography with the mass analysis capabilities of mass spectrometry (MS). LC (liquid chromatography) separates mixtures with multiple components, MS (mass spectrometry) provides structural identity of the individual components with high molecular specificity and detection sensitivity.

### Results

The feasibility of the β-lactam responsive molecule was tested by two independent techniques, where it was validated the cleavage of the β -lactam molecule based on the enzymatic activity of β - lactamases.

4-methoxybenzyl (6R,7R)-3-(((4-benzamidophenyl)thio)methyl)-8-oxo-7-(2 phenylacetamido)-5-thia-1-azabicyclo[4.2.0]oct-2-ene-2-carboxylate (starting product) is a truncated β-lactam responsive molecule that acts as a substrate for by β -lactamases (BL), this cleavage generating a hydrolyzed product. The mechanism by which BL inactivate the β-lactam antibiotics is through the hydrolysis of the β-lactam ring by a serine residue in the active site of the enzyme. After the rearrangement of the new amine formed, a potential leaving group at the C-3' position is eliminated during the enzymatic reaction.

In order to check this enzymatic reaction, two analytical methods have been used, 1H NMR (proton nuclear magnetic resonance) and LC-MS (Liquid chromatography-mass spectrometry).

In both techniques, the starting product (responsive molecule) was cleaved in the presence of β-lactamases, indicating the feasibility of this strategy for detecting antimicrobial resistance. In Figure 32, a schematic drawing of the starting product and hydrolyzed product generated by the actions of the β -lactamases is presented. The characterization of such reaction was carried out by 1H NMR and LC-MS. Figure 33 shows the NMR spectra of the starting and hydrolyzed products. By using the NMR method, it was demonstrated the displacement of the methylene located at 3'position (represented as asterisk at the β-lactam responsive molecule), after the treatment with β-lactamases. Next, the β-lactamase activity was also confirmed by LC-MS. Figure 33, shows the chromatogramms of both, A) starting and B) hydrolized products. Figure 3C, complements our findings by reporting the mass spectrometry analysis of the hydrolized product. Figure 34 ilustrates how this novel β -lactam molecule can be incorporated in a lateral flow system. In the absence of β -lactamases, no reaction is present and a visual line is observed (Line 1 in the picture). On the other hand, in the presence of β -lactamases, the responsive molecule is hydrolyzed and no line is observed (Line 1). In addition, a control line (C) is also present to validate the lateral flow assay.

Altogether, this evidence demonstrates the feasibility of the β-lactam responsive molecule to be used as detection strategy for identifying antimicrobial resistance based on β-lactamases, and this approach holds a great potential to be integrated into lateral flow devices.

## Claims

1. A method for detecting bacteria and its resistance to beta-lactam antibiotics treatment in a test sample, which comprises: 1) incubating in the test sample, a synthetic Substrate or mixture of Substrates for detecting the presence of a bacteria, and 2) simultaneously or subsequently establishing the resistance of the bacteria to beta-lactam antibiotics treatment by incubating in the test sample a β-lactamase responsive-molecule, for a time sufficient for cleavage of the β-lactamase responsive-molecule by a β-lactamase; wherein
a. the Substrate(s) comprises a single-stranded nucleic acid molecule containing at least one ribonucleotide or deoxyribonucleotide or chemically modified nucleotide residue at an internal position that functions as a nuclease cleavage site, a capture tag on one end and i) a reporter molecule or ii) a further capture tag capable of binding the reporter molecule located in the conjugate pad of a support of a lateral flow device, on the other end; and wherein
b. the β-lactamase responsive-molecule comprises a compound comprising a β-lactam ring, preferably a β-lactam antibiotic selected from the list consisting of penicillin derivatives (penams), cephalosporins (cephems), monobactams, and carbapenems, more preferably methicillin, a capture tag on one end and i) a reporter molecule or ii) a further capture tag capable of binding the reporter molecule located in the conjugate pad of a support of a lateral flow device, on the other end.

2. The method of claim 1, wherein the result is, preferably visually, detected, in particular
whether the substrate/s and the β-lactamase responsive-molecule has been cleaved or not by a test sample, by using a lateral flow device or by binding the complexes formed by the substrate/s and the β-lactamase responsive-molecule after the incubation to a solid support that facilitates immunoassay techniques such as enzyme linked immuno-sorbent assay (ELISA), a radioimmunoassay (RIA), an immuno radiometric assay (IRMA), a fluorescent immunoassay (FIA), a chemiluminescent immunoassay (CLIA), magnetic beads-based immunoassay (MBI), or an electro-chemiluminescent immunoassay (ECL).

3. The method of claim 1, wherein the result is, preferably visually, detected, in particular whether the substrate/s and the β-lactamase responsive-molecule has been cleaved or not by a test sample, by using a lateral flow device.

4. The method of any of claims 1 to 3, wherein said β-lactamase responsive-molecule comprises 4-methoxybenzyl (6R,7R)-3-(((4-benzamidophenyl)thio)methyl)-8-oxo-7-(2 phenylacetamido)-5-thia-1-azabicyclo[4.2.0]oct-2-ene-2-carboxylate which is a truncated β-lactam responsive molecule (CEProbe), or (6S,7S)-7-(((3',6'-dihydroxy-3-oxo-3*H-*spiro[isobenzofuran-1,9'-xanthen]-6-ylcarbonyl)oxy)amino)-8-oxo-3-(((4-(5-((3a*R*,4*R*,6a*S*)-2-oxohexahydro-1*H*-thieno[3,4-*d*]imidazol-4-yl)pentanamido)phenyl)thio)methyl)-5-thia-1-azabicyclo[4.2.0]oct-2-ene-2-carboxylic acid (FAM-CEProbe-Biotin), or 6S,7S)-7-(5-(((3*S*,5*R*,8*R*,9*S*,10*S*,12*R*,13*S*,14*S*,17*R*)-12,14-dihydroxy-10,13-dimethyl-17-(5-oxo-2,5-dihydrofuran-3-yl)hexadecahydro-1*H*-cyclopenta[a]phenanthren-3-yl)oxy)-5-oxopentanamido)-8-oxo-3-(((4-(5-((3a*R*,4*R*,6a*S*)-2-oxohexahydro-1*H*-thieno[3,4-*d*]imidazol-4-yl)pentanamido)phenyl)thio)methyl)-5-thia-1-azabicyclo[4.2.0]oct-2-ene-2-carboxylic acid (Digoxigenin-CEProbe-Biotin).

5. The *method* according to any of claims 1 to 4, wherein the capture tag of the Substrate/s and/or β-lactamase responsive-molecule is selected from the list consisting of BIOTIN, FITC, FAM, DIGOXIGENIN, DNP (DINITROPHENYL), TEXAS RED, TAMRA or any other small antigen.

6. The *method* according to any of claims 1 to 5, wherein the reporter molecule of the Substrate/s and/or β-lactamase responsive-molecule is selected from the list consisting of latex and gold nanoparticles, carbon particles or any other colorimetric reporter molecule.

7. A system that comprises:
a. A synthetic Substrate or mixture of Substrates for detecting the presence of a bacteria and the β-lactamase responsive-molecule for establishing the resistance of the bacteria to beta-lactam antibiotics and β-lactamase responsive-molecules; wherein
a1. the Substrate(s) comprises a single-stranded nucleic acid molecule containing at least one ribonucleotide or deoxyribonucleotide or chemically modified nucleotide residue at an internal position that functions as a nuclease cleavage site, a capture tag on one end and i) a reporter molecule or ii) a further capture tag capable of binding the reporter molecule located in the conjugate pad of a support of a lateral flow device, on the other end; and wherein
a2. the β-lactamase responsive-molecule comprises a compound comprising a β-lactam ring, preferably a β-lactam antibiotic selected from the list consisting of penicillin derivatives (penams), cephalosporins (cephems), monobactams, and carbapenems, more preferably methicillin, a capture tag on one end and i) a reporter molecule or ii) a further capture tag capable of binding the reporter molecule located in the conjugate pad of a support of a lateral flow device, on the other end;
b. A support suitable for lateral flow that comprises:
b1. A backing card; and
b2. A sample pad, a membrane and a wicking pad, all of them on top of the backing pad, wherein the membrane is located after the sample pad so that the sample flows from the sample pad to the membrane, wherein the membrane comprises at least two test lines which in turn respectively comprises molecules immobilized in each test line for capturing the capture tag of the polynucleotide sequence/s and for capturing the capture tag of the β-lactamase responsive-molecules, and wherein the wicking pad is located at the end of the membrane.

8. The system of claim 7, wherein the membrane further comprises a control line.

9. A system that comprises:
a. A synthetic Substrate or mixture of Substrates for detecting the presence of a bacteria and the β-lactamase responsive-molecule for establishing the resistance of the bacteria to beta-lactam antibiotics and β-lactamase responsive-molecules, wherein a1. the Substrate(s) comprises a single-stranded nucleic acid molecule containing at least one ribonucleotide or deoxyribonucleotide or chemically modified nucleotide residue at an internal position that functions as a nuclease cleavage site, a capture tag on one end and i) a reporter molecule or ii) a further capture tag capable of binding the reporter molecule located in the conjugate pad of a support of a lateral flow device, on the other end; and wherein a2. the β-lactamase responsive-molecule comprises a compound comprising a β-lactam ring, preferably a β-lactam antibiotic selected from the list consisting of penicillin derivatives (penams), cephalosporins (cephems), monobactams, and carbapenems, more preferably methicillin, a capture tag on one end and i) a reporter molecule or ii) a further capture tag capable of binding the reporter molecule located in the conjugate pad of a support of a lateral flow device, on the other end, and wherein the
substrate's and β-lactamase responsive-molecules comprise two capture molecules at each end of the molecules, and wherein one of the capture molecules is capable of binding the reporter molecule located in the conjugate pad of the support; and
b. A support suitable for lateral flow that comprises:
b1. A backing card; and
b2. A sample pad, a membrane and a wicking pad, all of them on top of the backing pad, wherein the membrane is located after the sample pad so that the sample flows from the sample pad to the membrane, wherein the membrane comprises at least two test lines which in turn respectively comprises molecules immobilized in each test line for capturing the capture tag of the polynucleotide sequence/s and for capturing the capture tag of the β-lactamase responsive-molecules, wherein the wicking pad is located at the end of the membrane,
and wherein the system further comprises a conjugate pad between the sample pad and the membrane, which in turn comprises a reporter molecule.

10. The system of claim 9, wherein the membrane further comprises a control line.

11. The system of any of claims 7 or 8, or the system of any of claims 9 or 10, wherein the system comprises a support to incubate a biological sample and the synthetic Substrate or mixture of Substrates and the β-lactamase responsive-molecules.

12. The system of any of claims 7 to 11, wherein the β-lactamase responsive-molecule is as defined in claim 4.

13. Use of the system of any of claims 7 to 12, for simultaneously detecting bacteria and its resistance to beta-lactam antibiotics such as methicillin in a test sample.

14. The method of any of claims 1 to 6, wherein the method further comprises the use of a synthetic Substrate or mixture of Substrates for detecting the presence of mammalian cancer cells in the test sample and/or the resistance to cancer treatment.

## Patentansprüche

1. Verfahren zur Detektion von Bakterien und deren Resistenz gegenüber der Behandlung mit Beta-Lactam-Antibiotika in einer Testprobe, welches Folgendes umfasst: 1) das Inkubieren in der Testprobe eines synthetischen Substrats oder einer Mischung von Substraten zur Detektion der Anwesenheit einer Bakterie, und 2) das gleichzeitige oder nachträgliche Feststellen der Resistenz der Bakterie gegenüber einer Behandlung mit Beta-Lactam-Antibiotika, indem in der Testprobe ein auf β-Lactamase reagierendes Molekül, für einen Zeitraum, der für die Spaltung des auf β-Lactamase reagierenden Moleküls mittels einer β-Lactamase ausreicht, inkubiert wird; wobei
a. das Substrat/die Substrate ein einzelsträngiges Nukleinsäuremolekül umfasst/umfassen, mindestens enthaltend ein Ribonukleotid oder Desoxyribonukleotid oder einen chemisch modifizierten Nukleotidrest an einer inneren Position agierend als Nuclease-Spaltstelle, eine Einfangmarkierung an einem Ende und i) ein Reportermolekül oder ii) eine zusätzliche Einfangmarkierung, welche in der Lage ist, sich mit dem Reportermolekül, welches sich im Konjugatpad einer Unterlage einer Lateralflussvorrichtung befindet, am anderen Ende zu binden; wobei
b. das auf β-Lactamase reagierende Molekül eine Verbindung umfasst, welche einen β-Lactamring, vorzugsweise ein β-Lactam-Antibiotikum ausgewählt aus der Liste bestehend aus Penicillinderivaten (Penamen), Cephalosporinen (Cephemen), Monobactamen und Carbapenemen, weiter vorzugsweise Methicillin, eine Einfangmarkierung an einem Ende und i) ein Reportermolekül oder ii) eine zusätzliche Einfangmarkierung, welche in der Lage ist, sich mit dem Reportermolekül, welches sich im Konjugatpad einer Unterlage einer Lateralflussvorrichtung befindet, am anderen Ende zu binden, umfasst.

2. Verfahren nach Anspruch 1, wobei das Ergebnis, vorzugsweise visuell, detektiert wird, insbesondere ob das Substrat/die Substrate und das auf β-Lactamase reagierende Molekül mittels einer Testprobe gespaltet worden sind oder nicht, unter Verwendung einer Lateralflussvorrichtung oder mittels der Bindung der aus dem Substrat/den Substrates und dem auf β-Lactamase reagierenden Molekül gebildeten Komplexe nach der Inkubation an einer festen Unterlage, was Immuntest-Techniken begünstigt, wie den enzymverbundenen Immun-Sorptions-Test (ELISA), einen Radioimmuntest (RIA), einen Immunradiometrischen Test (IRMA), einen Fluoreszenz-Immuntest (FIA), einen Chemolumineszenz-Immuntest (CLIA), einen auf magnetischen Kügelchen basierendes Immuntest (BI) oder einen Elektrochemolumineszenz-Immuntest (ECL).

3. Verfahren nach Anspruch 1, wobei das Ergebnis, vorzugsweise visuell, detektiert wird, insbesondere ob das Substrat/die Substrate und das auf β-Lactamase reagierende Molekül mittels einer Testprobe, unter Verwendung einer Lateralflussvorrichtung gespaltet worden ist oder nicht.

4. Verfahren nach einem der Ansprüche 1 bis 3, wobei das genannte auf β-Lactamase reagierende Molekül 4-Methoxybenzyl-(6R,7R)-3-(((4-benzamidophenyl)thio)methyl)-8-oxo-7-(2-phenylacetamido)-5-thia-1-azabicyclo[4.2.0]oct-2-en-2-carboxylat, welches ein verkürztes auf β-Lactam reagierendes Molekül (CEProbe) ist, oder (6S,7S)-7-(((3',6'-Dihydroxy-3-oxo-3H-spiro[isobenzofuran-1,9'-xanthen]-6-ylcarbonyl)oxy)amino)-8-oxo-3-(((4-(5-((3aR,4R,6aS)-2-oxohexahydro-1H-thieno[3,4-d]imidazol-4-yl)pentanamido)phenyl)thio)methyl)-5-thia-1-azabicyclo[4.2.0]oct-2-en-2-carbonsäure (FAM-CEProbe-Biotin) oder (6S,7S)-7-(5-(((3S,5R,8R,9S,10S,12R,13S,14S,17R)-12,14-Dihydroxy-10,13-dimethyl-17-(5-oxo-2,5-dihydrofuran-3-yl)hexadecahydro-1H-cyclopenta[a]phenanthren-3-yl)oxy)-5-oxopentanamido)-8-oxo-3-(((4-(5-((3aR,4R,6aS)-2-oxohexahydro-1H-thieno[3,4-d]imidazol-4-yl)pentanamido)phenyl)thio)methyl)-5-thia-1-azabicyclo[4.2.0]oct-2-en-2-carbonsäure (Digoxigenin-CEProbe-Biotin) umfasst.

5. Verfahren nach einem der Ansprüche 1 bis 4, wobei die Einfangmarkierung des Substrats/der Substrate und/oder des auf β-Lactamase reagierenden Moleküls aus der Liste bestehend aus BIOTIN, FITC, FAM, DIGOXIGENIN, DNP (DINITROPHENYL), TEXAS RED, TAMRA oder jedem anderen kleinen Antigen ausgewählt wird.

6. Verfahren nach einem der Ansprüche 1 bis 5, wobei das Reportermolekül des Substrats/der Substrate und/oder des auf β-Lactamase reagierendes Molekül aus der Liste bestehend aus Latex und Goldnanopartikeln, Kohlenstoffpartikeln oder jedem anderen kolorimetrischen Reportermolekül ausgewählt wird.

7. System umfassend:
a. ein synthetisches Substrat oder eine Mischung von Substraten zur Detektion der Anwesenheit einer Bakterie und/oder auf β-Lactamase reagierendes Molekül zur Feststellung der Resistenz der Bakterie gegenüber Beta-Lactam-Antibiotika und auf β-Lactamase reagierenden Molekülen; wobei
a1. das Substrat/die Substrate ein einzelsträngiges Nukleinsäuremolekül umfasst/umfassen, mindestens enthaltend ein Ribonukleotid oder Desoxyribonukleotid oder einen chemisch modifizierten Nukleotidrest an einer inneren Position agierend als Nuclease-Spaltstelle, eine Einfangmarkierung an einem Ende und i) ein Reportermolekül oder ii) eine zusätzliche Einfangmarkierung, welche in der Lage ist, sich mit dem Reportermolekül, welches sich im Konjugatpad einer Unterlage einer Lateralflussvorrichtung befindet, am anderen Ende zu binden; und wobei
a2. das auf β-Lactamase reagierende Molekül eine Verbindung umfasst, welche einen β-Lactamring, vorzugsweise ein β-Lactam-Antibiotikum ausgewählt aus der Liste bestehend aus Penicillinderivaten (Penamen), Cephalosporinen (Cephemen), Monobactamen und Carbapenemen, weiter vorzugsweise Methicillin, eine Einfangmarkierung an einem Ende und i) ein Reportermolekül oder ii) eine zusätzliche Einfangmarkierung, welche in der Lage ist, sich mit dem Reportermolekül, welches sich im Konjugatpad einer Unterlage einer Lateralflussvorrichtung befindet, am anderen Ende zu binden, umfasst;
b. eine Unterlage, welche für den Lateralfluss geeignet ist, welche Folgendes umfasst:
b1. eine Stützkarte; und
b2. ein Probenpad, eine Membran und ein Kapillarpad, alle davon auf dem Stützpad, wobei sich die Membran nach dem Probenpad befindet, sodass die Probe vom Probenpad zur Membran fließt, wobei die Membran mindestens zwei Testlinien umfasst, welche wiederum jeweils in jeder Testlinie immobilisierte Moleküle umfassen, zum Einfangen der Einfangmarkierung der Polynukleotidsequenz(en) und zum Einfangen der Einfangmarkierung der auf β-Lactamase reagierenden Moleküle, und wobei sich das Kapillarpad am Ende der Membran befindet.

8. System nach Anspruch 7, wobei die Membran zusätzlich eine Kontrolllinie umfasst.

9. System umfassend:
a. ein synthetisches Substrat oder eine Mischung von Substraten zur Detektion der Anwesenheit einer Bakterie und das auf β-Lactamase reagierende Molekül zur Feststellung der Resistenz der Bakterie gegenüber Beta-Lactam-Antibiotika und auf β-Lactamase reagierenden Molekülen, wobei a1. das Substrat/die Substrate ein einzelsträngiges Nukleinsäuremolekül umfasst/umfassen, mindestens enthaltend ein Ribonukleotid oder Desoxyribonukleotid oder einen chemisch modifizierten Nukleotidrest an einer inneren Position agierend als Nuclease-Spaltstelle, eine Einfangmarkierung an einem Ende und i) ein Reportermolekül oder ii) eine zusätzliche Einfangmarkierung, welche in der Lage ist, sich mit dem Reportermolekül, welches sich im Konjugatpad einer Unterlage einer Lateralflussvorrichtung befindet, am anderen Ende zu binden; und wobei a2. das auf β-Lactamase reagierende Molekül eine Verbindung umfasst, welche einen β-Lactamring, vorzugsweise ein β-Lactam-Antibiotikum ausgewählt aus der Liste bestehend aus Penicillinderivaten (Penamen), Cephalosporinen (Cephemen), Monobactamen und Carbapenemen, weiter vorzugsweise Methicillin, eine Einfangmarkierung an einem Ende und i) ein Reportermolekül oder ii) eine zusätzliche Einfangmarkierung, welche in der Lage ist, sich mit dem Reportermolekül, welches sich im Konjugatpad einer Unterlage einer Lateralflussvorrichtung befindet, am anderen Ende zu binden, umfasst, und wobei die Substrate und die auf β-Lactamase reagierenden Moleküle zwei Einfangmoleküle an jedem Ende der Moleküle umfassen, und wobei eines der Einfangmoleküle in der Lage ist, sich mit dem Reportermolekül, welches sich im Konjugatpad der Unterlage befindet, zu binden; und
b. eine Unterlage, welche für den Lateralfluss geeignet ist, welche Folgendes umfasst:
b1. eine Stützkarte; und
b2. ein Probenpad, eine Membran und ein Kapillarpad, alle davon auf dem Stützpad, wobei sich die Membran nach dem Probenpad befindet, sodass die Probe vom Probenpad zur Membran fließt, wobei die Membran mindestens zwei Testlinien umfasst, welche wiederum jeweils in jeder Testlinie immobilisierte Moleküle umfassen, zum Einfangen der Einfangmarkierung der Polynukleotidsequenz(en) und zum Einfangen der Einfangmarkierung der auf β-Lactamase reagierenden Moleküle, wobei sich das Kapillarpad am Ende der Membran befindet,
und wobei das System zusätzlich ein Konjugatpad zwischen dem Probenpad und der Membran umfasst, welches wiederum ein Reportermolekül umfasst.

10. System nach Anspruch 9, wobei die Membran zusätzlich eine Kontrolllinie umfasst.

11. System nach einem der Ansprüche 7 oder 8, oder System nach einem der Ansprüche 9 oder 10, wobei das System eine Unterlage umfasst, um eine biologische Probe und das synthetische Substrate oder die Mischung von Substraten und die auf β-Lactamase reagierenden Moleküle zu inkubieren.

12. System nach einem der Ansprüche 7 bis 11, wobei das auf β-Lactamase reagierende Molekül nach Anspruch 4 ist.

13. Verwendung des Systems nach einem der Ansprüche 7 oder 12, zur gleichzeitigen Detektion von Bakterien und deren Resistenz gegenüber Beta-Lactam-Antibiotika wie Methicillin in einer Testprobe.

14. Verfahren nach einem der Ansprüche 1 bis 6, wobei das Verfahren zusätzlich die Verwendung eines synthetischen Substrats oder einer Mischung von Substraten zur Detektion der Anwesenheit von Säugetierkrebszellen in der Testprobe und/oder der Resistenz gegenüber der Krebsbehandlung umfasst.

## Revendications

1. Méthode pour détecter des bactéries et sa résistance au traitement par antibiotiques de bêta-lactame dans un échantillon d'essai, qui comprend : (1) incuber dans l'échantillon d'essai un substrat synthétique ou mélange de substrats pour détecter la présence d'une bactérie ; et (2) simultanément ou postérieurement établir la résistance de la bactérie au traitement par antibiotiques de bêta-lactame en incubant dans l'échantillon d'essai une molécule sensible à β-lactamase, pendant un temps suffisant pour le clivage de la molécule sensible à β-lactamase au moyen d'une β-lactamase ; dans laquelle
a. le(s) substrat(s) comprend/comprennent une molécule d'acide nucléique monocaténaire qui contient au moins un ribonucléotide ou désoxyribonucléotide ou résidu de nucléotide chimiquement modifié dans une position interne qui agit comme site de clivage de nucléase, un marqueur de capture à une extrémité et i) une molécule indicatrice ou ii) un marqueur de capture additionnel capable de s'unir à la molécule indicatrice située dans la couche de conjugué d'un support d'un dispositif à écoulement latéral à l'autre extrémité ; et dans laquelle
b. la molécule sensible à β-lactamase comprend un composé qui comprend un anneau de β-lactame, préférablement un antibiotique de β-lactame sélectionné de la liste qui consiste en dérivés de pénicilline (pénames), céphalosporines (céphèmes), monobactames et carbapénèmes, plus préférablement méthicilline, un marqueur de capture à une extrémité et i) une molécule indicatrice ou ii) un marqueur de capture additionnel capable de s'unir à la molécule indicatrice située dans la couche de conjugué d'un support d'un dispositif à écoulement latéral, à l'autre extrémité.

2. Méthode selon la revendication 1, dans laquelle le résultat est détecté, préférablement de manière visuelle, en particulier si le(s) substrat(s) et la molécule sensible à β-lactamase ont été clivés ou non par un échantillon d'essai, en utilisant un dispositif à écoulement latéral ou en unissant les complexes formés par le(s) substrat(s) et la molécule sensible à β-lactamase après l'incubation à un support solide qui facilite des techniques d'immunoessai telles que l'essai d'immunoadsorption par enzyme liée (ELISA), un essai radio-immunologique (RIA), un essai immunoradiométrique (IRMA), un essai immunologique par fluorescence (FIA), un essai immunologique par chimioluminescence (CLIA), un essai immunologique à base de billes magnétiques (MBI) ou un essai immunologique par électrochimioluminescence (ECL).

3. Méthode selon la revendication 1, dans laquelle le résultat est détecté, préférablement de manière visuelle, en particulier si le(s) substrat(s) et la molécule sensible à β-lactamase ont été clivés ou non par un échantillon d'essai, en utilisant un dispositif à écoulement latéral.

4. Méthode selon l'une quelconque des revendications 1 à 3, dans laquelle ladite molécule sensible à β-lactamase comprend (6R,7R)-3-(((4-benzamidophényl)thio)méthyl)-8-oxo-7-(2-phénylacétamido)-5-thia-1-azabicyclo[4.2.0]oct-2-ène-2-carboxylate de 4-méthoxybenzyle qui est une molécule tronquée sensible à β-lactamase (sonde EC), ou acide (6S,7S)-7-(((3',6'-dihydroxy-3-oxo-3H-spiro[isobenzofuran-1,9'-xanthène]-6-ylcarbonyl)oxy)amino)-8-oxo-3-(((4-(5-((3aR,4R,6aS)-2-oxohexahydro-1H-thiéno[3,4-d]imidazol-4-yl)pentanamido)phényl)thio)méthyl)-5-thia-1-azabicyclo[4.2.0]oct-2-ène-2-carboxylique (FAM-sonde CE-biotine), ou acide (6S,7S)-7-(5-(((3S,5R,8R,9S,10S,12R,13S,14S,17R)-12,14-dihydroxy-10,13-diméthyl-17-(5-oxo-2,5-dihydrofuran-3-yl)hexadécahydro-1H-cyclopenta[a]phénanthren-3-yl)oxy)-5-oxopentanamido)-8-oxo-3-(((4-(5-((3aR,4R,6aS)-2-oxohexahydro-1H-thiéno[3,4-d]imidazol-4-yl)pentanamido)phényl)thio)méthyl)-5-thia-1-azabicyclo[4.2.0]oct-2-ène-2-carboxylique (digoxigénine-sonde CE-biotine).

5. Méthode selon l'une quelconque des revendications 1 à 4, dans laquelle le marqueur de capture du/des substrat(s) et/ou de la molécule sensible à β-lactamase est sélectionné de la liste qui consiste en biotine, FITC, FAM, digoxigénine, DNP (dinitrophényle), rouge Texas, TAMRA ou tout autre petit antigène.

6. Méthode selon l'une quelconque des revendications 1 à 5, dans laquelle la molécule indicatrice du/des substrat(s) et/ou de la molécule sensible à β-lactamase est sélectionnée de la liste qui consiste en latex et nanoparticules d'or, particules de carbone ou toute autre molécule indicatrice colorimétrique.

7. Système qui comprend :
a. un substrat synthétique ou mélange de substrats pour détecter la présence d'une bactérie et/ou molécule sensible à β-lactamase pour établir la résistance de la bactérie à antibiotiques de bêta-lactame et molécules sensibles à β-lactamase ; dans lequel
a1. le(s) substrat(s) comprend/comprennent une molécule d'acide nucléique monocaténaire qui contient au moins un ribonucléotide ou désoxyribonucléotide ou résidu nucléotidique chimiquement modifié dans une position interne qui agit comme site de clivage de nucléase, un marqueur de capture à une extrémité et i) une molécule indicatrice ou ii) un marqueur de capture additionnel capable de s'unir à la molécule indicatrice située dans la couche de conjugué d'un support d'un dispositif à écoulement latéral à l'autre extrémité ; et dans lequel
a2. la molécule sensible à β-lactamase comprend un composé qui comprend un anneau de β-lactame, préférablement un antibiotique de β-lactame sélectionné de la liste qui consiste en dérivés de pénicilline (pénames), céphalosporines (céphèmes), monobactames et carbapénèmes, plus préférablement méthicilline, un marqueur de capture à une extrémité et i) une molécule indicatrice ou ii) un marqueur de capture additionnel capable de s'unir à la molécule indicatrice située dans la couche de conjugué d'un support d'un dispositif à écoulement latéral sur l'autre extrémité ;
b. un support adéquat pour écoulement latéral qui comprend :
b1. une plaque de support ; et
b2. une couche d'échantillon, une membrane et une couche d'absorption capillaire, tous situés sur la couche de support, dans lequel la membrane est située après la couche d'échantillon de sorte que l'échantillon s'écoule depuis la couche d'échantillon jusqu'à la membrane, dans lequel la membrane comprend au moins deux lignes d'essai qui comprennent à leur tour respectivement des molécules immobilisées dans chaque ligne d'essai pour capturer le marqueur de capture de la/des séquence(s) de polynucléotide et pour capturer le marqueur de capture des molécules sensibles à β-lactamase et dans lequel la couche d'absorption capillaire est située à la fin de la membrane.

8. Système selon la revendication 7, dans lequel la membrane comprend en outre une ligne de contrôle.

9. Système qui comprend :
a. un substrat synthétique ou mélange de substrats pour détecter la présence d'une bactérie et la molécule sensible à β-lactamase pour établir la résistance de la bactérie à antibiotiques de bêta-lactame et molécules sensibles à β-lactamase dans lequel a1. le(s) substrat(s) comprend/comprennent une molécule d'acide nucléique monocaténaire qui contient au moins un ribonucléotide ou un désoxyribonucléotide ou résidu de nucléotide chimiquement modifié dans une position interne qui agit comme site de clivage de nucléase, un marqueur de capture à une extrémité et i) une molécule indicatrice ou ii) un marqueur de capture additionnel capable de s'unir à la molécule indicatrice située dans la couche de conjugué d'un support d'un dispositif à écoulement latéral à l'autre extrémité ; et dans lequel a2. la molécule sensible à β-lactame comprend un composé qui comprend un anneau de β-lactame, préférablement un antibiotique de β-lactame sélectionné de la liste qui consiste en dérivés de pénicilline (pénames), céphalosporines (céphèmes), monobactames et carbapénèmes, plus préférablement méthicilline, un marqueur de capture à une extrémité et i) une molécule indicatrice ou ii) un marqueur de capture additionnel capable de s'unir à la molécule indicatrice située dans la couche de conjugué d'un support d'un dispositif à écoulement latéral, à l'autre extrémité, et dans lequel les substrats et les molécules sensibles à β-lactamase comprennent deux molécules de capture à chaque extrémité des molécules, et dans lequel une des molécules de capture est capable de s'unir à la molécule indicatrice située dans la couche de conjugué du support ; et
b. un support adéquat pour écoulement latéral qui comprend :
b1. une plaque de support ; et
b2. une couche d'échantillon, une membrane et une couche d'absorption capillaire, tous sur la couche de support, dans lequel la membrane est située après la couche d'échantillon de sorte que l'échantillon s'écoule depuis la couche d'échantillon jusqu'à la membrane, dans lequel la membrane comprend au moins deux lignes d'essai qui comprennent à leur tour respectivement des molécules immobilisées dans chaque ligne d'essai pour capturer le marqueur de capture de la/des séquence(s) de polynucléotide et pour capturer le marqueur de capture des molécules sensibles à β-lactamase et dans lequel la couche d'absorption capillaire est située à la fin de la membrane
et dans lequel le système comprend en outre une couche de conjugué entre la couche d'échantillon et la membrane, qui comprend à son tour une molécule indicatrice.

10. Système selon la revendication 9, dans lequel la membrane comprend en outre une ligne de contrôle.

11. Système selon l'une quelconque des revendications 7 ou 8, ou système selon l'une quelconque des revendications 9 ou 10, dans lequel le système comprend un support pour incuber un échantillon biologique et le substrat synthétique ou mélange de substrats et les molécules sensibles à β-lactamase.

12. Système selon l'une quelconque des revendications 7 à 11, dans laquelle la molécule sensible à β-lactamase est selon la revendication 4.

13. Utilisation du système selon l'une quelconque des revendications 7 ou 12, pour détecter simultanément des bactéries et leur résistance à antibiotiques de bêta-lactame tels que méthicilline dans un échantillon d'essai.

14. Méthode selon l'une quelconque des revendications 1 à 6, dans laquelle la méthode comprend en outre l'utilisation d'un substrat synthétique ou mélange de substrats pour détecter la présence de cellules cancéreuses de mammifères dans l'échantillon d'essai et/ou la résistance à un traitement contre le cancer.
